# EUROPEAN PATENT APPLICATION

(11) **EP 4 129 994 A1**
(43) Date of publication of application: **08.02.2023**
(21) Application number: 21775221.1
(22) Date of filing: 12.03.2021
(51) Int. Cl.: C07D 403/04, A61K 31/506, A61K 31/5377, A61P 25/28, C07D 401/14, C07D 405/14

(54) **NOVEL PYRIMIDINE DERIVATIVE, AND COMPOSITION FOR PREVENTING OR TREATING NEURODEGENERATIVE DISEASES AND CANCER, COMPRISING SAME**

(30) Priority: 23.03.2020 KR 20200035257; 09.02.2021 KR 20210018223
(71) Applicant: Whan In Pharmaceutical Co., Ltd., Songpa-gu, Seoul 05855 (KR); Korea Research Institute of Chemical Technology, Daejeon 34114 (KR)
(72) Inventor: LEE, Ge Hyeong, Daejeon 34114 (KR); SEO, Jee Yeon, Suwon-si, Gyeonggi-do 16229 (KR); YU, Jeong Heon, Suwon-si, Gyeonggi-do 16229 (KR); YE, In Hae, Suwon-si, Gyeonggi-do 16229 (KR); SHIN, Ho Chul, Suwon-si, Gyeonggi-do 16229 (KR); SUH, Jee Hee, Daejeon 34114 (KR); KIM, Seong Hwan, Daejeon 34114 (KR); SONG, Jin Sook, Daejeon 34114 (KR); SIM, Seong Hyeon, Daejeon 34114 (KR); CHAE, Chong Hak, Daejeon 34114 (KR); KANG, Hee Yeon, Daejeon 34114 (KR); LEE, Ji Hun, Daejeon 34114 (KR); HWANG, Keon Ha, Daejeon 34114 (KR)
(74) Representative: Gerauer, Marc Philippé
(86) International application number: PCT/KR2021/003120
(87) International publication number: WO 2021/194144

(57) **Abstract**

The present invention relates to a novel pyrimidine derivative, and a composition for preventing or treating neurodegenerative diseases and cancer, comprising same, the pyrimidine derivative having a LRRK2 protein inhibitory activity, and effectively passing through a blood-brain barrier (BBB) so as to be effectively used as a pharmaceutical composition for preventing or treating neurodegenerative diseases and cancer.

## Description

### Technical Field

The present disclosure relates to a novel pyrimidine derivative and a composition including same for prevention or treatment of neurodegenerative disease and cancer.

### Background Art

Neurodegenerative disease is a brain disease that occurs with the accumulation of environmental and genetic factors with aging and is an umbrella term for a range of various symptoms that result from progressive degeneration and death of neurons in the central nervous system. Neurodegenerative diseases are characterized by neuronal damage and loss of structure or function of neurons, resulting in memory impairment. Representative of neurodegenerative diseases are Parkinson's disease, Alzheimer's disease, Huntington's disease, Pick's disease, amyotrophic lateral sclerosis, prion disease, motor neuron disease, spinocerebellar ataxia, spinal muscular atrophy, Creutzfeldt-Jakob disease, and alcohol related dementia.

Parkinson's disease, which is one of the representative neurodegenerative diseases along with Alzheimer's disease, is caused by a lack of the neurotransmitter dopamine due to the loss of dopaminergic neurons in the substantia nigra of the brainstem. A typical characteristic of Parkinson's disease is the formation of intracellular proteinaceous inclusions called Lewy bodies in the neurons, with the consequent onset of obvious symptoms in the motor system, such as crouching posture, tremor, rigidity, instability, and the like, and in the mental functions, such as depression, anxiety, sleep disturbance, etc.

The cause of Parkinson's disease is poorly understood, but is considered to include complex action of several factors, such as environmental factors, genetic factors, aging, mitochondrial dysfunction, and defective clearance of unnecessary proteins. Parkinson's disease occurs, for the most part, sporadically, but 5-10 % of the cases are accounted for by hereditary mutations in genes. Genes known to be associated with the etiology of Parkinson's disease include parkin, PINK1, DJ-1, α-synuclein, and LRRK2 (leucine-rich repeat kinase 2).

Among them, G2019S mutation in LRRK2 gene is the most common genetic cause with a dominant genotype. LRRK2, which is a member of the leucine-rich repeat kinase family, consists of 2,527 amino acids with high interspecies similarity and exhibits both GTPase and serine-threonine kinase activities in a single protein. LRRK2 is expressed at high expression levels in dopaminergic neurons present in the cerebral cortex, substantia nigra, striatum, hippocampus, and cerebellum, which are associated with the etiology of Parkinson's disease. The increased activity of LRRK2 affects the onset and progression of Parkinson's disease. Overexpression of normal LRRK2 in neuronal cells induces apoptosis and, in LRRK2-overexpressed mutant animals, dopaminergic neuron death, Lewy body formulation, and motor impairment are observed as for Parkinson's disease. LRRK2 knockout animals were reported to show neurite outgrowth in the brain and inhibition of the aggregation of aging-dependent α-synuclein which is closely related to the onset of Parkinson's disease, indicating the possibility of treating Parkinson's disease by LRRK2 control. In addition, it has been known that LRRK2 is clinically linked to the transition from mild cognitive impairment to Alzheimer's disease. Compounds and compositions effective for regulating LRRK2 activity may thus provide therapeutic effects on neurodegenerative diseases. Therefore, selective inhibition of LRRK2 activity could be a beneficial target for the development of new drugs for disease treatment.

In addition to the foregoing neurodegenerative diseases, research has been being conducted into the mechanism of inhibiting growth of cancer cells and promoting apoptosis of cancer cells by regulating LRRK2 in various cancer cell lines.

Among others, kidney cancer, thyroid cancer, and liver cancer tissues were reported to overexpress LRRK2 and the hyperactivity of LRRK2 variants (particularly i.e., G2019S type) increases the risk of breast cancer. In addition, there was a research report that brain cancer patients having overexpressed LRRK2 overexpressed survived for a significantly short period of time in Korea. Recently, Voronoi received approval to enter phase 1 clinical trials for an LRRK2 inhibitor as a therapeutic agent for brain cancer. Therefore, selective inhibition of LRRK2 activity can be a target for a wide range of drug development, not only for the treatment of neurodegenerative diseases, but also for anticancer treatment.

With respect to related documents for compositions including pyrimidine derivatives as active ingredients for prevention or treatment of neurodegenerative diseases, reference may be made to Korean Patent Number 10-1566091 that discloses pyrazole aminopyrimidine derivatives as LLRK2 modulators, Korean Patent Number 10-2025451 that discloses 2-(phenyl or pyrid-3-yl) aminopyrimidine derivatives as kinase LRRK2 modulators for the treatment of Parkinson's disease, Korean Patent Number 10-1208198 that discloses a pharmaceutical composition comprising a compound having inhibitory effect against LRRK2 kinase activity as an active ingredient for treating or preventing Parkinson's disease, and Korean Patent Number 10-2019-0018676 A that discloses pyrimidin-2-ylamino-1H-pyrazols as LRRK2 inhibitors for use in the treatment of neurodegenerative disorder. However, nowhere have the pyrimidine derivatives represented by Chemical Formula 1 of the present disclosure, inhibitory activity thereof LRRK2, and therapeutic possibility thereof for neurodegenerative disease been described in reports prior to the present disclosure.

Leading to the present disclosure, intensive and thorough research conducted by the present inventors into LRRK resulted in the finding that pyrimidine derivatives represented by Chemical Formula 1 of the present disclosure exhibits excellent inhibitory activity against LRRK2 and effectively cross the blood-brain barrier (BBB).

### Disclosure of Invention

### Technical Problem

An aspect of the present disclosure is to provide a novel pyrimidine and a composition containing same for prevention or treatment of neurodegenerative disease and cancer and, more specifically, to a novel pyrimidine derivative that is superb in inhibitory activity LRRK2 and transfer across the blood-brain barrier (BBB) and a composition including same for prevention or treatment of neurodegenerative disease and cancer.

### Solution to Problem

The present disclosure is drawn to a compound represented by the following Chemical Formula 1, an optical isomer thereof, or a pharmaceutically acceptable salt thereof:

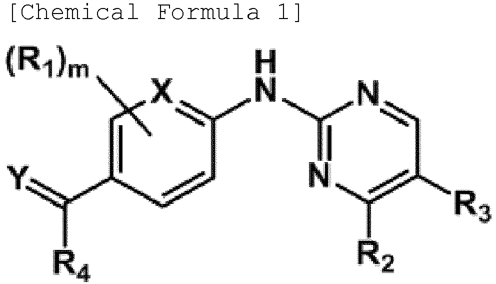

wherein,
R₁ is each independently selected from the group consisting of a hydrogen, a halogen, a hydroxy, a cyano, an amino, an amide, nitro, a C₁-C₆ alkyl, C₁-C₆ alkoxy, and a halo C₁-C₆ alkoxy;
R₂ is a substituted or unsubstituted pyrazole,
wherein the substituted pyrazole has as a substituent thereon at least one radical selected from the group consisting of a halogen, a hydroxy, a cyano, an amino, an amide, a nitro, a C₁-C₆ alkylsulfonyl, an acetyl, a methyloxycarbonyl, a substituted or unsubstituted C₁-C₆ alkyl, a substituted or unsubstituted benzyl, a substituted or unsubstituted C₃-C₁₂ cycloalkyl, a substituted or unsubstituted C₃-C₁₂ heterocycloalkyl, a substituted or unsubstituted C₄-C₁₂ aryl, and a substituted or unsubstituted C₄-C₁₂ hetaroaryl,
wherein the substituted alkyl, the substituted benzyl, the substituted C₃-C₁₂ cycloalkyl, the substituted C₄-C₁₂ aryl, and the substituted C₄-C₁₂ hetaroaryl have each as a substituent thereon at least one radical selected from the group consisting of a halogen, a hydroxy, a cyano, an amino, an amide, a nitro, a C₁-C₆ alkyl, a C₁-C₆ alkoxy, a C₃-C₁₂ heterocycloalkyl, a C₄-C₁₂ an aryl-C₁-C₆ alkoxy, and a - C(=O)O-alkyl(C₁-C₆);
R₃ is a halogen or a halo C₁-C₆ alkyl;
R₄ is represented by any one of B-1 to B-29, below,

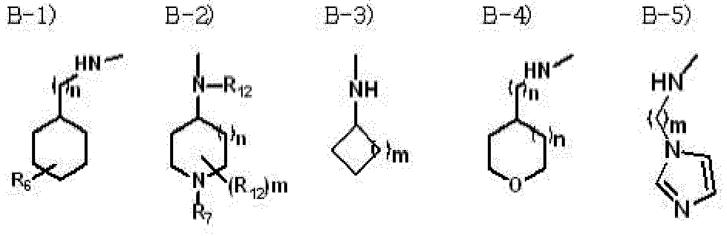

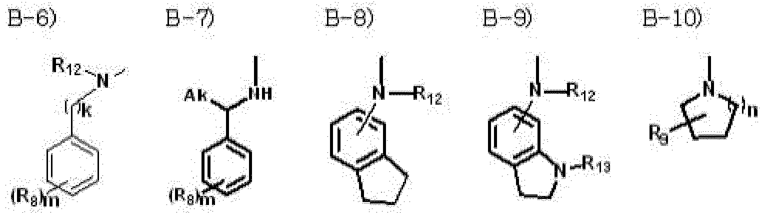

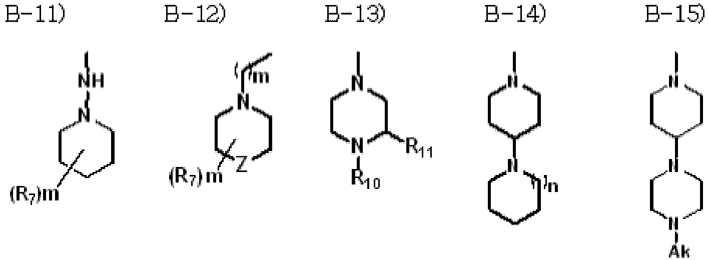

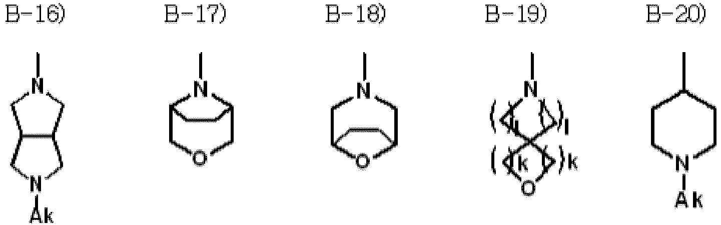

- 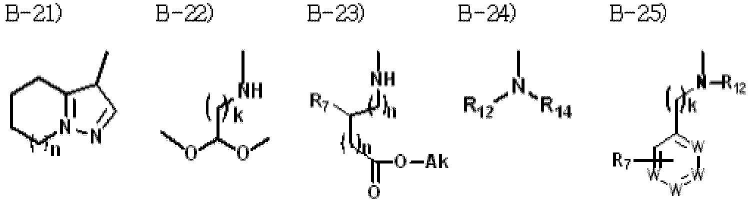 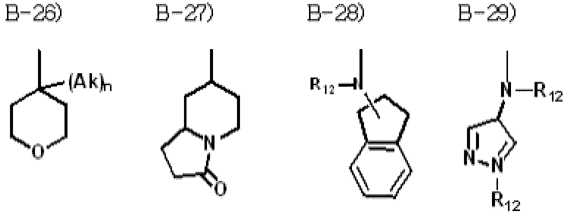
-
wherein,
   R₆ is selected from the group consisting of a hydrogen, a halogen, a hydroxy, a C₁-C₆ alkyl, a C₁-C₆ hydroxyalkyl, a C₁-C₆ alkylsulfonate, and a C₁-C₆ alkylamino;
R₇ is each independently selected from the group consisting of a hydrogen, a halogen, a halo C₁-C₆ alkyl, and a C₁-C₆ alkyl;
R₈ is each independently selected from the group consisting of a hydrogen, a halogen, an amino, a hydroxy, a cyano, a C₁-C₆ dialkylamino, an aminosulfonyl, a C₁-C₆ alkylsulfonamino, a C₁-C₆ alkyl, a halo C₁-C₆ alkyl, a halo C₁-C₆ alkyloxy, and a C₁-C₆ alkoxy;
R₉ is selected from the group consisting of a hydrogen, a halogen, a hydroxy, a hydroxy C₁-C₆ alkyl, a C₁-C₆ alkylsulfonyl, a C₁-C₆ alkylacetyl, a C₁-C₆ alkyloxycarbonyl, a C₁-C₆ alkylamino, a C₁-C₆ alkoxy, a morpholine, a piperidine, a pyrrolidine, an oxetanyl, a C₁-C₆ alkyl-substituted piperidine, and an acetate;
R₁₀ is selected from the group consisting of a hydrogen, a C₁-C₆ alkyl, a phenyl, an oxetanyl, and an acetate;
R₁₁ is a hydrogen or bonds to R₁₀ to form a saturated or unsaturated C₅-C₆ heterocycloalkyl;
R₁₂ is a hydrogen or a C₁-C₆ alkyl;
R₁₃ is a hydrogen, a C₁-C₆ alkylcarbonyl, or a C₁-C₆ alkylacetyl;
R₁₄ is a hydrogen, a C₁-C₆ alkyl, a hydroxy C₁-C₆ alkyl, a C₁-C₆ alkoxy C₁-C₆ alkyl, a C₁-C₆ alkyloxycarbonyl C₁-C₆ alkyl, or a methylsulfonyl C₁-C₆ alkyl;
Ak is a C₁-C₆ alkyl;
k is each independently 0, 1, or 2 (with a provision that all k's are non-zero simultaneously);
l is 1 or 2;
m is 0, 1, 2, 3, or 4;
n is 0 or 1,
W is each independently N;
Z is O or SO₂;
X is C, CH, or N; and
Y is selected from the group consisting of O and S.

Concrete examples of the compound of Chemical Formula 1 include:
(4-((5-chloro-4-(1-methyl-1H-pyrazol-4-yl)pyrimidin-2-yl)amino)-3-methoxyphenyl)(morpholino)methanone (Compound 1);
(4-((5-chloro-4-(1-methyl-1H-pyrazol-4-yl)pyrimidin-2-yl)amino)-3-methoxyphenyl)(4-morpholinopiperidin-1-yl)methanone (Compound 2);
1-(4-(5-chloro-2-((2-methoxy-4-(morpholine-4-carbonyl)phenyl)amino)pyrimidin-4-yl)-1H-pyrazol-1-yl)ethan-1-one (Compound 3);
(4-((5-chloro-4-(1-(2-hydroxyethyl)-1H-pyrazol-4-yl)pyrimidin-2-yl)amino)-3-methoxyphenyl)(morpholino)methanone (Compound 4);
3-(4-(5-chloro-2-((2-methoxy-4-(morpholine-4-carbonyl)phenyl)amino)pyrimidin-4-yl)-1H-pyrazol-1-yl)propanenitrile (Compound 5);
2-(4-(5-chloro-2-((2-methoxy-4-(morpholine-4-carbonyl)phenyl)amino)pyrimidin-4-yl)-1H-pyrazol-1-yl)acetonitrile (Compound 6);
3-(4-(5-chloro-2-((2-methoxy-4-(morpholine-4-carbonyl)phenyl)amino)pyrimidin-4-yl)-1H-pyrazol-1-yl)butanenitrile (Compound 7);
(4-((5-chloro-4-(1-(2-morpholinoethyl)-1H-pyrazol-4-yl)pyrimidin-2-yl)amino)-3-methoxyphenyl)(morpholino)methanone (Compound 8);
methyl 2-(4-(5-chloro-2-((2-methoxy-4-(morpholine-4-carbonyl)phenyl)amino)pyrimidin-4-yl)-1H-pyrazol-1-yl)acetate (Compound 9);
(4-((5-chloro-4-(1-(methylsulfonyl)-1H-pyrazol-4-yl)pyrimidin-2-yl)amino)-3-methoxyphenyl)(morpholino)methanone (Compound 10);
(4-((5-chloro-4-(1-(pyridin-2-yl)-1H-pyrazol-4-yl)pyrimidin-2-yl)amino)-3-methoxyphenyl)(morpholino)methanone (Compound 11);
(4-((5-chloro-4-(1-(pyridin-3-yl)-1H-pyrazol-4-yl)pyrimidin-2-yl)amino)-3-methoxyphenyl)(morpholino)methanone (Compound 12);
(4-((5-chloro-4-(1-(pyridin-4-yl)-1H-pyrazol-4-yl)pyrimidin-2-yl)amino)-3-methoxyphenyl)(morpholino)methanone (Compound 13);
(4-((5-chloro-4-(1-phenyl-1H-pyrazol-4-yl)pyrimidin-2-yl)amino)-3-methoxyphenyl)(morpholino)methanone (Compound 14);
(4-((5-chloro-4-(1-(difluoromethyl)-1H-pyrazol-4-yl)pyrimidin-2-yl)amino)-3-methoxyphenyl)(morpholino)methanone (Compound 15);
(4-((5-chloro-4-(1-(methoxymethyl)-1H-pyrazol-4-yl)pyrimidin-2-yl)amino)-3-methoxyphenyl)(morpholino)methanone (Compound 16);
(4-((5-chloro-4-(1-(4-methoxybenzyl)-1H-pyrazol-4-yl)pyrimidin-2-yl)amino)-3-methoxyphenyl)(morpholino)methanone (Compound 17);
(4-((5-chloro-4-(1H-pyrazol-4-yl)pyrimidin-2-yl)amino)-3-methoxyphenyl)(morpholino)methanone (Compound 18);
(4-((5-chloro-4-(1-methyl-1H-pyrazol-4-yl)pyrimidin-2-yl)amino)-3-methoxyphenyl)(4-(4-methylpiperazin-1-yl)piperidin-1-yl)methanone (Compound 19);
(4-((5-chloro-4-(1-(tetrahydro-2H-pyran-4-yl)-1H-pyrazol-4-yl)pyrimidin-2-yl)amino)-3-methoxyphenyl)(morpholino)methanone (Compound 20);
4-((5-chloro-4-(1-methyl-1H-pyrazol-4-yl)pyrimidin-2-yl)amino)-3-methoxy-N-(2-methoxyethyl)-N-methylbenzamide (Compound 21);
(4-((5-chloro-4-(1-methyl-1H-pyrazol-4-yl)pyrimidin-2-yl)amino)-3-methoxyphenyl)(pyrrolidin-1-yl)methanone (Compound 22);
4-((5-chloro-4-(1-methyl-1H-pyrazol-4-yl)pyrimidin-2-yl)amino)-N-ethyl-3-methoxy-N-(2-methoxyethyl)benzamide(Compound 23);
4-((5-chloro-4-(1-methyl-1H-pyrazol-4-yl)pyrimidin-2-yl)amino)-N-(1-hydroxy-2-methylpropan-2-yl)-3-methoxybenzamide (Compound 24);
N-(tert-butyl)-4-((5-chloro-4-(1-methyl-1H-pyrazol-4-yl)pyrimidin-2-yl)amino)-3-methoxybenzamide(Compound 25);
4-((5-chloro-4-(1-methyl-1H-pyrazol-4-yl)pyrimidin-2-yl)amino)-N-(2-hydroxy-2-methylpropyl)-3-methoxybenzamide (Compound 26);
3-(4-(5-chloro-2-((2-methoxy-4-(morpholine-4-carbonyl)phenyl)amino)pyrimidin-4-yl)-1H-pyrazol-1-yl)-3-methylbutanenitrile (Compound 27);
(4-((5-chloro-4-(1-(2-methoxyethyl)-1H-pyrazol-4-yl)pyrimidin-2-yl)amino)-3-methoxyphenyl)(morpholino)methanone (Compound 28);
(3-methoxy-4-((4-(1-(tetrahydro-2H-pyran-4-yl)-1H-pyrazol-4-yl)-5-(trifluoromethyl)pyrimidin-2-yl)amino)phenyl)(morpholino)methanone (Compound 29);
4-((5-chloro-4-(1-methyl-1H-pyrazol-4-yl)pyrimidin-2-yl)amino)-N-(4,4-difluorocyclohexyl)-3-methoxybenzamide (Compound 30);
(3-methoxy-4-((4-(1-methyl-1H-pyrazol-4-yl)-5-(trifluoromethyl)pyrimidin-2-yl)amino)phenyl)(4-(4-methylpiperazin-1-yl)piperidin-1-yl)methanone (Compound 31);
(4-((5-chloro-4-(1-methyl-1H-pyrazol-4-yl)pyrimidin-2-yl)amino)-3-methoxyphenyl)(2-oxa-7-azaspiro[3.5]nonan-7-yl)methanone (Compound 32);
(4-((5-chloro-4-(1-methyl-1H-pyrazol-4-yl)pyrimidin-2-yl)amino)-3-methoxyphenyl) (1-oxa-6-azaspiro[3.3]heptan-6-yl)methanone (Compound 33);
(4-((5-chloro-4-(1-methyl-1H-pyrazol-4-yl)pyrimidin-2-yl)amino)-3-methoxyphenyl)(4-isopropylpiperazin-1-yl)methanone (Compound 34);
(4-((5-chloro-4-(1-(tetrahydro-2H-pyran-4-yl)-1H-pyrazol-4-yl)pyrimidin-2-yl)amino)-3-methoxyphenyl)(2-oxa-7-azaspiro[3.5]nonan-7-yl)methanone (Compound 35);
(3-methoxy-4-((4-(1-methyl-1H-pyrazol-4-yl)-5-(trifluoromethyl)pyrimidin-2-yl)amino)phenyl)(morpholino)methanone (Compound 36);
(4-((5-chloro-4-(1-isopropyl-1H-pyrazol-4-yl)pyrimidin-2-yl)amino)-3-methoxyphenyl)(morpholino)methanone (Compound 37);
4-((5-chloro-4-(1-methyl-1H-pyrazol-4-yl)pyrimidin-2-yl)amino)-3-methoxy-N-(1-methylpiperidin-4-yl)benzamide (Compound 38);
(4-((5-chloro-4-(1-methyl-1H-pyrazol-4-yl)pyrimidin-2-yl)amino)-3-methoxyphenyl)(4-(2-hydroxypropan-2-yl)piperidin-1-yl)methanone (Compound 39);
(4-((5-chloro-4-(1-methyl-1H-pyrazol-4-yl)pyrimidin-2-yl)amino)-3-methoxyphenyl)(2-oxa-6-azaspiro[3.3]heptan-6-yl)methanone (Compound 40);
(4-((5-chloro-4-(1-methyl-1H-pyrazol-4-yl)pyrimidin-2-yl)amino)-3-methoxyphenyl)(4,4-difluoropiperidin-1-yl)methanone (Compound 41);
(4-((5-chloro-4-(1-methyl-1H-pyrazol-4-yl)pyrimidin-2-yl)amino)-3-methoxyphenyl)(2,2-dimethylmorpholino)methanone (Compound 42);
(4-((5-chloro-4-(1-methyl-1H-pyrazol-4-yl)pyrimidin-2-yl)amino)-3-methoxyphenyl)(3-morpholinoazetidin-1-yl)methanone (Compound 43);
1-(4-(4-((5-chloro-4-(1-methyl-1H-pyrazol-4-yl)pyrimidin-2-yl)amino)-3-methoxybenzoyl)piperazin-1-yl)ethan-1-one(Compound 44);
(4-isopropylpiperazin-1-yl)(3-methoxy-4-((4-(1-methyl-1H-pyrazol-4-yl)-5-(trifluoromethyl)pyrimidin-2-yl)amino)phenyl)methanone (Compound 45);
(2-fluoro-5-methoxy-4-((4-(1-methyl-1H-pyrazol-4-yl)-5-(trifluoromethyl)pyrimidin-2-yl)amino)phenyl)(morpholino)methanone (Compound 46);
(3-methoxy-4-((4-(1-methyl-1H-pyrazol-4-yl)-5-(trifluoromethyl)pyrimidin-2-yl)amino)phenyl)(4-phenylpiperazin-1-yl)methanone (Compound 47);
(2-fluoro-5-methoxy-4-((4-(1-methyl-1H-pyrazol-4-yl)-5-(trifluoromethyl)pyrimidin-2-yl)amino)phenyl) (4-isopropylpiperazin-1-yl)methanone (Compound 48);
(4-((5-chloro-4-(1-isopropyl-1H-pyrazol-4-yl)pyrimidin-2-yl)amino)-2-fluoro-5-methoxyphenyl)(morpholino)methanone (Compound 49);
(4-((5-chloro-4-(1-methyl-1H-pyrazol-4-yl)pyrimidin-2-yl)amino)-3-methoxyphenyl) (4-(oxetan-3-yl)piperazin-1-yl)methanone (Compound 50);
((1R,5S)-8-oxa-3-azabicyclo[3.2.1]octan-3-yl)(4-((5-chloro-4-(1-methyl-1H-pyrazol-4-yl)pyrimidin-2-yl)amino)-3-methoxyphenyl)methanone (Compound 51);
(4-((5-chloro-4-(1-ethyl-1H-pyrazol-4-yl)pyrimidin-2-yl)amino)-3-methoxyphenyl)(pyridin-4-yl)methanone (Compound 52);
(4-((5-chloro-4-(1-propyl-1H-pyrazol-4-yl)pyrimidin-2-yl)amino)-3-methoxyphenyl)(pyridin-4-yl)methanone (Compound 53);
(4-((5-chloro-4-(1-isopropyl-1H-pyrazol-4-yl)pyrimidin-2-yl)amino)-3-methoxyphenyl)(pyridin-4-yl)methanone (Compound 54);
(4-((5-chloro-4-(1-methyl-1H-pyrazol-4-yl)pyrimidin-2-yl)amino)-3-methoxyphenyl)(pyridin-4-yl)methanone (Compound 55);
(4-((5-chloro-4-(1-ethyl-1H-pyrazol-4-yl)pyrimidin-2-yl)amino)-3-methoxyphenyl)(morpholino)methanone (Compound 56);
(4-((5-chloro-4-(1-propyl-1H-pyrazol-4-yl)pyrimidin-2-yl)amino)-3-methoxyphenyl)(morpholino)methanone (Compound 57);
(4-((5-chloro-4-(1-cyclopropyl-1H-pyrazol-4-yl)pyrimidin-2-yl)amino)-3-methoxyphenyl)(morpholino)methanone (Compound 58);
(4-((4-(1-(tert-butyl)-1H-pyrazol-4-yl)-5-chloropyrimidin-2-yl)amino)-3-methoxyphenyl)(morpholino)methanone (Compound 59);
4-((5-chloro-4-(1-isopropyl-1H-pyrazol-4-yl)pyrimidin-2-yl)amino)-3-methoxy-N-(1-methylpiperidin-4-yl)benzamide (Compound 60);
(4-((5-chloro-4-(1-methyl-1H-pyrazol-4-yl)pyrimidin-2-yl)amino)-3-methoxyphenyl)(4-methylpiperazin-1-yl)methanone (Compound 61);
(4-((5-chloro-4-(1-isopropyl-1H-pyrazol-4-yl)pyrimidin-2-yl)amino)-3-methoxyphenyl)(4-methylpiperazin-1-yl)methanone (Compound 62);
(4-((4-(1-isopropyl-1H-pyrazol-4-yl)-5-(trifluoromethyl)pyrimidin-2-yl)amino)-3-methoxyphenyl)(morpholino)methanone (Compound 63);
(4-((5-chloro-4-(1-isopropyl-1H-pyrazol-4-yl)pyrimidin-2-yl)amino)-3-methoxyphenyl)(4-isopropylpiperazin-1-yl)methanone (Compound 64);
(2-fluoro-4-((4-(1-isopropyl-1H-pyrazol-4-yl)-5-(trifluoromethyl)pyrimidin-2-yl)amino)-5-methoxyphenyl)(morpholino)methanone (Compound 65);
(4-((5-chloro-4-(1-isopropyl-1H-pyrazol-4-yl)pyrimidin-2-yl)amino)-3-methoxyphenyl)(tetrahydro-2H-pyran-4-yl)methanone (Compound 66);
(4-((5-chloro-4-(1-methyl-1H-pyrazol-4-yl)pyrimidin-2-yl)amino)-3-methoxyphenyl)(4-methyltetrahydro-2H-pyran-4-yl)methanone (Compound 67);
(4-((5-chloro-4-(1-isopropyl-1H-pyrazol-4-yl)pyrimidin-2-yl)amino)-3-methoxyphenyl)(4-methyltetrahydro-2H-pyran-4-yl)methanone (Compound 68);
(4-((5-chloro-4-(1-methyl-1H-pyrazol-4-yl)pyrimidin-2-yl)amino)-2-fluoro-5-methoxyphenyl)(morpholino)methanone (Compound 69);
(4-((5-chloro-4-(1-isopropyl-1H-pyrazol-4-yl)pyrimidin-2-yl)amino)-3-methoxyphenyl)(4-(2-hydroxypropan-2-yl)piperidin-1-yl)methanone (Compound 70);
(4-((5-chloro-4-(1-isopropyl-1H-pyrazol-4-yl)pyrimidin-2-yl)amino)-3-methoxyphenyl)(2-oxa-7-azaspiro[3.5]nonan-7-yl)methanone (Compound 71);
(4-((5-chloro-4-(1-isopropyl-1H-pyrazol-4-yl)pyrimidin-2-yl)amino)-3-methoxyphenyl) (4-(oxetan-3-yl)piperazin-1-yl)methanone (Compound 72);
(4-((5-chloro-4-(1-isopropyl-1H-pyrazol-4-yl)pyrimidin-2-yl)amino)-3-methoxyphenyl)(3-morpholinoazetidin-1-yl)methanone (Compound 73);
(4-((5-chloro-4-(1-isopropyl-1H-pyrazol-4-yl)pyrimidin-2-yl)amino)-3-methoxyphenyl)(morpholino)methanethione (Compound 74);
(4-((5-chloro-4-(1-isopropyl-1H-pyrazol-4-yl)pyrimidin-2-yl)amino)-3-methoxyphenyl)(1-isopropylpiperidin-4-yl)methanone (Compound 75);
(4-((5-chloro-4-(1-isopropyl-1H-pyrazol-4-yl)pyrimidin-2-yl)amino)-3-methoxyphenyl)(4-methoxypiperidin-1-yl)methanone (Compound 76);
(4-((5-chloro-4-(1-isopropyl-1H-pyrazol-4-yl)pyrimidin-2-yl)amino)-3-methoxyphenyl)(3-oxa-9-azaspiro[5.5]undecan-9-yl)methanone (Compound 77);
1-(4-(4-((5-chloro-4-(1-isopropyl-1H-pyrazol-4-yl)pyrimidin-2-yl)amino)-3-methoxybenzoyl)piperazin-1-yl)ethan-1-one(Compound 78);
(4-((5-chloro-4-(1-isopropyl-1H-pyrazol-4-yl)pyrimidin-2-yl)amino)-3-methoxyphenyl)(4-(methylsulfonyl)piperidin-1-yl)methanone (Compound 79);
4-((5-chloro-4-(1-isopropyl-1H-pyrazol-4-yl)pyrimidin-2-yl)amino)-3-methoxy-N-(tetrahydro-2H-pyran-4-yl)benzamide(Compound 80);
N-(1-(2-(o-tolyl)quinolin-3-yl)ethyl)-6,7-dihydro-5H-pyrrolo[2,3-d]pyrimidin-4-amine (Compound 81);
4-((5-chloro-4-(1-isopropyl-1H-pyrazol-4-yl)pyrimidin-2-yl)amino)-N-cyclopropyl-3-methoxybenzamide(Compound 82);
(4-((5-chloro-4-(1-isopropyl-1H-pyrazol-4-yl)pyrimidin-2-yl)amino)-3-methoxyphenyl)(3-hydroxyazetidin-1-yl)methanone (Compound 83);
(4-((5-chloro-4-(1-isopropyl-1H-pyrazol-4-yl)pyrimidin-2-yl)amino)-3-methoxyphenyl)(1,1-dioxidothiomorpholino)methanone (Compound 84);
(4-((4-(1-(tert-butyl)-1H-pyrazol-4-yl)-5-chloropyrimidin-2-yl)amino)-3-methoxyphenyl)(4-methylpiperazin-1-yl)methanone (Compound 85);
(S)-(4-((5-chloro-4-(1-isopropyl-1H-pyrazol-4-yl)pyrimidin-2-yl)amino)-3-methoxyphenyl)(2-(hydroxymethyl)pyrrolidin-1-yl)methanone (Compound 86);
(4-((5-chloro-4-(1-isopropyl-1H-pyrazol-4-yl)pyrimidin-2-yl)amino)-3-methoxyphenyl)(hexahydropyrrolo[1,2-a]pyrazin-2(1H)-yl)methanone (Compound 87);
(4-((4-(1-(tert-butyl)-1H-pyrazol-4-yl)-5-chloropyrimidin-2-yl)amino)-3-methoxyphenyl)(4-isopropylpiperazin-1-yl)methanone (Compound 88);
((1R,5S)-8-oxa-3-azabicyclo[3.2.1]octan-3-yl)(4-((5-chloro-4-(1-isopropyl-1H-pyrazol-4-yl)pyrimidin-2-yl)amino)-3-methoxyphenyl)methanone (Compound 89);
(4-((5-chloro-4-(1-isopropyl-1H-pyrazol-4-yl)pyrimidin-2-yl)amino)-3-methoxyphenyl)(2-oxa-6-azaspiro[3.3]heptan-6-yl)methanone (Compound 90);
4-((5-chloro-4-(1-isopropyl-1H-pyrazol-4-yl)pyrimidin-2-yl)amino)-N-((1r,4r)-4-hydroxycyclohexyl)-3-methoxybenzamide(Compound 91);
(6-((5-chloro-4-(1-isopropyl-1H-pyrazol-4-yl)pyrimidin-2-yl)amino)pyridin-3-yl)(morpholino)methanone (Compound 92);
4-((5-chloro-4-(1-isopropyl-1H-pyrazol-4-yl)pyrimidin-2-yl)amino)-N-(4-(dimethylamino)cyclohexyl)-3-methoxybenzamide(Compound 93);
(4-((5-chloro-4-(1-isopropyl-1H-pyrazol-4-yl)pyrimidin-2-yl)amino)-3-methoxyphenyl)(4-(dimethylamino)piperidin-1-yl)methanone (Compound 94);
((1R,5S)-3-oxa-8-azabicyclo[3.2.1]octan-8-yl)(4-((5-chloro-4-(1-isopropyl-1H-pyrazol-4-yl)pyrimidin-2-yl)amino)-3-methoxyphenyl)methanone (Compound 95);
4-((4-(1-(tert-butyl)-1H-pyrazol-4-yl)-5-chloropyrimidin-2-yl)amino)-3-methoxy-N-(1-methylpiperidin-4-yl)benzamide(Compound 96);
(R)-(4-((5-chloro-4-(1-isopropyl-1H-pyrazol-4-yl)pyrimidin-2-yl)amino)-3-methoxyphenyl)(3-hydroxypyrrolidin-1-yl)methanone (Compound 97);
(4-((5-chloro-4-(1-isopropyl-1H-pyrazol-4-yl)pyrimidin-2-yl)amino)-3-methoxyphenyl)(5-methylhexahydropyrrolo[3,4-c]pyrrol-2(1H)-yl)methanone (Compound 98);
4-((5-chloro-4-(1-isopropyl-1H-pyrazol-4-yl)pyrimidin-2-yl)amino)-N-(1-isopropylpiperidin-4-yl)-3-methoxybenzamide (Compound 99);
2-(4-((5-chloro-4-(1-isopropyl-1H-pyrazol-4-yl)pyrimidin-2-yl)amino)-3-methoxybenzoyl)hexahydropyrrolo[1,2-a]pyrazin-6(2H)-one (Compound 100);
4-((5-chloro-4-(1-isopropyl-1H-pyrazol-4-yl)pyrimidin-2-yl)amino)-3-methoxy-N,N-dimethylbenzamide(Compound 101);
(4-((5-chloro-4-(1-isopropyl-1H-pyrazol-4-yl)pyrimidin-2-yl)amino)-2-fluoro-5-methoxyphenyl)(4-methylpiperazin-1-yl) methanethione (Compound 102);
(4-((5-chloro-4-(1-isopropyl-1H-pyrazol-4-yl)pyrimidin-2-yl)amino)-3-methoxyphenyl)(4-methylpiperazin-1-yl) methanethione (Compound 103);
(4-((5-chloro-4-(1-isopropyl-1H-pyrazol-4-yl)pyrimidin-2-yl)amino)-2-fluoro-5-methoxyphenyl)(4-methylpiperazin-1-yl)methanone (Compound 104);
(4-((5-chloro-4-(1-isopropyl-1H-pyrazol-4-yl)pyrimidin-2-yl)amino)-3-(difluoromethoxy)phenyl)(morpholino)methanone (Compound 105);
(4-((5-chloro-4-(1-isopropyl-1H-pyrazol-4-yl)pyrimidin-2-yl)amino)-2-fluoro-5-methoxyphenyl)(morpholino) methanethione (Compound 106);
(5-chloro-4-((5-chloro-4-(1-isopropyl-1H-pyrazol-4-yl)pyrimidin-2-yl)amino)-2-fluorophenyl)(4-methylpiperazin-1-yl)methanone (Compound 107);
(4-((5-chloro-4-(1-isopropyl-1H-pyrazol-4-yl)pyrimidin-2-yl)amino)-3-(difluoromethoxy)phenyl)(4-methylpiperazin-1-yl)methanone (Compound 108);
(4-((5-chloro-4-(1-isopropyl-1H-pyrazol-4-yl)pyrimidin-2-yl)amino)-3-methylphenyl)(4-methylpiperazin-1-yl)methanone (Compound 109);
(4-((5-chloro-4-(1-isopropyl-1H-pyrazol-4-yl)pyrimidin-2-yl)amino)-2-fluoro-5-methoxyphenyl)(4-(dimethylamino)piperidin-1-yl)methanone (Compound 110);
(4-((5-chloro-4-(1-isopropyl-1H-pyrazol-4-yl)pyrimidin-2-yl)amino)-2-fluoro-5-methoxyphenyl)(4-(dimethylamino)piperidin-1-yl)methanone (Compound 111);
4-((5-chloro-4-(1-isopropyl-1H-pyrazol-4-yl)pyrimidin-2-yl)amino)-3-methoxy-N-phenylbenzamide(Compound 112);
4-((5-chloro-4-(1-isopropyl-1H-pyrazol-4-yl)pyrimidin-2-yl)amino)-N-(2-fluorophenyl)-3-methoxybenzamide(Compound 113);
4-((5-chloro-4-(1-isopropyl-1H-pyrazol-4-yl)pyrimidin-2-yl)amino)-N-(3-fluorophenyl)-3-methoxybenzamide(Compound 114);
4-((5-chloro-4-(1-isopropyl-1H-pyrazol-4-yl)pyrimidin-2-yl)amino)-N-(4-fluorophenyl)-3-methoxybenzamide(Compound 115);
4-((5-chloro-4-(1-isopropyl-1H-pyrazol-4-yl)pyrimidin-2-yl)amino)-N-(3-cyanophenyl)-3-methoxybenzamide(Compound 116);
4-((5-chloro-4-(1-isopropyl-1H-pyrazol-4-yl)pyrimidin-2-yl)amino)-N-(4-cyanophenyl)-3-methoxybenzamide(Compound 117);
4-((5-chloro-4-(1-isopropyl-1H-pyrazol-4-yl)pyrimidin-2-yl)amino)-N-(2-fluorobenzyl)-3-methoxybenzamide(Compound 118);
4-((5-chloro-4-(1-isopropyl-1H-pyrazol-4-yl)pyrimidin-2-yl)amino)-N-(2-fluorobenzyl)-3-methoxybenzamide(Compound 119);
4-((5-chloro-4-(1-isopropyl-1H-pyrazol-4-yl)pyrimidin-2-yl)amino)-N-(4-fluorobenzyl)-3-methoxybenzamide(Compound 120);
4-((5-chloro-4-(1-isopropyl-1H-pyrazol-4-yl)pyrimidin-2-yl)amino)-3-methoxy-N-(o-tolyl)benzamide(Compound 121);
4-((5-chloro-4-(1-isopropyl-1H-pyrazol-4-yl)pyrimidin-2-yl)amino)-3-methoxy-N-(m-tolyl)benzamide(Compound 122);
4-((5-chloro-4-(1-isopropyl-1H-pyrazol-4-yl)pyrimidin-2-yl)amino)-3-methoxy-N-(p-tolyl)benzamide(Compound 123);
4-((5-chloro-4-(1-isopropyl-1H-pyrazol-4-yl)pyrimidin-2-yl)amino)-3-methoxy-N-(2-methylbenzyl)benzamide (Compound 124);
4-((5-chloro-4-(1-isopropyl-1H-pyrazol-4-yl)pyrimidin-2-yl)amino)-3-methoxy-N-(3-methylbenzyl)benzamide (Compound 125);
4-((5-chloro-4-(1-isopropyl-1H-pyrazol-4-yl)pyrimidin-2-yl)amino)-3-methoxy-N-(4-methylbenzyl)benzamide (Compound 126);
4-((5-chloro-4-(1-isopropyl-1H-pyrazol-4-yl)pyrimidin-2-yl)amino)-N-(4-(dimethylamino)phenyl)-3-methoxybenzamide (Compound 127);
4-((5-chloro-4-(1-isopropyl-1H-pyrazol-4-yl)pyrimidin-2-yl)amino)-N-(4-cyanobenzyl)-3-methoxybenzamide (Compound 128);
4-((5-chloro-4-(1-isopropyl-1H-pyrazol-4-yl)pyrimidin-2-yl)amino)-N-(2,3-dihydro-1H-inden-2-yl)-3-methoxybenzamide (Compound 129);
4-((5-chloro-4-(1-isopropyl-1H-pyrazol-4-yl)pyrimidin-2-yl)amino)-3-methoxy-N-((tetrahydrofuran-3-yl)methyl)benzamide (Compound 130);
4-((5-chloro-4-(1-isopropyl-1H-pyrazol-4-yl)pyrimidin-2-yl)amino)-N-(3-cyano-4-fluorophenyl)-3-methoxybenzamide(Compound 131);
N-(5-chloro-2-fluorophenyl)-4-((5-chloro-4-(1-isopropyl-1H-pyrazol-4-yl)pyrimidin-2-yl)amino)-3-methoxybenzamide (Compound 132);
4-((5-chloro-4-(1-isopropyl-1H-pyrazol-4-yl)pyrimidin-2-yl)amino)-N-(1-(4-fluorophenyl)ethyl)-3-methoxybenzamide (Compound 133);
4-((5-chloro-4-(1-isopropyl-1H-pyrazol-4-yl)pyrimidin-2-yl)amino)-N-(2,4-difluorobenzyl)-3-methoxybenzamide (Compound 134);
4-((5-chloro-4-(1-isopropyl-1H-pyrazol-4-yl)pyrimidin-2-yl)amino)-N-(3,5-difluorobenzyl)-3-methoxybenzamide (Compound 135);
4-((5-chloro-4-(1-isopropyl-1H-pyrazol-4-yl)pyrimidin-2-yl)amino)-3-methoxy-N-(2-methylpyridin-4-yl)benzamide (Compound 136);
4-((5-chloro-4-(1-isopropyl-1H-pyrazol-4-yl)pyrimidin-2-yl)amino)-N-isopropyl-3-methoxybenzamide(Compound 137);
4-((5-chloro-4-(1-isopropyl-1H-pyrazol-4-yl)pyrimidin-2-yl)amino)-N-(2-hydroxyethyl)-3-methoxybenzamide (Compound 138);
4-((5-chloro-4-(1-isopropyl-1H-pyrazol-4-yl)pyrimidin-2-yl)amino)-N,N-diethyl-3-methoxybenzamide (Compound 139);
4-((5-chloro-4-(1-isopropyl-1H-pyrazol-4-yl)pyrimidin-2-yl)amino)-3-methoxy-N-(2-methoxyethyl)benzamide (Compound 140);
4-((5-chloro-4-(1-isopropyl-1H-pyrazol-4-yl)pyrimidin-2-yl)amino)-N-(2-hydroxyethyl)-3-methoxy-N-methylbenzamide (Compound 141);
4-((5-chloro-4-(1-isopropyl-1H-pyrazol-4-yl)pyrimidin-2-yl)amino)-N-isopropyl-3-methoxy-N-methylbenzamide (Compound 142);
4-((5-chloro-4-(1-isopropyl-1H-pyrazol-4-yl)pyrimidin-2-yl)amino)-3-methoxy-N-methyl-N-(1-methylpiperidin-4-yl)benzamide (Compound 143);
4-((5-chloro-4-(1-isopropyl-1H-pyrazol-4-yl)pyrimidin-2-yl)amino)-3-methoxy-N-(1-methyl-1H-pyrazol-4-yl)benzamide (Compound 144);
4-((5-chloro-4-(1-isopropyl-1H-pyrazol-4-yl)pyrimidin-2-yl)amino)-N-(1-isopropyl-1H-pyrazol-4-yl)-3-methoxybenzamide (Compound 145);
4-((5-chloro-4-(1-isopropyl-1H-pyrazol-4-yl)pyrimidin-2-yl)amino)-N-(4-chlorobenzyl)-3-methoxybenzamide (Compound 146);
4-((5-chloro-4-(1-isopropyl-1H-pyrazol-4-yl)pyrimidin-2-yl)amino)-N-(3-chlorobenzyl)-3-methoxybenzamide (Compound 147);
4-((5-chloro-4-(1-isopropyl-1H-pyrazol-4-yl)pyrimidin-2-yl)amino)-N-(2-chlorobenzyl)-3-methoxybenzamide (Compound 148);
4-((5-chloro-4-(1-isopropyl-1H-pyrazol-4-yl)pyrimidin-2-yl)amino)-N-(2-chlorophenyl)-3-methoxybenzamide (Compound 149);
4-((5-chloro-4-(1-isopropyl-1H-pyrazol-4-yl)pyrimidin-2-yl)amino)-N-(3-chlorophenyl)-3-methoxybenzamide (Compound 150);
4-((5-chloro-4-(1-isopropyl-1H-pyrazol-4-yl)pyrimidin-2-yl)amino)-N-(4-chlorophenyl)-3-methoxybenzamide (Compound 151);
4-((5-chloro-4-(1-isopropyl-1H-pyrazol-4-yl)pyrimidin-2-yl)amino)-N-(3-hydroxybenzyl)-3-methoxybenzamide (Compound 152);
4-((5-chloro-4-(1-isopropyl-1H-pyrazol-4-yl)pyrimidin-2-yl)amino)-N-(4-hydroxybenzyl)-3-methoxybenzamide (Compound 153);
4-((5-chloro-4-(1-isopropyl-1H-pyrazol-4-yl)pyrimidin-2-yl)amino)-3-methoxy-N-(2-methoxybenzyl)benzamide (Compound 154);
4-((5-chloro-4-(1-isopropyl-1H-pyrazol-4-yl)pyrimidin-2-yl)amino)-3-methoxy-N-(pyridin-2-ylmethyl)benzamide (Compound 155);
4-((5-chloro-4-(1-isopropyl-1H-pyrazol-4-yl)pyrimidin-2-yl)amino)-3-methoxy-N-(pyridin-3-ylmethyl)benzamide (Compound 156);
4-((5-chloro-4-(1-isopropyl-1H-pyrazol-4-yl)pyrimidin-2-yl)amino)-3-methoxy-N-(pyridin-4-ylmethyl)benzamide (Compound 157);
4-((5-chloro-4-(1-isopropyl-1H-pyrazol-4-yl)pyrimidin-2-yl)amino)-N-(2-hydroxyphenyl)-3-methoxybenzamide (Compound 158);
4-((5-chloro-4-(1-isopropyl-1H-pyrazol-4-yl)pyrimidin-2-yl)amino)-N-(3-hydroxyphenyl)-3-methoxybenzamide (Compound 159);
4-((5-chloro-4-(1-isopropyl-1H-pyrazol-4-yl)pyrimidin-2-yl)amino)-N-(4-hydroxyphenyl)-3-methoxybenzamide (Compound 160);
4-((5-chloro-4-(1-isopropyl-1H-pyrazol-4-yl)pyrimidin-2-yl)amino)-3-methoxy-N-(2-methoxyphenyl)benzamide (Compound 161);
4-((5-chloro-4-(1-isopropyl-1H-pyrazol-4-yl)pyrimidin-2-yl)amino)-3-methoxy-N-(3-methoxyphenyl)benzamide (Compound 162);
4-((5-chloro-4-(1-isopropyl-1H-pyrazol-4-yl)pyrimidin-2-yl)amino)-3-methoxy-N-(4-methoxyphenyl)benzamide (Compound 163);
4-((5-chloro-4-(1-isopropyl-1H-pyrazol-4-yl)pyrimidin-2-yl)amino)-N-(2,3-dihydro-1H-inden-1-yl)-3-methoxybenzamide (Compound 164);
4-((5-chloro-4-(1-isopropyl-1H-pyrazol-4-yl)pyrimidin-2-yl)amino)-3-methoxy-N-(2-methoxybenzyl)-N-methylbenzamide (Compound 165);
4-((5-chloro-4-(1-isopropyl-1H-pyrazol-4-yl)pyrimidin-2-yl)amino)-3-methoxy-N-(2,2,6,6-tetramethylpiperidin-4-yl)benzamide (Compound 166);
4-((5-chloro-4-(1-isopropyl-1H-pyrazol-4-yl)pyrimidin-2-yl)amino)-N-(2,6-dimethylpiperidin-1-yl)-3-methoxybenzamide (Compound 167);
4-((5-chloro-4-(1-isopropyl-1H-pyrazol-4-yl)pyrimidin-2-yl)amino)-N-((6-chloropyridin-3-yl)methyl)-3-methoxybenzamide (Compound 168);
4-((5-chloro-4-(1-isopropyl-1H-pyrazol-4-yl)pyrimidin-2-yl)amino)-3-methoxy-N-methyl-N-(2-(pyridin-2-yl)ethyl)benzamide (Compound 169);
4-((5-chloro-4-(1-isopropyl-1H-pyrazol-4-yl)pyrimidin-2-yl)amino)-N-(3-(difluoromethoxy)phenyl)-3-methoxybenzamide (Compound 170);
4-((5-chloro-4-(1-isopropyl-1H-pyrazol-4-yl)pyrimidin-2-yl)amino)-N-(3-ethylphenyl)-3-methoxybenzamide (Compound 171);
4-((5-chloro-4-(1-isopropyl-1H-pyrazol-4-yl)pyrimidin-2-yl)amino)-N-(2-ethylphenyl)-3-methoxybenzamide (Compound 172);
4-((5-chloro-4-(1-isopropyl-1H-pyrazol-4-yl)pyrimidin-2-yl)amino)-N-(4-ethylphenyl)-3-methoxybenzamide (Compound 173);
N-benzyl-4-((5-chloro-4-(1-isopropyl-1H-pyrazol-4-yl)pyrimidin-2-yl)amino)-3-methoxy-N-methylbenzamide (Compound 174);
4-((5-chloro-4-(1-isopropyl-1H-pyrazol-4-yl)pyrimidin-2-yl)amino)-3-methoxy-N-methyl-N-(3-methylbenzyl)benzamide (Compound 175);
ethyl 3-(4-((5-chloro-4-(1-isopropyl-1H-pyrazol-4-yl)pyrimidin-2-yl)amino)-3-methoxybenzamido)propanoate (Compound 176);
N-benzyl-4-((5-chloro-4-(1-isopropyl-1H-pyrazol-4-yl)pyrimidin-2-yl)amino)-N-isopropyl-3-methoxybenzamide (Compound 177);
4-((5-chloro-4-(1-isopropyl-1H-pyrazol-4-yl)pyrimidin-2-yl)amino)-N-(3-isopropoxyphenyl)-3-methoxybenzamide(Compound 178);
4-((5-chloro-4-(1-isopropyl-1H-pyrazol-4-yl)pyrimidin-2-yl)amino)-3-methoxy-N-(1-phenylethyl)benzamide(Compound 179);
4-((5-chloro-4-(1-isopropyl-1H-pyrazol-4-yl)pyrimidin-2-yl)amino)-3-methoxy-N-(3-(trifluoromethyl)phenyl)benzamide (Compound 180);
4-((5-chloro-4-(1-isopropyl-1H-pyrazol-4-yl)pyrimidin-2-yl)amino)-3-methoxy-N-(4-(trifluoromethyl)phenyl)benzamide (Compound 181);
4-((5-chloro-4-(1-isopropyl-1H-pyrazol-4-yl)pyrimidin-2-yl)amino)-3-methoxy-N-(1-phenylpropyl)benzamide (Compound 182);
N-(4-aminobenzyl)-4-((5-chloro-4-(1-isopropyl-1H-pyrazol-4-yl)pyrimidin-2-yl)amino)-3-methoxybenzamide(Compound 183);
4-((5-chloro-4-(1-isopropyl-1H-pyrazol-4-yl)pyrimidin-2-yl)amino)-N-(2-fluoro-5-methylphenyl)-3-methoxybenzamide (Compound 184);
4-((5-chloro-4-(1-isopropyl-1H-pyrazol-4-yl)pyrimidin-2-yl)amino)-N-(2-fluoro-4-methylphenyl)-3-methoxybenzamide (Compound 185);
4-((5-chloro-4-(1-isopropyl-1H-pyrazol-4-yl)pyrimidin-2-yl)amino)-N-(4-fluoro-2-methylphenyl)-3-methoxybenzamide (Compound 186);
4-((5-chloro-4-(1-isopropyl-1H-pyrazol-4-yl)pyrimidin-2-yl)amino)-N-(4-fluoro-3-methylphenyl)-3-methoxybenzamide (Compound 187);
4-((5-chloro-4-(1-isopropyl-1H-pyrazol-4-yl)pyrimidin-2-yl)amino)-N-(3-fluoro-2-methylphenyl)-3-methoxybenzamide (Compound 188);
4-((5-chloro-4-(1-isopropyl-1H-pyrazol-4-yl)pyrimidin-2-yl)amino)-3-methoxy-N-(4-sulfamoylbenzyl)benzamide (Compound 189);
4-((5-chloro-4-(1-isopropyl-1H-pyrazol-4-yl)pyrimidin-2-yl)amino)-3-methoxy-N-(3-(methylsulfonamido)phenyl)benzamide (Compound 190);
4-((5-chloro-4-(1-isopropyl-1H-pyrazol-4-yl)pyrimidin-2-yl)amino)-3-methoxy-N-(3-sulfamoylphenyl)benzamide (Compound 191);
4-((5-chloro-4-(1-isopropyl-1H-pyrazol-4-yl)pyrimidin-2-yl)amino)-3-methoxy-N-(2-(methylsulfonyl)ethyl)benzamide (Compound 192);
methyl (4-((5-chloro-4-(1-isopropyl-1H-pyrazol-4-yl)pyrimidin-2-yl)amino)-3-methoxybenzoyl)-L-valinate (Compound 193);
methyl (4-((5-chloro-4-(1-isopropyl-1H-pyrazol-4-yl)pyrimidin-2-yl)amino)-3-methoxybenzoyl)-L-isoleucinate (Compound 194);
ethyl (4-((5-chloro-4-(1-isopropyl-1H-pyrazol-4-yl)pyrimidin-2-yl)amino)-3-methoxybenzoyl)glycinate (Compound 195);
N-(4-aminophenethyl)-4-((5-chloro-4-(1-isopropyl-1H-pyrazol-4-yl)pyrimidin-2-yl)amino)-3-methoxybenzamide(Compound 196);
N-(3-(1H-imidazol-1-yl)propyl)-4-((5-chloro-4-(1-isopropyl-1H-pyrazol-4-yl)pyrimidin-2-yl)amino)-3-methoxybenzamide (Compound 197);
4-((5-chloro-4-(1-isopropyl-1H-pyrazol-4-yl)pyrimidin-2-yl)amino)-N-(2,2-dimethoxyethyl)-3-methoxybenzamide (Compound 198);
methyl (4-((5-chloro-4-(1-isopropyl-1H-pyrazol-4-yl)pyrimidin-2-yl)amino)-3-methoxybenzoyl)-L-leucinate(Compound 199);
methyl (4-((5-chloro-4-(1-isopropyl-1H-pyrazol-4-yl)pyrimidin-2-yl)amino)-3-methoxybenzoyl)-L-prolinate (Compound 200);
4-((5-chloro-4-(1-isopropyl-1H-pyrazol-4-yl)pyrimidin-2-yl)amino)-3-methoxy-N-(4-methoxybenzyl)-N-methylbenzamide (Compound 201);
4-((5-chloro-4-(1-isopropyl-1H-pyrazol-4-yl)pyrimidin-2-yl)amino)-N-ethyl-3-methoxy-N-methylbenzamide (Compound 202);
4-((5-chloro-4-(1-isopropyl-1H-pyrazol-4-yl)pyrimidin-2-yl)amino)-3-methoxy-N-(4-methoxybenzyl)benzamide (Compound 203);
4-((5-chloro-4-(1-isopropyl-1H-pyrazol-4-yl)pyrimidin-2-yl)amino)-3-methoxy-N-(4-methoxy-3,5-dimethylphenyl)benzamide (Compound 204);
N-(3-aminobenzyl)-4-((5-chloro-4-(1-isopropyl-1H-pyrazol-4-yl)pyrimidin-2-yl)amino)-3-methoxybenzamide (Compound 205);
4-((5-chloro-4-(1-isopropyl-1H-pyrazol-4-yl)pyrimidin-2-yl)amino)-N-(2,3-dihydro-1H-inden-5-yl)-3-methoxybenzamide (Compound 206);
4-((5-chloro-4-(1-isopropyl-1H-pyrazol-4-yl)pyrimidin-2-yl)amino)-N-(2,3-dihydro-1H-inden-4-yl)-3-methoxybenzamide (Compound 207);
4-((5-chloro-4-(1-isopropyl-1H-pyrazol-4-yl)pyrimidin-2-yl)amino)-N-(2,3-dihydro-1H-inden-2-yl)-3-methoxy-N-methylbenzamide (Compound 208);
4-((5-chloro-4-(1-isopropyl-1H-pyrazol-4-yl)pyrimidin-2-yl)amino)-N-(2,3-dihydro-1H-inden-1-yl)-3-methoxy-N-methylbenzamide (Compound 209);
(4-((5-chloro-4-(1-isopropyl-1H-pyrazol-4-yl)pyrimidin-2-yl)amino)-3-methoxyphenyl)(4-(ethyl(methyl)amino)piperidin-1-yl)methanone (Compound 210) ;
N-(1-acetylindolin-5-yl)-4-((5-chloro-4-(1-isopropyl-1H-pyrazol-4-yl)pyrimidin-2-yl)amino)-3-methoxy-N-methylbenzamide (Compound 211);
N-(1-acetylindolin-5-yl)-4-((5-chloro-4-(1-isopropyl-1H-pyrazol-4-yl)pyrimidin-2-yl)amino)-3-methoxybenzamide (Compound 212);
ethyl 3-(4-((5-chloro-4-(1-isopropyl-1H-pyrazol-4-yl)pyrimidin-2-yl)amino)-3-methoxybenzamido)-4,4,4-trifluorobutanoate (Compound 213);
4-((5-chloro-4-(1-isopropyl-1H-pyrazol-4-yl)pyrimidin-2-yl)amino)-N-(2,3-dihydro-1H-inden-5-yl)-3-methoxy-N-methylbenzamide (Compound 214);
4-((5-chloro-4-(1-isopropyl-1H-pyrazol-4-yl)pyrimidin-2-yl)amino)-N-(2,3-dihydro-1H-inden-4-yl)-3-methoxy-N-methylbenzamide (Compound 215);
(4-((5-chloro-4-(1-isopropyl-1H-pyrazol-4-yl)pyrimidin-2-yl)amino)-3-methoxyphenyl)(4-(pyrrolidin-1-yl)piperidin-1-yl)methanone (Compound 216); and
[1,4'-bipiperidin]-1'-yl(4-((5-chloro-4-(1-isopropyl-1H-pyrazol-4-yl)pyrimidin-2-yl)amino)-3-methoxyphenyl)methanone (Compound 217).

In addition, the compound represented by Chemical Formula 1 according to the present disclosure is prepared by the methods illustrated in the following reaction schemes 1 (amide coupling), 2 (Suzuki reaction), and 3 (Buchwald reaction).

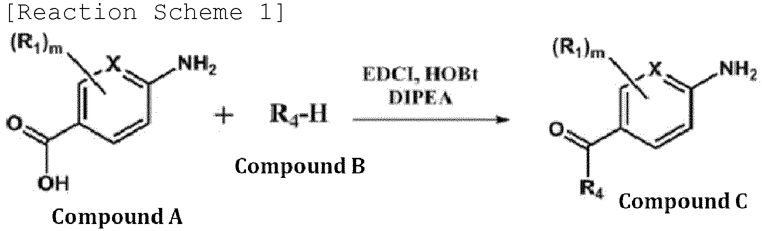

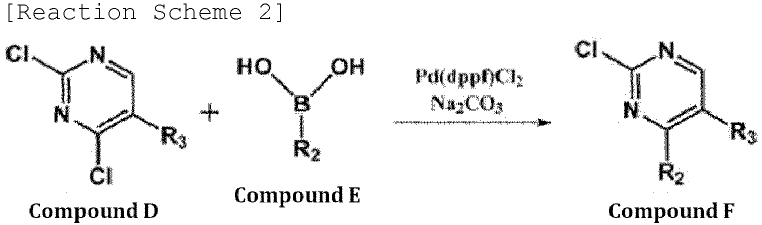

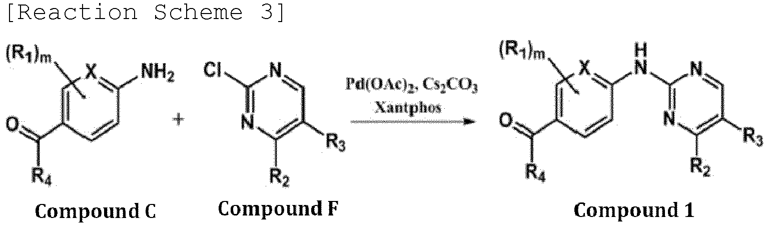

Through the reactions illustrated in Reaction Schemes 1, 2, and 3, the compound represented by Chemical Formula 1 is synthesized.

In Reaction Schemes 1, 2, and 3,
X, m, R₁, R₂, R₃, and R₄ are as defined in Chemical Formula 1.

Compounds A, B, D, and E used in Reaction Schemes 1, 2, and 3 can be purchased from Aldrich, Merck, TCI, and ACROS, respectively.

Below, a detailed description will be given of a method for preparing a compound represented by Chemical Formula 1 according to the present disclosure.

In a method for preparing a compound represented by Chemical Formula 1 according to the present disclosure,
the reaction condition set forth in Reaction Scheme 1 is to react compound A with compound B to form compound C.

Reaction Scheme 1 is a step where compound A and compound B are subjected to an amination reaction to give compound C. The reaction may be conducted without a base, but is generally achieved in the presence of a base useful for an amination reaction, for example, an organic base such as pyridine, triethylamine, diethylisopropylamine, etc., in dichloromethane, chloroform, tetrahydrofuran, diethylether, toluene, N, N-dimethylformamide, etc., which do not adversely affect the reaction. No particular limitations are imparted to the reaction temperature. However, the reaction may be generally conducted at cold to room temperature and preferably at room temperature.

Reaction Scheme 2 is a step where compound D is reacted with compound E to form compound F.

As illustrated in Reaction Scheme 2, compound D is reacted with compound E in the presence of a metal-catalyst to form compound F. For this reaction, palladium is typically used as a metal-catalyst. Tetrakistriphenylphosphine palladium(0) ((PPh₃)₄Pd), palladiumacetate(II)(Pd(OAc)₂), tris(dibenzylideneacetone)dipalladium(0) (Pd₂dba₃), bis(triphenylphosphine)palladium(II) chloride (PdCl₂(PPh₃)₂), and [1,1'-bis(diphenylphosphino)ferrocene]palladium(0) chloride (Pd(dppf)Cl₂) may be used. A base useful in this reaction may be exemplified by potassium carbonate, KOtBu, cesium carbonate, potassium phosphate, sodium hydroxide, triethylamine, etc. This reaction is carried out in a solvent which does not have any adverse effect on the reaction, such as purified water, toluene, tetrahydrofuran, dioxane, N,N-dimethylformamide, etc. No particular limitations are imparted to the reaction temperature. However, the reaction may be generally conducted at room to warm temperatures and preferably at warm temperatures.

Reaction Scheme 3 is a step where compound C, which is the product of Reaction Scheme 1, is reacted with compound F, which is the product of Reaction Scheme 2, in the presence of a metal-catalyst to afford compound 1. In this reaction, palladium is used as a typical metal-catalyst as exemplified by tetrakistriphenyl phosphine palladium(0) ((PPh₃)₄Pd), palladiumacetate(II) (Pd(OAc)₂), tris(dibenzylideneacetone)dipalladium(0) (Pd₂dba₃), bis(triphenylphosphine)palladium(II) chloride (PdCl₂(PPh₃)₂), and [1,1'-bis(diphenylphosphino)ferrocene]palladium(0) chloride (Pd(dppf)Cl₂). In the presence of the metal-catalyst, this reaction can be conducted without a ligand, but generally takes advantage of a ligand useful for the Buchwald reaction, such as triphenylphosphine ((PPh3)4), 1,2-bis(diphenylphosphino)propane (DPPP), 2,2'-bis(diphenylphosphino)-1,1'-binaphthyl (BINAP), xantphos, XPhos, etc. Examples of a base useful for this reaction include potassium carbonate, KOtBu, cesium carbonate, potassium phosphate, sodium hydroxide, and triethylamine. This reaction is carried out in a solvent which does not adversely affect the reaction, such as toluene, tetrahydrofuran, dioxane, N,N-dimethylformamide, etc. No particular limitations are imparted to the reaction temperature. Generally, the reaction temperature is set to be room temperature to a warm temperature, and preferably to be a warm temperature.

In this regard, the reaction according to an embodiment may be preferably carried out in a Pd(OAc)₂/Cs₂CO₃/Xantphos reaction condition or a ZnCl₂/Trifluoroacetic acid reaction condition, without limitations.

So long as it dissolves compounds A and B and materials used in the reactions, any solvent may be employed in the present disclosure. Examples of the solvent include: ether solvents such as dioxane, tetrahydrofuran (THF), ethylether, 1,2-dimethoxyethane, etc.; lower alcohols, such as methanol, ethanol, propanol, and butanol; dimethylformamide (DMF), dimethylsulfoxide (DMSO), dichloromethane (DCM), dichloroethane, and a combination thereof.

In addition, the reaction temperature is not particularly limited, but may preferably range from 0°C to 100°C, for example, may be room temperature. Furthermore, no particular limitations are imparted to the reaction time. For instance, the reaction may be carried out for 2 hours to 20 hours, and, in another embodiment, for 20 minutes to 8 hours.

The following terms have the following meanings unless otherwise indicated. Any undefined term has a meaning generally understood therefor in the art.

The terms "cyclic" and "ring" refer to alicyclic or aromatic groups that may or may not be substituted and/or heteroatom containing, and that may be monocyclic, bicyclic, or polycyclic.

The terms "halo" and "halogen" are used in the conventional sense to refer to a fluoro (F), chloro (Cl), bromo (Br), or iodo (I) substituent.

The term "alkyl", as used herein, refers to a linear or branched hydrocarbon, as exemplified by methyl, ethyl, n-propyl, isopropyl, n-butyl, isobutyl, tert-butyl, 1-methylpropyl, pentyl, hexyl, and the like.

The term "alkoxy" refers to a linear or branched, saturated hydrocarbon bound through a single terminal ether linkage. Examples include methoxy, ethoxy, propoxy, n-butoxy, tert-butoxy, 1-methylpropoxy, and so on.

The term "cycloalkyl" refers to a monofunctional, cyclic saturated hydrocarbon. Examples include cyclopropyl, cyclobutyl, cyclopentyl, cyclohexyl, cycloheptyl, cyclooctyl, bicyclo[3.1.1]heptyl, spiro[4.5]decyl, spiro[5.5]undexyl, adamantyl, and so on.

The term "aryl" means an aromatic substituent including at least one ring having a covalent pi electron system, such as phenyl, benzyl, naphthyl, anthryl, biphenyl, triphenyl, and the like.

The term "arylalkyl" refers to an aryl derivative of an alkyl group where "aryl" and "alkyl" are as defined above.

The term "heterocycloalkyl" refers to a monofunctional cyclic saturated hydrocarbon bearing at least one heteroatom, such as N, O, or S, as a ring member. There is a wide spectrum of heterocycloalkyls according to numbers and kinds of heteroatoms and numbers of carbon atoms, examples of which include aziridinyl, pyrrolidinyl, piperidinyl, oxopiperidinyl, morpholinyl, piperazinyl, oxopiperazinyl, morpholinyl, thiomorpholinyl, azepanyl, diazepanyl, oxazepanyl, thiazepanyl, dioxothiazepanyl, azocanyl, tetrahydrofuranyl, tetrahydropyranyl, oxazolidinyl, dioxanyl, and dioxolanyl.

The term "heteroaryl" refers to an aromatic ring compound bearing at least one heteroatom such as N, O, or S. There is a wide spectrum of heteroaryls according to numbers and kinds of heteroatoms contained in the ring moieties and numbers of carbon atoms, examples of which include pyridyl, pyrrolyl, pyrrolidinyl, pyridinyl, furanyl, quinolidinyl, indolyl, pyrimidinyl, imidazolyl, 1,2,4-triazolyl, tetrazolyl, pyranyl, thiophenyl, thiazolyl, dibenzothiophenyl, dibenzofuranyl, dibenzoselenophenyl, thiophenyl, benzofuranyl, benzothiophenyl, benzoselenophenyl, carbazolyl, indolocarbazolyl, pyridylindolyl, pyrrolodipyridinyl, pyrazolyl, imidazolyl, triazolyl, oxazolyl, thiazolyl, oxadiazolyl, oxatriazolyl, dioxazolyl, thiadiazolyl, pyridinyl, pyridazinyl, pyrazinyl, triazinyl, oxazinyl, oxathiazinyl, oxadiazinyl, indolyl, benzimidazolyl, indazolyl, indoxazinyl, benzoxazolyl, benzisoxazolyl, benzothiazolyl, quinolinyl, isoquinolinyl, cinnolinyl, quinazolinyl, quinoxalinyl, naphthyridinyl, phthalazinyl, pteridinyl, xanthenyl, acridinyl, phenazinyl, phenothiazinyl, phenoxazinyl, benzofuropyridinyl, furodipyridinyl, benzothienopyridinyl, thienodipyridinyl, benzoselenophenopyridinyl, and selenophenodipyridinyl.

Furthermore, the compound of the present disclosure may contain at least one asymmetric carbon atom and may exist in a racemic form or an optically active form. All of the compounds and diastereomers fall within the scope of the present disclosure.

As used herein, the term "pharmaceutically acceptable salt thereof" indicates a salt or complex of Chemical Formula 1 that retains a desired biological activity. Examples of the salt include, but are not limited to, acid addition salts formed with inorganic acids (e.g., hydrochloric acid, hydrobromic acid, sulfuric acid, phosphoric acid, nitric acid, etc.), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and polygalacturonic acid. The compound may also be administered in the form of a pharmaceutically acceptable quaternary salt. Among others, the salt includes chloride, bromide, iodide, -O-alkyl, toluene sulfonate, methylsulfonate, sulfonate, phosphate, or carboxylate (e.g., benzoate, succinate, acetate, glycolate, maleate, malate, fumarate, citrate, tartrate, ascorbate, cinnamate, mandelate, and diphenylacetate). The compound of Chemical Formula 1 according to the present disclosure is intended to encompass any salt, hydrate, solvate, and prodrug that can be prepared using typical methods.

The acid addition salts according to the present disclosure can be prepared using typical methods. For instance, a derivative of Chemical Formula 1 may be dissolved in an organic solvent, such as methanol, ethanol, acetone, dichloromethane, acetonitrile, etc., and added with an organic acid or an inorganic acid to form a precipitate which may be then filtered and dried to obtain an acid addition salt. Alternatively, the solvent and the excess of acid are evaporated at a reduced pressure and the residue is crystalized in an organic solvent to afford the acid addition salt.

In addition, a pharmaceutically acceptable metal salt may be prepared using a base. An alkali metal or alkaline earth metal salt may be obtained, for example, by dissolving a compound in an excess amount of alkali metal hydroxide or alkaline earth metal hydroxide solution, filtering out the undissolved salt, and then evaporating and drying the filtrate. As the metal salts, sodium, potassium or calcium salts are pharmaceutically suitable. Also, the corresponding silver salts may be obtained by reacting an alkali metal or alkaline earth metal salt with a proper silver salt (e.g., silver nitrate) .

Another aspect of the present disclosure pertains to a pharmaceutical composition including the compound represented by Chemical Formula 1 as an active ingredient for prevention or treatment of a neurodegenerative disease and a cancer. All the compounds represented by Chemical Formula 1 have inhibitory activity against LRRK2.

The neurodegenerative disease refers to a disease that causes various symptoms as degenerative changes due to gradual and progressive death of nerve cells appear in the central nervous system and may be selected from the group consisting of Parkinson's disease, Alzheimer's disease, Huntington's disease, Pick's disease, amyotrophic lateral sclerosis, prion disease, motor neuron disease, spinocerebellar ataxia, spinal muscular atrophy, Creutzfeldt-Jakob disease and alcohol-related dementia, but with no limitations thereto.

In addition, the cancer may be selected from the group consisting of kidney cancer, thyroid cancer, breast cancer, liver cancer, and brain cancer, but with no limitations thereto.

The pharmaceutical composition according to the present disclosure may be formulated into suitable dosage forms using a pharmaceutically acceptable carrier which is commonly used. The "pharmaceutically acceptable" refers to a composition that is physiologically acceptable and does not cause an allergic reaction or a similar reaction thereto, such as gastrointestinal disorder, dizziness, etc., when administered to humans. In addition, the composition may be formulated and used in the form of oral formulations such as powders, granules, tablets, capsules, suspensions, emulsions, syrups, aerosols, etc., external preparations, suppositories, and sterile injectable solutions according to a general method.

Carriers, excipients, and diluents that may be included in the composition may include lactose, dextrose, sucrose, sorbitol, mannitol, xylitol, erythritol, maltitol, starch, Arabic rubber, alginate, gelatin, calcium phosphate, calcium silicate, cellulose, methyl cellulose, microcrystalline cellulose, polyvinylpyrrolidone, water, methyl paraoxybenzoate, propyl paraoxybenzoate, talc, magnesium stearate, and mineral oil, but are not limited thereto. The formulations may be prepared by using a diluent or an excipient, such as a filler, a stabilizer, a binder, a disintegrating agent, a surfactant, etc., which are commonly used. Solid formulations for oral administration include a tablet, a pill, a powder, a granule, a capsule, and the like, and these solid formulations may be prepared by mixing at least one or more excipients, for example, starch, microcrystalline cellulose, sucrose or lactose, low-substituted hydroxypropyl cellulose, hypromellose, and the like with the compound of the present disclosure. Further, lubricants such as magnesium stearate and talc may also be used in addition to simple excipients. Liquid formulations for oral administration may correspond to suspensions, liquids for internal use, emulsions, syrups, and the like, and may include various excipients, for example, a humectant, a sweetener, an aromatic agent, a preservative, and the like, in addition to water and liquid paraffin which are commonly used as simple diluents. Formulations for parenteral administration include a sterile aqueous solution, a non-aqueous solution, a suspension, an emulsion, a lyophilizing agent, and a suppository. As the non-aqueous solution and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurin butter, glycerol, gelatin, and the like may be used. For preparation as formulations for parenteral administration, the pyrimidine derivative compound of Chemical Formula 1 or a pharmaceutically acceptable salt is sterilized and/or mixed with a preservative, a stabilizer, a wettable powder or emulsifier, an adjuvant such as salts and/or buffers for regulation of osmotic pressure, and other therapeutically useful materials in water to be prepared by a solution or suspension, and the prepared solution or suspension may be prepared by an ampoule or vial unit dose type.

The pharmaceutical composition comprising the compound of Chemical Formula 1 disclosed in the present disclosure as an active ingredient may be administered to mammals such as rats, livestock, and humans in various routes. All modes of administration may be contemplated and for example, the pharmaceutical composition may be administered by oral, rectal or intravenous, intramuscular, subcutaneous, intrauterine dural, or cerebrovascular injection. A dose may vary according to the age, sex, and body weight of a subject to be treated, a specific disease or pathology to be treated, the severity of disease or pathology, an administration time, an administration route, the absorption of a drug, distribution and excretion rate, types of other drugs to be used, judgment of prescribers, etc. The dose determination based on these factors is within a level of those skilled in the art, and in general, the dose is in the range of 0.01 mg/kg/day to about 2000 mg/kg/day. A more preferable dose is 1 mg/kg/day to 500 mg/kg/day. The administration may be performed once a day or several times a day. The dose does not limit the scope of the present disclosure in any way.

The pharmaceutical composition of the present disclosure may be used alone or in combination with surgery, hormone therapy, chemotherapy, and a biological response modulator, for prevention or treatment of neurodegenerative disease and cancer.

### Advantageous Effects of Invention

The present disclosure is drawn to a novel pyrimidine derivative and a composition comprising same for prevention or treatment of neurodegenerative disease and cancer. With excellent inhibitory activity against LRRK2 and ability to effectively cross the blood-brain barrier (BBB), the pyrimidine derivatives find advantageous applications in pharmaceutical compositions for prevention and treatment of neurodegenerative diseases and cancers.

### Best Mode for Carrying out the Invention

Hereinafter, embodiments of the disclosure will be described in detail. This disclosure may, however, be embodied in many different forms and should not be construed as limited to the illustrated embodiments set forth herein. Rather, these illustrated embodiments are provided so that this disclosure will be thorough and complete, and will fully convey the scope of the invention to those skilled in the art.

### <EXAMPLE 1. Synthesis and Physicochemical Characterization of Pyrimidine Derivatives>

Synthesis procedures for compounds 1 to 217 of the present disclosure are as follows.

### Compound 1. (4-((5-chloro-4-(1-methyl-1H-pyrazol-4-yl)pyrimidin-2-yl)amino)-3-methoxyphenyl)(morpholino)methanone

### 1) Synthesis of (4-amino-3-methoxyphenyl)(morpholino)methanone

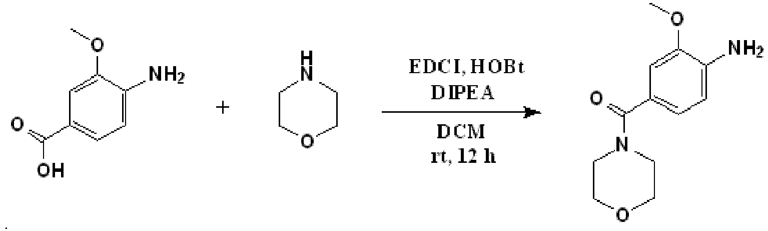

In a 25-mL round-bottom flask, 4-amino-3-methoxybenzoic acid (500 mg, 3 mmol), morpholine (0.31 mL, 3.6 mmol), EDCI (1-ethyl-3-(3-dimethylaminopropyl) carbodiimide HCl) (747 mg, 3.9 mmol), HOBt (hydroxybenzotriazole) (527 mg, 3.9 mmol), and DCM (dichloromethane) (10 mL) were put, followed by DIPEA (diisopropylethylamine) (1.05 mL, 6 mmol) at 0°C. Then, the mixture was stirred at room temperature for 12 hours. Ethyl acetate and a saturated aqueous NaCl solution were added, and the organic layer thus formed was dried (Mg₂SO₄), filtered, and concentrated. The crude product was dissolved in DCM, followed by isolation through column chromatography to afford the title compound as a pale yellow solid (634mg, 2.68 mmol) (yield 89.1%).

¹H NMR (300 MHz, Chloroform-d) δ 6.94 (d, J= 1.8 Hz, 1H), 6.85 (dd, J= 7.9, 1.8 Hz, 1H), 6.70 (d, J= 7.9 Hz, 1H), 3.87 (s, 3H), 3.56 - 3.75 (m, 8H).

### 2) Synthesis of 2,5-dichloro-4-(1-methyl-1H-pyrazol-4-yl)pyrimidine

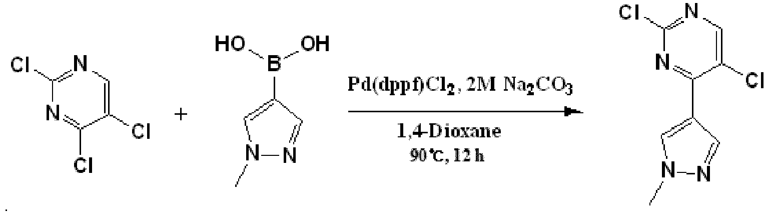

To a 5-mL microwave vial were added 2,4,5-trichloropyrimidine (0.63 mL, 5.5 mmol), (1-methyl-1H-pyrazol-4-yl) boronic acid (720 mg, 5.78 mmol), a 2M Na₂CO₃ solution (5.5 mL, 11 mmol), and dioxane (9 mL), and degassing was conducted twice. After addition of Pd(dppf)Cl₂ (1,1'-bis(diphenylphosphino)ferrocene]dichloropalladium(II)) (402 mg, 0.55 mmol), degassing was conducted one more time. The mixture was stirred at 90°C for 12 hours and allowed to pass through a Celite filter. After addition of ethyl acetate and water, the organic layer thus formed was dried (Mg₂SO₄), filtered, and concentrated. The crude product was dissolved in DCM, followed by isolation through column chromatography to afford the title compound as a pale yellow solid (820 mg, 3.58 mmol (yield 65%).

¹H NMR (300 MHz, Chloroform-d) δ 8.57 (s, 1H), 8.14 (s, 1H), 8.08 (s, 1H), 3.96 (s, 3H).

### 3) Synthesis of (4-((5-chloro-4-(1-methyl-1H-pyrazol-4-yl)pyrimidin-2-yl)amino)-3-methoxyphenyl)(morpholino)methanone

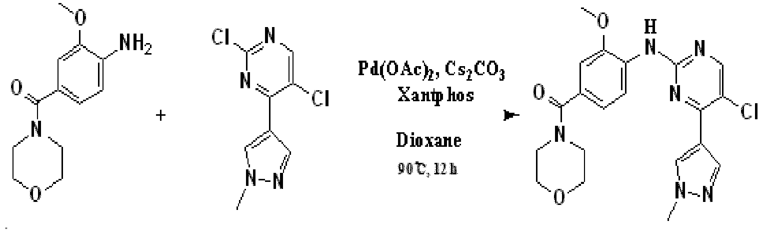

To a 5-mL microwave vial were added (4-amino-3-methoxyphenyl) (morpholino)methanone (120 mg, 0.525 mmol), 2,5-dichloro-4-(1-methyl-1H-pyrazol-4-yl)pyrimidine (118 mg, 0.5 mmol), xantphos (4,5-bis(diphenylphosphino)-9,9-dimethylxanthene) (29 mg, 0.05 mmol), cesium carbonate (326 mg, 1 mmol), and dioxane (2 mL) which were then degassed three times. Then, Pd(OAc)₂ (11 mg, 0.05 mmol) was added and degassing was conducted one more time. The mixture was stirred at 90°C for 12 hours. After addition of ethyl acetate and water, the organic layer thus formed was dried (Mg₂SO4), filtered, and concentrated. The crude product was dissolved in DCM (dichloromethan), followed by isolation through column chromatography to afford the title compound as a white solid (163mg, 0.38 mmol) (yield 76%) .

¹H NMR (300 MHz, Chloroform-d) δ 8.50 (d, J = 8.8 Hz, 1H), 8.35 - 8.38 (m, 3H), 8.13 (s, 1H), 7.02 - 7.08 (m, 2H), 4.01 (s, 3H), 3.96 (s, 3H), 3.60 - 3.80 (m, 8H) .

The following compounds were synthesized in similar manner to that for compound 1 in Example 1, and the chemical structures and NMR spectrum data of the synthesized compounds are summarized in Table 1, below.
(4-((5-chloro-4-(1-methyl-1H-pyrazol-4-yl)pyrimidin-2-yl)amino)-3-methoxyphenyl)(morpholino)methanone (Compound 1);
(4-((5-chloro-4-(1-methyl-1H-pyrazol-4-yl)pyrimidin-2-yl)amino)-3-methoxyphenyl)(4-morpholinopiperidin-1-yl)methanone (Compound 2);
1-(4-(5-chloro-2-((2-methoxy-4-(morpholine-4-carbonyl)phenyl)amino)pyrimidin-4-yl)-1H-pyrazol-1-yl)ethan-1-one (Compound 3);
(4-((5-chloro-4-(1-(2-hydroxyethyl)-1H-pyrazol-4-yl)pyrimidin-2-yl)amino)-3-methoxyphenyl)(morpholino)methanone (Compound 4);
3-(4-(5-chloro-2-((2-methoxy-4-(morpholine-4-carbonyl)phenyl)amino)pyrimidin-4-yl)-1H-pyrazol-1-yl)propanenitrile (Compound 5);
2-(4-(5-chloro-2-((2-methoxy-4-(morpholine-4-carbonyl)phenyl)amino)pyrimidin-4-yl)-1H-pyrazol-1-yl)acetonitrile (Compound 6);
3-(4-(5-chloro-2-((2-methoxy-4-(morpholine-4-carbonyl)phenyl)amino)pyrimidin-4-yl)-1H-pyrazol-1-yl)butanenitrile (Compound 7);
(4-((5-chloro-4-(1-(2-morpholinoethyl)-1H-pyrazol-4-yl)pyrimidin-2-yl)amino)-3-methoxyphenyl)(morpholino)methanone (Compound 8);
methyl 2-(4-(5-chloro-2-((2-methoxy-4-(morpholine-4-carbonyl)phenyl)amino)pyrimidin-4-yl)-1H-pyrazol-1-yl)acetate (Compound 9);
(4-((5-chloro-4-(1-(methylsulfonyl)-1H-pyrazol-4-yl)pyrimidin-2-yl)amino)-3-methoxyphenyl)(morpholino)methanone (Compound 10);
(4-((5-chloro-4-(1-(pyridin-2-yl)-1H-pyrazol-4-yl)pyrimidin-2-yl)amino)-3-methoxyphenyl)(morpholino)methanone (Compound 11);
(4-((5-chloro-4-(1-(pyridin-3-yl)-1H-pyrazol-4-yl)pyrimidin-2-yl)amino)-3-methoxyphenyl)(morpholino)methanone (Compound 12);
(4-((5-chloro-4-(1-(pyridin-4-yl)-1H-pyrazol-4-yl)pyrimidin-2-yl)amino)-3-methoxyphenyl)(morpholino)methanone (Compound 13);
(4-((5-chloro-4-(1-phenyl-1H-pyrazol-4-yl)pyrimidin-2-yl)amino)-3-methoxyphenyl)(morpholino)methanone (Compound 14);
(4-((5-chloro-4-(1-(difluoromethyl)-1H-pyrazol-4-yl)pyrimidin-2-yl)amino)-3-methoxyphenyl)(morpholino)methanone (Compound 15);
(4-((5-chloro-4-(1-(methoxymethyl)-1H-pyrazol-4-yl)pyrimidin-2-yl)amino)-3-methoxyphenyl)(morpholino)methanone (Compound 16);
(4-((5-chloro-4-(1-(4-methoxybenzyl)-1H-pyrazol-4-yl)pyrimidin-2-yl)amino)-3-methoxyphenyl)(morpholino)methanone (Compound 17);
(4-((5-chloro-4-(1H-pyrazol-4-yl)pyrimidin-2-yl)amino)-3-methoxyphenyl)(morpholino)methanone (Compound 18);
(4-((5-chloro-4-(1-methyl-1H-pyrazol-4-yl)pyrimidin-2-yl)amino)-3-methoxyphenyl)(4-(4-methylpiperazin-1-yl)piperidin-1-yl)methanone (Compound 19);
(4-((5-chloro-4-(1-(tetrahydro-2H-pyran-4-yl)-1H-pyrazol-4-yl)pyrimidin-2-yl)amino)-3-methoxyphenyl)(morpholino)methanone (Compound 20);
4-((5-chloro-4-(1-methyl-1H-pyrazol-4-yl)pyrimidin-2-yl)amino)-3-methoxy-N-(2-methoxyethyl)-N-methylbenzamide (Compound 21);
(4-((5-chloro-4-(1-methyl-1H-pyrazol-4-yl)pyrimidin-2-yl)amino)-3-methoxyphenyl)(pyrrolidin-1-yl)methanone (Compound 22);
4-((5-chloro-4-(1-methyl-1H-pyrazol-4-yl)pyrimidin-2-yl)amino)-N-ethyl-3-methoxy-N-(2-methoxyethyl)benzamide(Compound 23);
4-((5-chloro-4-(1-methyl-1H-pyrazol-4-yl)pyrimidin-2-yl)amino)-N-(1-hydroxy-2-methylpropan-2-yl)-3-methoxybenzamide (Compound 24);
N-(tert-butyl)-4-((5-chloro-4-(1-methyl-1H-pyrazol-4-yl)pyrimidin-2-yl)amino)-3-methoxybenzamide(Compound 25);
4-((5-chloro-4-(1-methyl-1H-pyrazol-4-yl)pyrimidin-2-yl)amino)-N-(2-hydroxy-2-methylpropyl)-3-methoxybenzamide (Compound 26);
3-(4-(5-chloro-2-((2-methoxy-4-(morpholine-4-carbonyl)phenyl)amino)pyrimidin-4-yl)-1H-pyrazol-1-yl)-3-methylbutanenitrile (Compound 27);
(4-((5-chloro-4-(1-(2-methoxyethyl)-1H-pyrazol-4-yl)pyrimidin-2-yl)amino)-3-methoxyphenyl)(morpholino)methanone (Compound 28);
(3-methoxy-4-((4-(1-(tetrahydro-2H-pyran-4-yl)-1H-pyrazol-4-yl)-5-(trifluoromethyl)pyrimidin-2-yl)amino)phenyl)(morpholino)methanone (Compound 29);
4-((5-chloro-4-(1-methyl-1H-pyrazol-4-yl)pyrimidin-2-yl)amino)-N-(4,4-difluorocyclohexyl)-3-methoxybenzamide (Compound 30);
(3-methoxy-4-((4-(1-methyl-1H-pyrazol-4-yl)-5-(trifluoromethyl)pyrimidin-2-yl)amino)phenyl)(4-(4-methylpiperazin-1-yl)piperidin-1-yl)methanone (Compound 31);
(4-((5-chloro-4-(1-methyl-1H-pyrazol-4-yl)pyrimidin-2-yl)amino)-3-methoxyphenyl)(2-oxa-7-azaspiro[3.5]nonan-7-yl)methanone (Compound 32);
(4-((5-chloro-4-(1-methyl-1H-pyrazol-4-yl)pyrimidin-2-yl)amino)-3-methoxyphenyl) (1-oxa-6-azaspiro[3.3]heptan-6-yl)methanone (Compound 33);
(4-((5-chloro-4-(1-methyl-1H-pyrazol-4-yl)pyrimidin-2-yl)amino)-3-methoxyphenyl)(4-isopropylpiperazin-1-yl)methanone (Compound 34);
(4-((5-chloro-4-(1-(tetrahydro-2H-pyran-4-yl)-1H-pyrazol-4-yl)pyrimidin-2-yl)amino)-3-methoxyphenyl)(2-oxa-7-azaspiro[3.5]nonan-7-yl)methanone (Compound 35);
(3-methoxy-4-((4-(1-methyl-1H-pyrazol-4-yl)-5-(trifluoromethyl)pyrimidin-2-yl)amino)phenyl)(morpholino)methanone (Compound 36);
(4-((5-chloro-4-(1-isopropyl-1H-pyrazol-4-yl)pyrimidin-2-yl)amino)-3-methoxyphenyl)(morpholino)methanone (Compound 37);
4-((5-chloro-4-(1-methyl-1H-pyrazol-4-yl)pyrimidin-2-yl)amino)-3-methoxy-N-(1-methylpiperidin-4-yl)benzamide (Compound 38);
(4-((5-chloro-4-(1-methyl-1H-pyrazol-4-yl)pyrimidin-2-yl)amino)-3-methoxyphenyl)(4-(2-hydroxypropan-2-yl)piperidin-1-yl)methanone (Compound 39);
(4-((5-chloro-4-(1-methyl-1H-pyrazol-4-yl)pyrimidin-2-yl)amino)-3-methoxyphenyl)(2-oxa-6-azaspiro[3.3]heptan-6-yl)methanone (Compound 40);
(4-((5-chloro-4-(1-methyl-1H-pyrazol-4-yl)pyrimidin-2-yl)amino)-3-methoxyphenyl)(4,4-difluoropiperidin-1-yl)methanone (Compound 41);
(4-((5-chloro-4-(1-methyl-1H-pyrazol-4-yl)pyrimidin-2-yl)amino)-3-methoxyphenyl)(2,2-dimethylmorpholino)methanone (Compound 42);
(4-((5-chloro-4-(1-methyl-1H-pyrazol-4-yl)pyrimidin-2-yl)amino)-3-methoxyphenyl)(3-morpholinoazetidin-1-yl)methanone (Compound 43);
1-(4-(4-((5-chloro-4-(1-methyl-1H-pyrazol-4-yl)pyrimidin-2-yl)amino)-3-methoxybenzoyl)piperazin-1-yl)ethan-1-one(Compound 44);
(4-isopropylpiperazin-1-yl)(3-methoxy-4-((4-(1-methyl-1H-pyrazol-4-yl)-5-(trifluoromethyl)pyrimidin-2-yl)amino)phenyl)methanone (Compound 45);
(2-fluoro-5-methoxy-4-((4-(1-methyl-1H-pyrazol-4-yl)-5-(trifluoromethyl)pyrimidin-2-yl)amino)phenyl)(morpholino)methanone (Compound 46);
(3-methoxy-4-((4-(1-methyl-1H-pyrazol-4-yl)-5-(trifluoromethyl)pyrimidin-2-yl)amino)phenyl)(4-phenylpiperazin-1-yl)methanone (Compound 47);
(2-fluoro-5-methoxy-4-((4-(1-methyl-1H-pyrazol-4-yl)-5-(trifluoromethyl)pyrimidin-2-yl)amino)phenyl)(4-isopropylpiperazin-1-yl)methanone (Compound 48);
(4-((5-chloro-4-(1-isopropyl-1H-pyrazol-4-yl)pyrimidin-2-yl)amino)-2-fluoro-5-methoxyphenyl)(morpholino)methanone (Compound 49);
(4-((5-chloro-4-(1-methyl-1H-pyrazol-4-yl)pyrimidin-2-yl)amino)-3-methoxyphenyl)(4- (oxetan-3-yl)piperazin-1-yl)methanone (Compound 50);
((1R,5S)-8-oxa-3-azabicyclo[3.2.1]octan-3-yl)(4-((5-chloro-4-(1-methyl-1H-pyrazol-4-yl)pyrimidin-2-yl)amino)-3-methoxyphenyl)methanone (Compound 51);
(4-((5-chloro-4-(1-ethyl-1H-pyrazol-4-yl)pyrimidin-2-yl)amino)-3-methoxyphenyl)(pyridin-4-yl)methanone (Compound 52);
(4-((5-chloro-4-(1-propyl-1H-pyrazol-4-yl)pyrimidin-2-yl)amino)-3-methoxyphenyl)(pyridin-4-yl)methanone (Compound 53);
(4-((5-chloro-4-(1-isopropyl-1H-pyrazol-4-yl)pyrimidin-2-yl)amino)-3-methoxyphenyl)(pyridin-4-yl)methanone (Compound 54);
(4-((5-chloro-4-(1-methyl-1H-pyrazol-4-yl)pyrimidin-2-yl)amino)-3-methoxyphenyl)(pyridin-4-yl)methanone (Compound 55);
(4-((5-chloro-4-(1-ethyl-1H-pyrazol-4-yl)pyrimidin-2-yl)amino)-3-methoxyphenyl)(morpholino)methanone (Compound 56);
(4-((5-chloro-4-(1-propyl-1H-pyrazol-4-yl)pyrimidin-2-yl)amino)-3-methoxyphenyl)(morpholino)methanone (Compound 57);
(4-((5-chloro-4-(1-cyclopropyl-1H-pyrazol-4-yl)pyrimidin-2-yl)amino)-3-methoxyphenyl)(morpholino)methanone (Compound 58);
(4-((4-(1-(tert-butyl)-1H-pyrazol-4-yl)-5-chloropyrimidin-2-yl)amino)-3-methoxyphenyl)(morpholino)methanone (Compound 59);
4-((5-chloro-4-(1-isopropyl-1H-pyrazol-4-yl)pyrimidin-2-yl)amino)-3-methoxy-N-(1-methylpiperidin-4-yl)benzamide (Compound 60);
(4-((5-chloro-4-(1-methyl-1H-pyrazol-4-yl)pyrimidin-2-yl)amino)-3-methoxyphenyl)(4-methylpiperazin-1-yl)methanone (Compound 61);
(4-((5-chloro-4-(1-isopropyl-1H-pyrazol-4-yl)pyrimidin-2-yl)amino)-3-methoxyphenyl)(4-methylpiperazin-1-yl)methanone (Compound 62);
(4-((4-(1-isopropyl-1H-pyrazol-4-yl)-5-(trifluoromethyl)pyrimidin-2-yl)amino)-3-methoxyphenyl)(morpholino)methanone (Compound 63);
(4-((5-chloro-4-(1-isopropyl-1H-pyrazol-4-yl)pyrimidin-2-yl)amino)-3-methoxyphenyl)(4-isopropylpiperazin-1-yl)methanone (Compound 64);
(2-fluoro-4-((4-(1-isopropyl-1H-pyrazol-4-yl)-5-(trifluoromethyl)pyrimidin-2-yl)amino)-5-methoxyphenyl)(morpholino)methanone (Compound 65);
(4-((5-chloro-4-(1-isopropyl-1H-pyrazol-4-yl)pyrimidin-2-yl)amino)-3-methoxyphenyl)(tetrahydro-2H-pyran-4-yl)methanone (Compound 66);
(4-((5-chloro-4-(1-methyl-1H-pyrazol-4-yl)pyrimidin-2-yl)amino)-3-methoxyphenyl)(4-methyltetrahydro-2H-pyran-4-yl)methanone (Compound 67);
(4-((5-chloro-4-(1-isopropyl-1H-pyrazol-4-yl)pyrimidin-2-yl)amino)-3-methoxyphenyl)(4-methyltetrahydro-2H-pyran-4-yl)methanone (Compound 68);
(4-((5-chloro-4-(1-methyl-1H-pyrazol-4-yl)pyrimidin-2-yl)amino)-2-fluoro-5-methoxyphenyl)(morpholino)methanone (Compound 69);
(4-((5-chloro-4-(1-isopropyl-1H-pyrazol-4-yl)pyrimidin-2-yl)amino)-3-methoxyphenyl)(4-(2-hydroxypropan-2-yl)piperidin-1-yl)methanone (Compound 70);
(4-((5-chloro-4-(1-isopropyl-1H-pyrazol-4-yl)pyrimidin-2-yl)amino)-3-methoxyphenyl)(2-oxa-7-azaspiro[3.5]nonan-7-yl)methanone (Compound 71);
(4-((5-chloro-4-(1-isopropyl-1H-pyrazol-4-yl)pyrimidin-2-yl)amino)-3-methoxyphenyl) (4-(oxetan-3-yl)piperazin-1-yl)methanone (Compound 72);
(4-((5-chloro-4-(1-isopropyl-1H-pyrazol-4-yl)pyrimidin-2-yl)amino)-3-methoxyphenyl)(3-morpholinoazetidin-1-yl)methanone (Compound 73);
(4-((5-chloro-4-(1-isopropyl-1H-pyrazol-4-yl)pyrimidin-2-yl)amino)-3-methoxyphenyl)(morpholino)methanethione (Compound 74);
(4-((5-chloro-4-(1-isopropyl-1H-pyrazol-4-yl)pyrimidin-2-yl)amino)-3-methoxyphenyl)(1-isopropylpiperidin-4-yl)methanone (Compound 75);
(4-((5-chloro-4-(1-isopropyl-1H-pyrazol-4-yl)pyrimidin-2-yl)amino)-3-methoxyphenyl)(4-methoxypiperidin-1-yl)methanone (Compound 76);
(4-((5-chloro-4-(1-isopropyl-1H-pyrazol-4-yl)pyrimidin-2-yl)amino)-3-methoxyphenyl)(3-oxa-9-azaspiro[5.5]undecan-9-yl)methanone (Compound 77);
1-(4-(4-((5-chloro-4-(1-isopropyl-1H-pyrazol-4-yl)pyrimidin-2-yl)amino)-3-methoxybenzoyl)piperazin-1-yl)ethan-1-one(Compound 78);
(4-((5-chloro-4-(1-isopropyl-1H-pyrazol-4-yl)pyrimidin-2-yl)amino)-3-methoxyphenyl)(4-(methylsulfonyl)piperidin-1-yl)methanone (Compound 79);
4-((5-chloro-4-(1-isopropyl-1H-pyrazol-4-yl)pyrimidin-2-yl)amino)-3-methoxy-N-(tetrahydro-2H-pyran-4-yl)benzamide(Compound 80);
N-(1-(2-(o-tolyl)quinolin-3-yl)ethyl)-6,7-dihydro-5H-pyrrolo[2,3-d]pyrimidin-4-amine (Compound 81);
4-((5-chloro-4-(1-isopropyl-1H-pyrazol-4-yl)pyrimidin-2-yl)amino)-N-cyclopropyl-3-methoxybenzamide(Compound 82);
(4-((5-chloro-4-(1-isopropyl-1H-pyrazol-4-yl)pyrimidin-2-yl)amino)-3-methoxyphenyl)(3-hydroxyazetidin-1-yl)methanone (Compound 83);
(4-((5-chloro-4-(1-isopropyl-1H-pyrazol-4-yl)pyrimidin-2-yl)amino)-3-methoxyphenyl)(1,1-dioxidothiomorpholino)methanone (Compound 84);
(4-((4-(1-(tert-butyl)-1H-pyrazol-4-yl)-5-chloropyrimidin-2-yl)amino)-3-methoxyphenyl)(4-methylpiperazin-1-yl)methanone (Compound 85);
(S)-(4-((5-chloro-4-(1-isopropyl-1H-pyrazol-4-yl)pyrimidin-2-yl)amino)-3-methoxyphenyl)(2-(hydroxymethyl)pyrrolidin-1-yl)methanone (Compound 86);
(4-((5-chloro-4-(1-isopropyl-1H-pyrazol-4-yl)pyrimidin-2-yl)amino)-3-methoxyphenyl)(hexahydropyrrolo[1,2-a]pyrazin-2(1H)-yl)methanone (Compound 87);
(4-((4-(1-(tert-butyl)-1H-pyrazol-4-yl)-5-chloropyrimidin-2-yl)amino)-3-methoxyphenyl)(4-isopropylpiperazin-1-yl)methanone (Compound 88);
((1R,5S)-8-oxa-3-azabicyclo[3.2.1]octan-3-yl)(4-((5-chloro-4-(1-isopropyl-1H-pyrazol-4-yl)pyrimidin-2-yl)amino)-3-methoxyphenyl)methanone (Compound 89);
(4-((5-chloro-4-(1-isopropyl-1H-pyrazol-4-yl)pyrimidin-2-yl)amino)-3-methoxyphenyl)(2-oxa-6-azaspiro[3.3]heptan-6-yl)methanone (Compound 90);
4-((5-chloro-4-(1-isopropyl-1H-pyrazol-4-yl)pyrimidin-2-yl)amino)-N-((1r,4r)-4-hydroxycyclohexyl)-3-methoxybenzamide(Compound 91);
(6-((5-chloro-4-(1-isopropyl-1H-pyrazol-4-yl)pyrimidin-2-yl)amino)pyridin-3-yl)(morpholino)methanone (Compound 92);
4-((5-chloro-4-(1-isopropyl-1H-pyrazol-4-yl)pyrimidin-2-yl)amino)-N-(4-(dimethylamino)cyclohexyl)-3-methoxybenzamide(Compound 93);
(4-((5-chloro-4-(1-isopropyl-1H-pyrazol-4-yl)pyrimidin-2-yl)amino)-3-methoxyphenyl)(4-(dimethylamino)piperidin-1-yl)methanone (Compound 94);
((1R,5S)-3-oxa-8-azabicyclo[3.2.1]octan-8-yl)(4-((5-chloro-4-(1-isopropyl-1H-pyrazol-4-yl)pyrimidin-2-yl)amino)-3-methoxyphenyl)methanone (Compound 95);
4-((4-(1-(tert-butyl)-1H-pyrazol-4-yl)-5-chloropyrimidin-2-yl)amino)-3-methoxy-N-(1-methylpiperidin-4-yl)benzamide(Compound 96);
(R)-(4-((5-chloro-4-(1-isopropyl-1H-pyrazol-4-yl)pyrimidin-2-yl)amino)-3-methoxyphenyl)(3-hydroxypyrrolidin-1-yl)methanone (Compound 97);
(4-((5-chloro-4-(1-isopropyl-1H-pyrazol-4-yl)pyrimidin-2-yl)amino)-3-methoxyphenyl)(5-methylhexahydropyrrolo[3,4-c]pyrrol-2(1H)-yl)methanone (Compound 98);
4-((5-chloro-4-(1-isopropyl-1H-pyrazol-4-yl)pyrimidin-2-yl)amino)-N-(1-isopropylpiperidin-4-yl)-3-methoxybenzamide (Compound 99);
2-(4-((5-chloro-4-(1-isopropyl-1H-pyrazol-4-yl)pyrimidin-2-yl)amino)-3-methoxybenzoyl)hexahydropyrrolo[1,2-a]pyrazin-6(2H)-one (Compound 100);
4-((5-chloro-4-(1-isopropyl-1H-pyrazol-4-yl)pyrimidin-2-yl)amino)-3-methoxy-N,N-dimethylbenzamide(Compound 101);
(4-((5-chloro-4-(1-isopropyl-1H-pyrazol-4-yl)pyrimidin-2-yl)amino)-2-fluoro-5-methoxyphenyl)(4-methylpiperazin-1-yl) methanethione (Compound 102);
(4-((5-chloro-4-(1-isopropyl-1H-pyrazol-4-yl)pyrimidin-2-yl)amino)-3-methoxyphenyl)(4-methylpiperazin-1-yl) methanethione (Compound 103);
(4-((5-chloro-4-(1-isopropyl-1H-pyrazol-4-yl)pyrimidin-2-yl)amino)-2-fluoro-5-methoxyphenyl)(4-methylpiperazin-1-yl)methanone (Compound 104);
(4-((5-chloro-4-(1-isopropyl-1H-pyrazol-4-yl)pyrimidin-2-yl)amino)-3-(difluoromethoxy)phenyl)(morpholino)methanone (Compound 105);
(4-((5-chloro-4-(1-isopropyl-1H-pyrazol-4-yl)pyrimidin-2-yl)amino)-2-fluoro-5-methoxyphenyl)(morpholino) methanethione (Compound 106);
(5-chloro-4-((5-chloro-4-(1-isopropyl-1H-pyrazol-4-yl)pyrimidin-2-yl)amino)-2-fluorophenyl)(4-methylpiperazin-1-yl)methanone (Compound 107);
(4-((5-chloro-4-(1-isopropyl-1H-pyrazol-4-yl)pyrimidin-2-yl)amino)-3-(difluoromethoxy)phenyl) (4-methylpiperazin-1-yl)methanone (Compound 108);
(4-((5-chloro-4-(1-isopropyl-1H-pyrazol-4-yl)pyrimidin-2-yl)amino)-3-methylphenyl)(4-methylpiperazin-1-yl)methanone (Compound 109);
(4-((5-chloro-4-(1-isopropyl-1H-pyrazol-4-yl)pyrimidin-2-yl)amino)-2-fluoro-5-methoxyphenyl)(4-(dimethylamino)piperidin-1-yl)methanone (Compound 110);
(4-((5-chloro-4-(1-isopropyl-1H-pyrazol-4-yl)pyrimidin-2-yl)amino)-2-fluoro-5-methoxyphenyl)(4-(dimethylamino)piperidin-1-yl)methanone (Compound 111);
4-((5-chloro-4-(1-isopropyl-1H-pyrazol-4-yl)pyrimidin-2-yl)amino)-3-methoxy-N-phenylbenzamide(Compound 112);
4-((5-chloro-4-(1-isopropyl-1H-pyrazol-4-yl)pyrimidin-2-yl)amino)-N-(2-fluorophenyl)-3-methoxybenzamide(Compound 113);
4-((5-chloro-4-(1-isopropyl-1H-pyrazol-4-yl)pyrimidin-2-yl)amino)-N-(3-fluorophenyl)-3-methoxybenzamide(Compound 114);
4-((5-chloro-4-(1-isopropyl-1H-pyrazol-4-yl)pyrimidin-2-yl)amino)-N-(4-fluorophenyl)-3-methoxybenzamide(Compound 115);
4-((5-chloro-4-(1-isopropyl-1H-pyrazol-4-yl)pyrimidin-2-yl)amino)-N-(3-cyanophenyl)-3-methoxybenzamide(Compound 116);
4-((5-chloro-4-(1-isopropyl-1H-pyrazol-4-yl)pyrimidin-2-yl)amino)-N-(4-cyanophenyl)-3-methoxybenzamide(Compound 117);
4-((5-chloro-4-(1-isopropyl-1H-pyrazol-4-yl)pyrimidin-2-yl)amino)-N-(2-fluorobenzyl)-3-methoxybenzamide(Compound 118);
4-((5-chloro-4-(1-isopropyl-1H-pyrazol-4-yl)pyrimidin-2-yl)amino)-N-(2-fluorobenzyl)-3-methoxybenzamide(Compound 119);
4-((5-chloro-4-(1-isopropyl-1H-pyrazol-4-yl)pyrimidin-2-yl)amino)-N-(4-fluorobenzyl)-3-methoxybenzamide(Compound 120);
4-((5-chloro-4-(1-isopropyl-1H-pyrazol-4-yl)pyrimidin-2-yl)amino)-3-methoxy-N-(o-tolyl)benzamide(Compound 121);
4-((5-chloro-4-(1-isopropyl-1H-pyrazol-4-yl)pyrimidin-2-yl)amino)-3-methoxy-N-(m-tolyl)benzamide(Compound 122);
4-((5-chloro-4-(1-isopropyl-1H-pyrazol-4-yl)pyrimidin-2-yl)amino)-3-methoxy-N-(p-tolyl)benzamide(Compound 123);
4-((5-chloro-4-(1-isopropyl-1H-pyrazol-4-yl)pyrimidin-2-yl)amino)-3-methoxy-N-(2-methylbenzyl)benzamide (Compound 124);
4-((5-chloro-4-(1-isopropyl-1H-pyrazol-4-yl)pyrimidin-2-yl)amino)-3-methoxy-N-(3-methylbenzyl)benzamide (Compound 125);
4-((5-chloro-4-(1-isopropyl-1H-pyrazol-4-yl)pyrimidin-2-yl)amino)-3-methoxy-N-(4-methylbenzyl)benzamide (Compound 126);
4-((5-chloro-4-(1-isopropyl-1H-pyrazol-4-yl)pyrimidin-2-yl)amino)-N-(4-(dimethylamino)phenyl)-3-methoxybenzamide (Compound 127);
4-((5-chloro-4-(1-isopropyl-1H-pyrazol-4-yl)pyrimidin-2-yl)amino)-N-(4-cyanobenzyl)-3-methoxybenzamide (Compound 128);
4-((5-chloro-4-(1-isopropyl-1H-pyrazol-4-yl)pyrimidin-2-yl)amino)-N-(2,3-dihydro-1H-inden-2-yl)-3-methoxybenzamide (Compound 129);
4-((5-chloro-4-(1-isopropyl-1H-pyrazol-4-yl)pyrimidin-2-yl)amino)-3-methoxy-N-((tetrahydrofuran-3-yl)methyl)benzamide (Compound 130);
4-((5-chloro-4-(1-isopropyl-1H-pyrazol-4-yl)pyrimidin-2-yl)amino)-N-(3-cyano-4-fluorophenyl)-3-methoxybenzamide(Compound 131);
N-(5-chloro-2-fluorophenyl)-4-((5-chloro-4-(1-isopropyl-1H-pyrazol-4-yl)pyrimidin-2-yl)amino)-3-methoxybenzamide (Compound 132);
4-((5-chloro-4-(1-isopropyl-1H-pyrazol-4-yl)pyrimidin-2-yl)amino)-N-(1-(4-fluorophenyl)ethyl)-3-methoxybenzamide (Compound 133);
4-((5-chloro-4-(1-isopropyl-1H-pyrazol-4-yl)pyrimidin-2-yl)amino)-N-(2,4-difluorobenzyl)-3-methoxybenzamide (Compound 134);
4-((5-chloro-4-(1-isopropyl-1H-pyrazol-4-yl)pyrimidin-2-yl)amino)-N-(3,5-difluorobenzyl)-3-methoxybenzamide (Compound 135);
4-((5-chloro-4-(1-isopropyl-1H-pyrazol-4-yl)pyrimidin-2-yl)amino)-3-methoxy-N-(2-methylpyridin-4-yl)benzamide (Compound 136);
4-((5-chloro-4-(1-isopropyl-1H-pyrazol-4-yl)pyrimidin-2-yl)amino)-N-isopropyl-3-methoxybenzamide(Compound 137);
4-((5-chloro-4-(1-isopropyl-1H-pyrazol-4-yl)pyrimidin-2-yl)amino)-N-(2-hydroxyethyl)-3-methoxybenzamide (Compound 138);
4-((5-chloro-4-(1-isopropyl-1H-pyrazol-4-yl)pyrimidin-2-yl)amino)-N,N-diethyl-3-methoxybenzamide (Compound 139);
4-((5-chloro-4-(1-isopropyl-1H-pyrazol-4-yl)pyrimidin-2-yl)amino)-3-methoxy-N-(2-methoxyethyl)benzamide (Compound 140);
4-((5-chloro-4-(1-isopropyl-1H-pyrazol-4-yl)pyrimidin-2-yl)amino)-N-(2-hydroxyethyl)-3-methoxy-N-methylbenzamide (Compound 141);
4-((5-chloro-4-(1-isopropyl-1H-pyrazol-4-yl)pyrimidin-2-yl)amino)-N-isopropyl-3-methoxy-N-methylbenzamide (Compound 142);
4-((5-chloro-4-(1-isopropyl-1H-pyrazol-4-yl)pyrimidin-2-yl)amino)-3-methoxy-N-methyl-N-(1-methylpiperidin-4-yl)benzamide (Compound 143);
4-((5-chloro-4-(1-isopropyl-1H-pyrazol-4-yl)pyrimidin-2-yl)amino)-3-methoxy-N-(1-methyl-1H-pyrazol-4-yl)benzamide (Compound 144);
4-((5-chloro-4-(1-isopropyl-1H-pyrazol-4-yl)pyrimidin-2-yl)amino)-N-(1-isopropyl-1H-pyrazol-4-yl)-3-methoxybenzamide (Compound 145);
4-((5-chloro-4-(1-isopropyl-1H-pyrazol-4-yl)pyrimidin-2-yl)amino)-N-(4-chlorobenzyl)-3-methoxybenzamide (Compound 146);
4-((5-chloro-4-(1-isopropyl-1H-pyrazol-4-yl)pyrimidin-2-yl)amino)-N-(3-chlorobenzyl)-3-methoxybenzamide (Compound 147);
4-((5-chloro-4-(1-isopropyl-1H-pyrazol-4-yl)pyrimidin-2-yl)amino)-N-(2-chlorobenzyl)-3-methoxybenzamide (Compound 148);
4-((5-chloro-4-(1-isopropyl-1H-pyrazol-4-yl)pyrimidin-2-yl)amino)-N-(2-chlorophenyl)-3-methoxybenzamide (Compound 149);
4-((5-chloro-4-(1-isopropyl-1H-pyrazol-4-yl)pyrimidin-2-yl)amino)-N-(3-chlorophenyl)-3-methoxybenzamide (Compound 150);
4-((5-chloro-4-(1-isopropyl-1H-pyrazol-4-yl)pyrimidin-2-yl)amino)-N-(4-chlorophenyl)-3-methoxybenzamide (Compound 151);
4-((5-chloro-4-(1-isopropyl-1H-pyrazol-4-yl)pyrimidin-2-yl)amino)-N-(3-hydroxybenzyl)-3-methoxybenzamide (Compound 152);
4-((5-chloro-4-(1-isopropyl-1H-pyrazol-4-yl)pyrimidin-2-yl)amino)-N-(4-hydroxybenzyl)-3-methoxybenzamide (Compound 153);
4-((5-chloro-4-(1-isopropyl-1H-pyrazol-4-yl)pyrimidin-2-yl)amino)-3-methoxy-N-(2-methoxybenzyl)benzamide (Compound 154);
4-((5-chloro-4-(1-isopropyl-1H-pyrazol-4-yl)pyrimidin-2-yl)amino)-3-methoxy-N-(pyridin-2-ylmethyl)benzamide (Compound 155);
4-((5-chloro-4-(1-isopropyl-1H-pyrazol-4-yl)pyrimidin-2-yl)amino)-3-methoxy-N-(pyridin-3-ylmethyl)benzamide (Compound 156);
4-((5-chloro-4-(1-isopropyl-1H-pyrazol-4-yl)pyrimidin-2-yl)amino)-3-methoxy-N-(pyridin-4-ylmethyl)benzamide (Compound 157);
4-((5-chloro-4-(1-isopropyl-1H-pyrazol-4-yl)pyrimidin-2-yl)amino)-N-(2-hydroxyphenyl)-3-methoxybenzamide (Compound 158);
4-((5-chloro-4-(1-isopropyl-1H-pyrazol-4-yl)pyrimidin-2-yl)amino)-N-(3-hydroxyphenyl)-3-methoxybenzamide (Compound 159);
4-((5-chloro-4-(1-isopropyl-1H-pyrazol-4-yl)pyrimidin-2-yl)amino)-N-(4-hydroxyphenyl)-3-methoxybenzamide (Compound 160);
4-((5-chloro-4-(1-isopropyl-1H-pyrazol-4-yl)pyrimidin-2-yl)amino)-3-methoxy-N-(2-methoxyphenyl)benzamide (Compound 161);
4-((5-chloro-4-(1-isopropyl-1H-pyrazol-4-yl)pyrimidin-2-yl)amino)-3-methoxy-N-(3-methoxyphenyl)benzamide (Compound 162);
4-((5-chloro-4-(1-isopropyl-1H-pyrazol-4-yl)pyrimidin-2-yl)amino)-3-methoxy-N-(4-methoxyphenyl)benzamide (Compound 163);
4-((5-chloro-4-(1-isopropyl-1H-pyrazol-4-yl)pyrimidin-2-yl)amino)-N-(2,3-dihydro-1H-inden-1-yl)-3-methoxybenzamide (Compound 164);
4-((5-chloro-4-(1-isopropyl-1H-pyrazol-4-yl)pyrimidin-2-yl)amino)-3-methoxy-N-(2-methoxybenzyl)-N-methylbenzamide (Compound 165);
4-((5-chloro-4-(1-isopropyl-1H-pyrazol-4-yl)pyrimidin-2-yl)amino)-3-methoxy-N-(2,2,6,6-tetramethylpiperidin-4-yl)benzamide (Compound 166);
4-((5-chloro-4-(1-isopropyl-1H-pyrazol-4-yl)pyrimidin-2-yl)amino)-N-(2,6-dimethylpiperidin-1-yl)-3-methoxybenzamide (Compound 167);
4-((5-chloro-4-(1-isopropyl-1H-pyrazol-4-yl)pyrimidin-2-yl)amino)-N-((6-chloropyridin-3-yl)methyl)-3-methoxybenzamide (Compound 168);
4-((5-chloro-4-(1-isopropyl-1H-pyrazol-4-yl)pyrimidin-2-yl)amino)-3-methoxy-N-methyl-N-(2-(pyridin-2-yl)ethyl)benzamide (Compound 169);
4-((5-chloro-4-(1-isopropyl-1H-pyrazol-4-yl)pyrimidin-2-yl)amino)-N-(3-(difluoromethoxy)phenyl)-3-methoxybenzamide (Compound 170);
4-((5-chloro-4-(1-isopropyl-1H-pyrazol-4-yl)pyrimidin-2-yl)amino)-N-(3-ethylphenyl)-3-methoxybenzamide (Compound 171);
4-((5-chloro-4-(1-isopropyl-1H-pyrazol-4-yl)pyrimidin-2-yl)amino)-N-(2-ethylphenyl)-3-methoxybenzamide (Compound 172);
4-((5-chloro-4-(1-isopropyl-1H-pyrazol-4-yl)pyrimidin-2-yl)amino)-N-(4-ethylphenyl)-3-methoxybenzamide (Compound 173);
N-benzyl-4-((5-chloro-4-(1-isopropyl-1H-pyrazol-4-yl)pyrimidin-2-yl)amino)-3-methoxy-N-methylbenzamide (Compound 174);
4-((5-chloro-4-(1-isopropyl-1H-pyrazol-4-yl)pyrimidin-2-yl)amino)-3-methoxy-N-methyl-N-(3-methylbenzyl)benzamide (Compound 175);
ethyl 3-(4-((5-chloro-4-(1-isopropyl-1H-pyrazol-4-yl)pyrimidin-2-yl)amino)-3-methoxybenzamido)propanoate (Compound 176);
N-benzyl-4-((5-chloro-4-(1-isopropyl-1H-pyrazol-4-yl)pyrimidin-2-yl)amino)-N-isopropyl-3-methoxybenzamide (Compound 177);
4-((5-chloro-4-(1-isopropyl-1H-pyrazol-4-yl)pyrimidin-2-yl)amino)-N-(3-isopropoxyphenyl)-3-methoxybenzamide(Compound 178);
4-((5-chloro-4-(1-isopropyl-1H-pyrazol-4-yl)pyrimidin-2-yl)amino)-3-methoxy-N-(1-phenylethyl)benzamide(Compound 179);
4-((5-chloro-4-(1-isopropyl-1H-pyrazol-4-yl)pyrimidin-2-yl)amino)-3-methoxy-N-(3-(trifluoromethyl)phenyl)benzamide (Compound 180);
4-((5-chloro-4-(1-isopropyl-1H-pyrazol-4-yl)pyrimidin-2-yl)amino)-3-methoxy-N-(4-(trifluoromethyl)phenyl)benzamide (Compound 181);
4-((5-chloro-4-(1-isopropyl-1H-pyrazol-4-yl)pyrimidin-2-yl)amino)-3-methoxy-N-(1-phenylpropyl)benzamide (Compound 182);
N-(4-aminobenzyl)-4-((5-chloro-4-(1-isopropyl-1H-pyrazol-4-yl)pyrimidin-2-yl)amino)-3-methoxybenzamide(Compound 183);
4-((5-chloro-4-(1-isopropyl-1H-pyrazol-4-yl)pyrimidin-2-yl)amino)-N-(2-fluoro-5-methylphenyl)-3-methoxybenzamide (Compound 184);
4-((5-chloro-4-(1-isopropyl-1H-pyrazol-4-yl)pyrimidin-2-yl)amino)-N-(2-fluoro-4-methylphenyl)-3-methoxybenzamide (Compound 185);
4-((5-chloro-4-(1-isopropyl-1H-pyrazol-4-yl)pyrimidin-2-yl)amino)-N-(4-fluoro-2-methylphenyl)-3-methoxybenzamide (Compound 186);
4-((5-chloro-4-(1-isopropyl-1H-pyrazol-4-yl)pyrimidin-2-yl)amino)-N-(4-fluoro-3-methylphenyl)-3-methoxybenzamide (Compound 187);
4-((5-chloro-4-(1-isopropyl-1H-pyrazol-4-yl)pyrimidin-2-yl)amino)-N-(3-fluoro-2-methylphenyl)-3-methoxybenzamide (Compound 188);
4-((5-chloro-4-(1-isopropyl-1H-pyrazol-4-yl)pyrimidin-2-yl)amino)-3-methoxy-N-(4-sulfamoylbenzyl)benzamide (Compound 189);
4-((5-chloro-4-(1-isopropyl-1H-pyrazol-4-yl)pyrimidin-2-yl)amino)-3-methoxy-N-(3-(methylsulfonamido)phenyl)benzamide (Compound 190);
4-((5-chloro-4-(1-isopropyl-1H-pyrazol-4-yl)pyrimidin-2-yl)amino)-3-methoxy-N-(3-sulfamoylphenyl)benzamide (Compound 191);
4-((5-chloro-4-(1-isopropyl-1H-pyrazol-4-yl)pyrimidin-2-yl)amino)-3-methoxy-N-(2-(methylsulfonyl)ethyl)benzamide (Compound 192);
methyl (4-((5-chloro-4-(1-isopropyl-1H-pyrazol-4-yl)pyrimidin-2-yl)amino)-3-methoxybenzoyl)-L-valinate (Compound 193);
methyl (4-((5-chloro-4-(1-isopropyl-1H-pyrazol-4-yl)pyrimidin-2-yl)amino)-3-methoxybenzoyl)-L-isoleucinate (Compound 194);
ethyl (4-((5-chloro-4-(1-isopropyl-1H-pyrazol-4-yl)pyrimidin-2-yl)amino)-3-methoxybenzoyl)glycinate (Compound 195);
N-(4-aminophenethyl)-4-((5-chloro-4-(1-isopropyl-1H-pyrazol-4-yl)pyrimidin-2-yl)amino)-3-methoxybenzamide(Compound 196);
N-(3-(1H-imidazol-1-yl)propyl)-4-((5-chloro-4-(1-isopropyl-1H-pyrazol-4-yl)pyrimidin-2-yl)amino)-3-methoxybenzamide (Compound 197);
4-((5-chloro-4-(1-isopropyl-1H-pyrazol-4-yl)pyrimidin-2-yl)amino)-N-(2,2-dimethoxyethyl)-3-methoxybenzamide (Compound 198);
methyl (4-((5-chloro-4-(1-isopropyl-1H-pyrazol-4-yl)pyrimidin-2-yl)amino)-3-methoxybenzoyl)-L-leucinate(Compound 199);
methyl (4-((5-chloro-4-(1-isopropyl-1H-pyrazol-4-yl)pyrimidin-2-yl)amino)-3-methoxybenzoyl)-L-prolinate (Compound 200);
4-((5-chloro-4-(1-isopropyl-1H-pyrazol-4-yl)pyrimidin-2-yl)amino)-3-methoxy-N-(4-methoxybenzyl)-N-methylbenzamide (Compound 201);
4-((5-chloro-4-(1-isopropyl-1H-pyrazol-4-yl)pyrimidin-2-yl)amino)-N-ethyl-3-methoxy-N-methylbenzamide (Compound 202);
4-((5-chloro-4-(1-isopropyl-1H-pyrazol-4-yl)pyrimidin-2-yl)amino)-3-methoxy-N-(4-methoxybenzyl)benzamide (Compound 203);
4-((5-chloro-4-(1-isopropyl-1H-pyrazol-4-yl)pyrimidin-2-yl)amino)-3-methoxy-N-(4-methoxy-3,5-dimethylphenyl)benzamide (Compound 204);
N-(3-aminobenzyl)-4-((5-chloro-4-(1-isopropyl-1H-pyrazol-4-yl)pyrimidin-2-yl)amino)-3-methoxybenzamide (Compound 205);
4-((5-chloro-4-(1-isopropyl-1H-pyrazol-4-yl)pyrimidin-2-yl)amino)-N-(2,3-dihydro-1H-inden-5-yl)-3-methoxybenzamide (Compound 206);
4-((5-chloro-4-(1-isopropyl-1H-pyrazol-4-yl)pyrimidin-2-yl)amino)-N-(2,3-dihydro-1H-inden-4-yl)-3-methoxybenzamide (Compound 207);
4-((5-chloro-4-(1-isopropyl-1H-pyrazol-4-yl)pyrimidin-2-yl)amino)-N-(2,3-dihydro-1H-inden-2-yl)-3-methoxy-N-methylbenzamide (Compound 208);
4-((5-chloro-4-(1-isopropyl-1H-pyrazol-4-yl)pyrimidin-2-yl)amino)-N-(2,3-dihydro-1H-inden-1-yl)-3-methoxy-N-methylbenzamide (Compound 209);
(4-((5-chloro-4-(1-isopropyl-1H-pyrazol-4-yl)pyrimidin-2-yl)amino)-3-methoxyphenyl)(4-(ethyl(methyl)amino)piperidin-1-yl)methanone (Compound 210) ;
N-(1-acetylindolin-5-yl)-4-((5-chloro-4-(1-isopropyl-1H-pyrazol-4-yl)pyrimidin-2-yl)amino)-3-methoxy-N-methylbenzamide (Compound 211);
N-(1-acetylindolin-5-yl)-4-((5-chloro-4-(1-isopropyl-1H-pyrazol-4-yl)pyrimidin-2-yl)amino)-3-methoxybenzamide (Compound 212);
ethyl 3-(4-((5-chloro-4-(1-isopropyl-1H-pyrazol-4-yl)pyrimidin-2-yl)amino)-3-methoxybenzamido)-4,4,4-trifluorobutanoate (Compound 213);
4-((5-chloro-4-(1-isopropyl-1H-pyrazol-4-yl)pyrimidin-2-yl)amino)-N-(2,3-dihydro-1H-inden-5-yl)-3-methoxy-N-methylbenzamide (Compound 214);
4-((5-chloro-4-(1-isopropyl-1H-pyrazol-4-yl)pyrimidin-2-yl)amino)-N-(2,3-dihydro-1H-inden-4-yl)-3-methoxy-N-methylbenzamide (Compound 215);
(4-((5-chloro-4-(1-isopropyl-1H-pyrazol-4-yl)pyrimidin-2-yl)amino)-3-methoxyphenyl)(4-(pyrrolidin-1-yl)piperidin-1-yl)methanone (Compound 216); and
[1,4'-bipiperidin]-1'-yl(4-((5-chloro-4-(1-isopropyl-1H-pyrazol-4-yl)pyrimidin-2-yl)amino)-3-methoxyphenyl)methanone (Compound 217).

**TABLE 1**

| Cpd. # | Chemical Structure | NMR Spectrum Data |
|---|---|---|
| 2 | 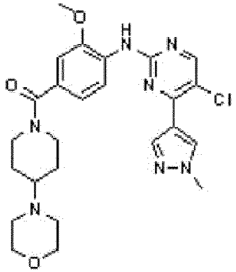 | ¹H NMR (300 MHz, DMSO-d6) δ 8.62 (s, 1H), 8.52 (s,1H), 8.28 (s, 1H), 8.21 - 8.24 (m, 2H), 7.07 (s, 1H), 7.06 (m, 1H), 4.51 - 4.55 (m, 2H), 3.96 (s, 3H), 3.89 (s, 3H), 3.53 - 3.60 (m, 4H), 2.89 - 2.93 (m, 2H), 2.45 - 2.53 (m, 5H), 1.78 - 1.82 (m, 2H), 1.35 - 1.38 (m, 2H). |
| 3 | 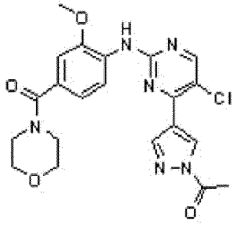 | ¹H NMR (300 MHz, Chloroform-*d*) δ 9.14 (d, J = 0.7 Hz, 1H), 8.49 - 8.55 (m, 2H), 8.45 (s, 1H), 7.94 (s, 1H), 7.02 - 7.10 (m, 2H), 3.97 (s, 3H), 3.50 - 3.85 (s, 8H), 2.79 (s, 3H). |
| 4 | 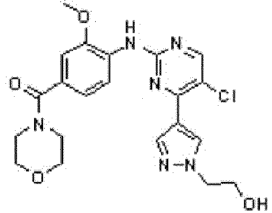 | ¹H NMR (400 MHz, CDCl3) δ 8.54 (d, J = 6.0 Hz, 1H),8.40 (s, 1H), 8.39 (s, 1H), 8.37 (s, 1H), 7.86 (s,1H), 7.06 (s, 1H), 7.04 (d, J = 6.0 Hz, 1H), 4.33 - 4.37 (m, 2H), 4.07 - 4.11 (m, 2H), 3.96 (s, 3H), 3.60 - 3.81 (m, 8H). |
| 5 | 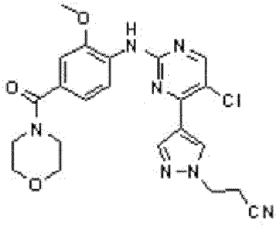 | ¹H NMR (300 MHz, Methanol-d⁴) d 8.73 (s, 1H), 8.55 (d, J = 8.7 Hz, 1H), 8.48 (s, 1H), 8.42 (s, 1H), 7.13 (dd, J = 4.5, 2.7 Hz, 2H), 4.57 (t, J = 6.4Hz, 2H), 4.00 (s, 3H), 3.71 (s, 7H), 3.12 (t, J = 6.4 Hz, 2H), 2.63 (p, J = 1.9 Hz, 4H), 2.05 (s, 1H). |
| 6 | 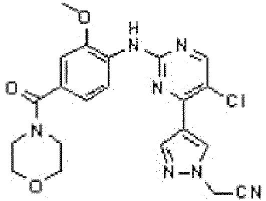 | ¹H NMR (300 MHz, Chloroform-*d*) δ 8.51 (d, J = 9.2 Hz, 2H), 8.45 (s, 1H), 8.41 (s, 1H), 7.89 (s, 1H), 6.98 - 7.13 (m, 2H), 5.18 (s, 2H), 3.97 (s, 3H), 3.50 - 3.85 (m, 8H). |
| 7 | 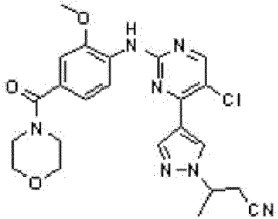 | ¹H NMR (400 MHz, DMSO-d6) δ 8.75 (s, 1H), 8.55 (s, 1H), 8.32 (d, J = 6.3 Hz, 2H), 8.26 (d, J = 8.1 Hz, 1H), 7.09 (d, J = 8.9 Hz, 2H), 3.90 (s, 3H), 3.57 (d, J = 31.2 Hz, 9H), 3.17 (d, J = 6.6 Hz, 2H), 1.55 (d, J = 6.7 Hz, 3H). |
| 8 | 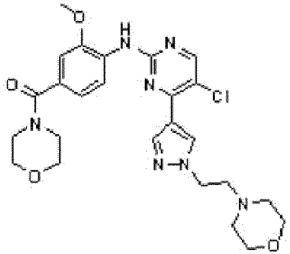 | ¹H NMR (400 MHz, Chloroform-*d*) δ 8.54 (d, J = 8.1 Hz, 1H), 8.43 (d, J = 2.0 Hz, 1H), 8.35 (d, J = 3.0 Hz, 2H), 7.85 (s, 1H), 7.03 (d, J = 9.8 Hz, 2H), 4.30 (t, J = 6.4 Hz, 2H), 3.94 (s, 3H), 3.79 - 3.59 (m, 12H), 2.85 (t, J = 6.4 Hz, 2H), 2.49 (t, J = 4.6 Hz, 4H). |
| 9 | 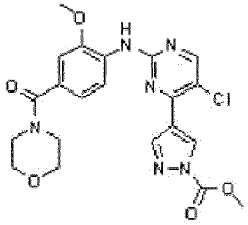 | ¹H NMR (400 MHz, CDCl3) δ 8.53 (d, J = 8.0 Hz, 1H),8.46 (s, 1H), 8.43 (s, 1H), 8.39 (s, 1H), 7.97 (s, NH), 7.06 (s, 1H), 7.04 (d, J = 8.0 Hz, 1H), 5.02 (s, 2H), 3.96 (s, 3H), 3.82 (s, 3H), 3.60 - 3.81 (m, 8H). |
| 10 | 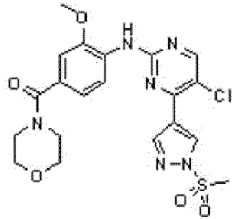 | ¹H NMR (300 MHz, Chloroform-*d*) δ 8.92 (d, J = 0.7 Hz, 1H), 8.61 (d, J = 0.7 Hz, 1H), 8.48 (d, J = 8.2 Hz, 1H), 8.44 (s, 1H), 7.92 (s, 1H), 7.00 - 7.13 (m, 2H), 3.96 (s, 3H), 3.55 - 3.83 (m, 8H), 3.44 (s, 3H). |
| 11 | 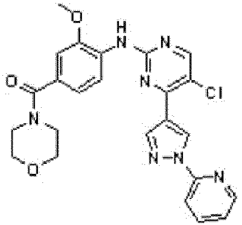 | ¹H NMR (300 MHz, Chloroform-*d*) δ 9.47 (d, J = 0.8 Hz, 1H), 8.55 - 8.63 (m, 2H), 8.45 - 8.50 (m, 1H), 8.42 (s, 1H), 8.05 (dt, J = 8.3, 1.0 Hz, 1H), 7.83 - 7.94 (m, 2H), 7.23 - 7.30 (m, 1H), 7.04 - 7.10 (m, 2H), 3.97 (s, 3H), 3.61 - 3.79 (m, 8H). |
| 12 | 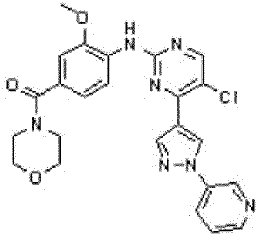 | ¹H NMR (400 MHz, Chloroform-*d*) δ 9.10 (s, 1H), 8.88 (s, 1H), 8.63 (s, 2H), 8.56 (d, J = 8.1 Hz, 1H), 8.45 (s, 1H), 8.14 (dt, J = 8.6, 1.7 Hz, 1H), 7.92 (s, 1H), 7.49 (t, J = 6.4 Hz, 1H), 7.04 - 7.10 (m, 2H), 3.98 (s, 3H), 3.56 - 3.82 (m, 8H). |
| 13 | 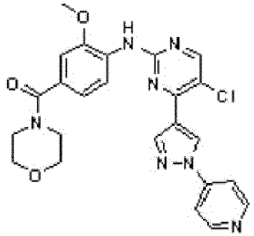 | ¹H NMR (400 MHz, Chloroform-*d*) δ 8.93 (s, 1H), 8.75 (s, 2H), 8.59 (s, 1H), 8.53 (d, J = 8.3 Hz, 1H), 8.42 (s, 1H), 7.90 (s, 1H), 7.68 - 7.80 (m, 2H), 7.02 - 7.10 (m, 2H), 3.96 (s, 3H), 3.54 - 3.82 (m, 8H). |
| 14 | 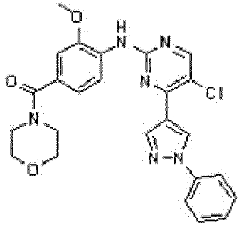 | ¹H NMR (300 MHz, Chloroform-*d*) δ 8.84 (d, J = 0.7 Hz, 1H), 8.55 - 8.60 (m, 2H), 8.42 (s, 1H), 7.90 (s, 1H), 7.74 - 7.81 (m, 2H), 7.48 - 7.56 (m, 2H), 7.32 - 7.41 (m, 1H), 7.03 - 7.10 (m, 2H), 3.97 (s, 3H), 3.61 - 3.79 (m, 8H). |
| 15 | 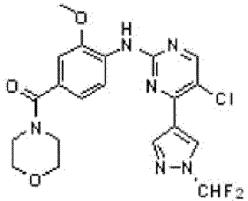 | ¹H NMR (300 MHz, Chloroform-*d*) δ 8.74 (s, 1H), 8.49 - 8.55 (m, 2H), 8.45 (s, 1H), 7.91 (s, 1H), 7.07 - 7.49 (t, 1H), 7.02 - 7.10 (m, 2H), 3.97 (s, 3H), 3.59 - 3.80 (m, 8H). |
| 16 | 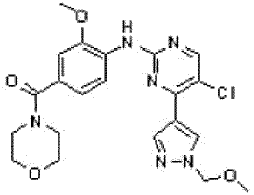 | ¹H NMR (400 MHz, Chloroform-*d*) δ 8.55 (d, J = 8.1 Hz, 1H), 8.52 (d, J = 3.6 Hz, 1H), 8.36 - 8.48 (m, 2H), 7.88 (s, 1H), 7.02 - 7.10 (m, 2H), 5.48 (s, 2H), 3.96 (s, 3H), 3.54 - 3.83 (m, 8H), 3.40 (s, 3H). |
| 17 | 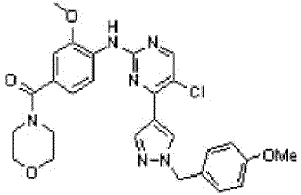 | ¹H NMR (300 MHz, Chloroform-*d*) δ 8.51 (d, J = 8.3 Hz, 1H), 8.41 (d, J = 0.7 Hz, 1H), 8.35 (s, 1H), 8.28 (d, J = 0.7 Hz, 1H), 7.88 (s, 1H), 7.22 - 7.30 (m, 2H), 7.05 (d, J = 1.8 Hz, 1H), 7.00 (dd, J = 8.3, 1.8 Hz, 1H), 6.87 - 6.95 (m, 2H), 5.32 (s, 2H), 3.95 (s, 3H), 3.81 (s, 3H), 3.59 - 3.78 (m, 8H). |
| 18 | 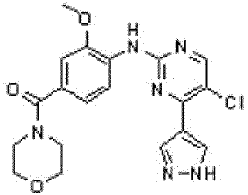 | ¹H NMR (300 MHz, Chloroform-*d*) δ 8.57 (d, J = 8.8 Hz, 1H), 8.49 (s, 2H), 8.39 (s, 1H), 7.88 (s, 1H), 7.03 - 7.09 (m, 2H), 3.96 (s, 3H), 3.54 - 3.85 (m, 8H). |
| 19 | 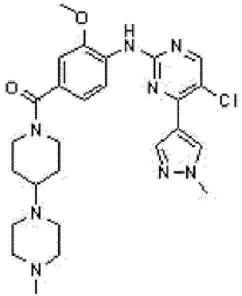 | ¹H NMR (300 MHz, Methanol-d⁴) δ 8.58 - 8.48 (m, 2H), 8.42 (d, J = 4.3Hz, 1H), 8.31 (d, J = 0.7 Hz, 1H), 7.10 (tt, J = 4.0, 1.9 Hz, 2H), 3.99 (d, J = 3.6 Hz, 6H), 3.92 (s, 1H), 3.42 (s, 4H), 3.19 (d, J = 32.8 Hz, 9H), 2.91 (s, 3H), 2.05 (s, 2H), 1.78 - 1.54 (m, 2H). |
| 20 | 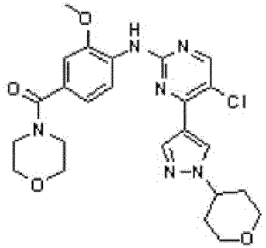 | ¹H NMR (400 MHz, Chloroform-*d*) δ 8.56 (d, J = 8.2 Hz, 1H), 8.44 - 8.31 (m, 3H), 7.88 (s, 1H), 7.11 - 7.01 (m, 2H), 4.59 (p, J = 6.7 Hz, 1H), 3.96 (s, 3H), 3.72 (s, 9H), 1.68 (s, 9H), 1.59 (d, J = 6.7 Hz, 6H). |
| 21 | 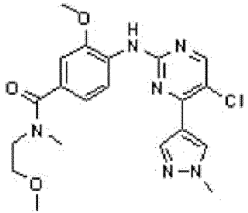 | ¹H NMR (400 MHz, Chloroform-*d*) δ 8.43 (s, 1H), 8.09 (d, *J* = 7.7 Hz, 2H), 7.11 (d, *J* = 21.2 Hz, 2H), 3.91 (s, 3H), 3.67 (s, 2H), 3.37 (d, *J* = 19.5 Hz, 2H), 3.15 (s, 2H). |
| 22 | 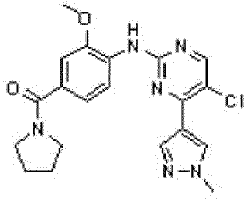 | ¹H NMR (400 MHz, CDCl3) δ 8.53 (d, J = 8.0 Hz, 1H), 8.37 (s, 1H), 8.36 (s, 1H), 8.30 (s, 1H), 7.86 (s, NH), 7.20 (d, J = 8.0 Hz, 1H), 7.19 (s, 1H), 4.00 (s, 3H), 3.96 (s, 3H), 3.66 (t, J = 6.0 Hz, 2H), 3.55 (t, J = 6.0 Hz, 2H), 1.95 - 1.99 (m, 2H), 1.86 - 1.90 (m, 2H). |
| 23 | 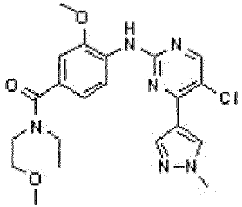 | ¹H NMR (300 MHz, CDCl3) δ 8.50 (d, J = 9.0 Hz, 1H), 8.36 (s, 2H), 8.31 (s, 1H), 7.90 (s, NH), 7.08 (s, 1H), 7.04 (d, J = 9.0 Hz, 1H), 4.00 (s, 3H), 3.94 (s, 3H), 3.59 - 3.63 (m, 6H), 3.37 (s, 3H), 1.25 (s, 3H). |
| 24 | 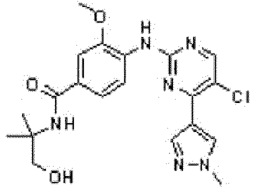 | ¹H NMR (300 MHz, Chloroform-*d*) δ 8.51 (d, J = 8.4 Hz, 1H), 8.35 - 8.28 (m, 2H), 8.26 (s, 1H), 7.86 (s, 1H), 7.40 (d, J = 1.9 Hz, 1H), 6.30 (s, 1H), 5.02 (t, J = 6.0 Hz, 1H), 3.98 (s, 3H), 3.94 (s, 3H), 3.70 (d, J = 5.2 Hz, 2H), 1.43 (s, 6H). |
| 25 | 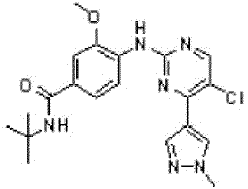 | ¹H NMR (300 MHz, Chloroform-*d*) δ 8.51 (d, J = 8.4 Hz, 1H), 8.35 - 8.28 (m, 2H), 8.26 (s, 1H), 7.86 (s, 1H), 7.40 (d, J = 1.9 Hz, 1H), 6.30 (s, 1H), 5.02 (t, J = 6.0 Hz, 1H), 3.98 (s, 3H), 3.94 (s, 3H), 3.70 (d, J = 5.2 Hz, 2H), 1.43 (s, 6H). |
| 26 | 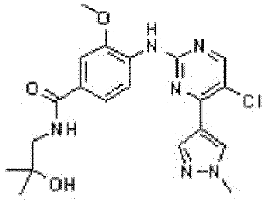 | ¹H NMR (400 MHz, Chloroform-*d*) δ 8.54 (d, J = 8.4 Hz, 1H), 8.37 (d, J = 1.9 Hz, 2H), 8.32 (s, 1H), 8.03 (s, 1H), 7.49 (d, J = 1.9 Hz, 1H), 7.40 - 7.34 (m, 1H), 6.68 (s, 1H), 4.02 (s, 3H), 3.99 (s, 3H), 3.50 (d, J = 5.9 Hz, 2H), 1.32 (s, 6H) |
| 27 | 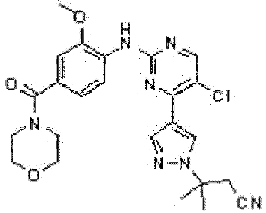 | ¹H NMR (300 MHz, Chloroform-*d*) δ 8.55 (d, J = 8.1 Hz, 1H), 8.50 - 8.42 (m, 2H), 8.39 (s, 1H), 7.87 (s, 1H),7.09 - 7.01 (m, 2H), 3.96 (s, 3H), 3.70 (s, 8H), 3.07 (s, 2H), 1.84 (s, 6H). |
| 28 | 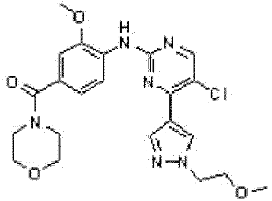 | ¹H NMR (300 MHz, Chloroform-*d*) δ 8.51 8.59 (m, 1H), 8.40 (d, J = 0.7 Hz, 1H), 8.37 (d, J = 0.7 Hz, 1H), 8.35 (s, 1H), 7.85 (s, 1H), 7.00 7.07 (m, 2H), 4.36 (t, J = 5.1 Hz, 2H), 3.94 (s, 3H), 3.79 (t, J = 5.1 Hz, 2H), 3.60 3.75 (m, 8H), 3.35 (s, 3H). |
| 29 | 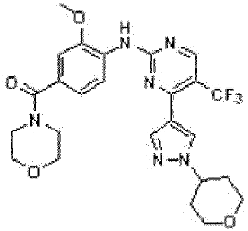 | ¹H NMR (300 MHz, Chloroform-d) δ 8.67 (s, 1H), 8.58 (d, J = 8.2 Hz, 1H), 8.21 (s, 1H), 8.11 (s, 1H), 8.05 (s, 1H), 7.01 - 7.11 (m, 2H), 4.35 - 4.50 (m, 1H), 4.10 - 4.20 (m, 2H), 3.97 (s, 3H), 3.50 - 3.80 (m, 10H), 2.09 - 2.21 (m, 4H). |
| 30 | 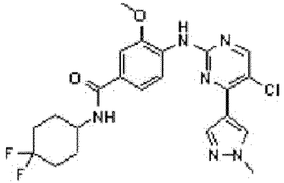 | ¹H NMR (300 MHz, DMSO-d6) δ 8.64 (s, 1H), 8.55 (s, 1H), 8.31 (d, J = 9.1 Hz, 2H), 8.23 (s, 1H), 8.19 (d, J = 7.8 Hz, 1H), 7.58 (dd, J = 8.4, 1.9 Hz, 1H), 7.51 (d, J = 1.8 Hz, 1H), 3.98 - 4.06 (m, 1H), 3.96 (s, 3H), 3.94 (s, 3H), 1.58 - 2.13 (m, 8H). |
| 31 | 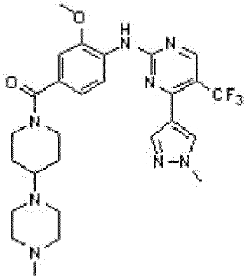 | ¹H NMR (300 MHz, Chloroform-d) δ 8.64 (s, 1H), 8.53 (d, J = 8.7 Hz, 1H), 8.16 (s, 1H), 8.03 (s, 1H), 8.01 (s, 1H), 7.04 (dd, J = 8.7, 1.8 Hz, 1H), 7.02 (d, J = 1.8 Hz, 1H), 3.98 (s, 3H), 3.94 (s, 3H), 2.35 - 3.05 (m, 13H), 2.29 (s, 3H), 1.82 - 1.97 (m, 2H), 1.40 - 1.61 (m, 2H). |
| 32 | 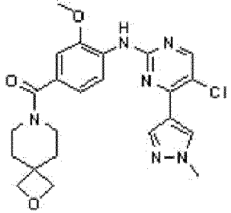 | ¹H NMR (300 MHz, Chloroform-d) δ 8.53 (d, J = 8.8 Hz, 1H), 8.36 (s, 1H), 8.36 (s, 1H), 8.30 (s, 1H), 7.84 (s, 1H), 7.03 (dd, J = 8.8, 1.8 Hz, 1H), 7.01 (s, J = 1.8 Hz, 1H), 4.45 - 4.52 (m, 4H), 4.00 (s, 3H), 3.94 (s, 3H), 3.44 - 3.62 (m, 4H), 1.84 - 1.96 (m, 4H). |
| 33 | 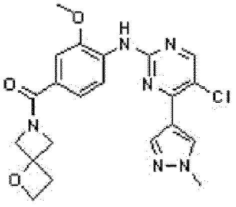 | ¹H NMR (400 MHz, 3hloroform-d) δ 8.54 (d, J = 8.4 Hz, 1H), 8.37 (s, 1H), 8.36 (s, 1H), 8.30 (s, 1H), 7.90 (s, 1H), 7.31 (d, J = 1.8 Hz, 1H), 7.22 (dd, J = 8.4, 1.8 Hz, 1H), 4.34 - 4.62 (m, 6H), 4.01 (s, 3H), 3.96 (s, 3H), 2.86 (t, J = 7.9 Hz, 2H). |
| 34 | 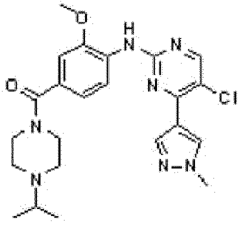 | ¹H NMR (300 MHz, 3hloroform-d) δ 8.54 (d, J = 8.7 Hz, 1H), 8.37 (d, J = 1.1 Hz, 2H), 8.31 (s, 1H), 7.85 (s, 1H), 7.02 - 7.10 (m, 2H), 4.01 (s, 3H), 3.95 (s, 3H), 3.54 - 3.85 (m, 4H), 2.75 (p, J = 6.5 Hz, 1H), 2.45 - 2.68 (m, 4H), 1.07 (d, J = 6.5 Hz, 6H). |
| 35 | 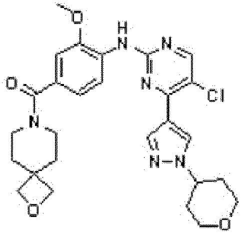 | ¹H NMR (300 MHz, 3hloroform-d) δ 8.54 (d, J = 8.7 Hz, 1H), 8.42 - 8.35 (m, 3H), 7.85 (s, 1H), 7.02 (dq, J = 3.8, 1.8 Hz, 2H), 4.49 (s, 4H), 4.45 - 4.37 (m, 1H), 4.20 - 4.11 (m, 2H), 3.95 (s, 3H), 3.63 - 3.48 (m, 6H), 2.23 - 2.11 (m, 4H), 1.91 (s, 4H). |
| 36 | 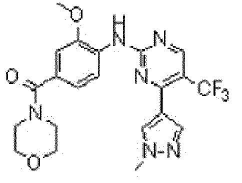 | ¹H NMR (300 MHz, Chloroform-d) δ 8.66 (s, 1H), 8.57 (d, J = 8.1 Hz, 1H), 8.18 (s, 1H), 8.05 (s, 1H), 8.03 (s, 1H), 7.07 (d, J = 1.8 Hz, 1H), 7.06 (dd, J = 8.1, 1.8 Hz, 1H), 4.00 (s, 3H), 3.97 (s, 3H), 3.60 - 3.79 (m, 8H). |
| 37 | 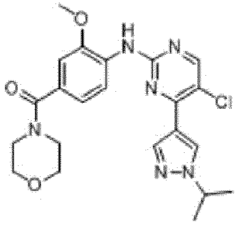 | ¹H NMR (300 MHz, Chloroform-*d*) δ 8.57 (d, *J*= 8.1 Hz, 1H), 8.33 - 8.41 (m, 3H), 7.85 (s, 1H), 7.02 - 7.09 (m, 2H), 4.59 (m, *J*= 6.7 Hz, 1H), 3.96 (s, 3H), 3.62- 3.79 (m, 8H), 1.59 (d, *J*= 6.7 Hz, 6H). |
| 38 | 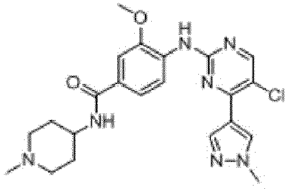 | ¹H NMR (300 MHz, Chloroform-d) δ 8.55 (dd, J = 8.5, 1.2 Hz, 1H), 8.36 (s, 1H), 8.35 (s, 1H), 3.29 (s, 1H), 7.89 (s, 1H), 7.46 (d, J = 1.9 Hz, 1H), 7.30 (dd, J = 8.4, 1.9 Hz, 1H), 6.04 (s, 1H), 4.00 (s, 3H), 3.97 (s, 1H), 2.77 - 2.90 (m, 2H), 2.31 (s, 3H), 1.98 - 2.25 (m, 5H), 1.53 - 1.69 (m, 2H). |
| 39 | 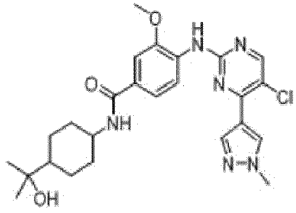 | ¹H NMR (300 MHz, Chloroform-d) δ 8.50 (d, J = 8.7 Hz, 1H), 8.34 (s, 1H), 8.33 (s, 1H), 8.28 (s, 1H), 7.83 (s, 1H), 7.04 (dd, J = 8.7, 1.8 Hz, 1H), 7.02 (d, J = 1.8 Hz, 1H), 3.98 (s, 3H), 3.93 (s, 3H), 2.58 - 3.06 (m, 2H), 1.74 - 2.06 (m, 3H), 1.22 - 1.64 (m, 4H), 1.19 (s, 6H). |
| 40 | 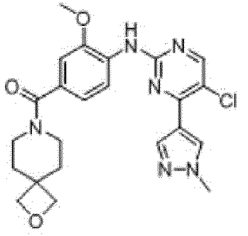 | ¹H NMR (400 MHz, Chloroform-d) δ 8.56 (d, J = 8.4 Hz, 1H), 8.36 (d, J = 4.5 Hz, 2H), 8.31 (s, 1H), 7.91 (s, 1H), 7.32 (d, J = 1.9 Hz, 1H), 7.21 (dd, J = 8.4, 1.8 Hz, 1H), 4.82 (s, 4H), 4.28 - 4.61 (m, 4H), 4.01 (s, 3H), 3.96 (s, 3H). |
| 41 | 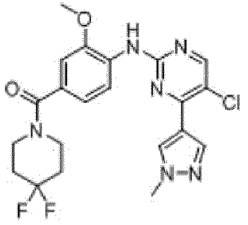 | ¹H NMR (400 MHz, Chloroform-d) δ 8.54 (d, J = 8.4 Hz, 1H), 8.34 (s, 1H), 8.33 (s, 1H), 8.28 (s, 1H), 7.84 (s, 1H), 7.05 (d, J = 8.4 Hz, 2H), 3.98 (s, 3H), 3.94 (s, 3H), 3.63 - 3.86 (m, 4H), 1.87 - 2.22 (m, 4H). |
| 42 | 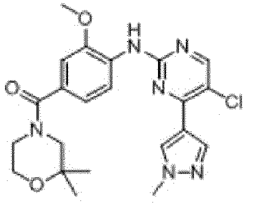 | ¹H NMR (300 MHz, Chloroform-d) δ 8.55 (d, J = 8.7 Hz, 1H), 8.38 - 8.34 (m, 2H), 8.30 (s, 1H), 7.85 (s, 1H), 7.04 (dd, J = 1.8 Hz, 1H), 7.03 (d, J = 1.8 Hz, 1H), 4.00 (s, 3H), 3.95 (s, 3H), 3.35 - 3.82 (m, 6H), 1.24 (s, 6H). |
| 43 | 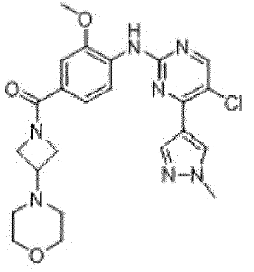 | ¹H NMR (300 MHz, Chloroform-d) δ 8.56 (d, J = 8.4 Hz, 1H), 8.36 - 8.39 (m, 2H), 8.32 (s, 1H), 7.91 (s, 1H), 7.35 (d, J = 1.8 Hz, 1H), 7.24 (dd, J = 8.4, 1.8 Hz, 1H), 4.18 - 4.41 (m, 3H), 4.04 - 4.13 (m, 1H), 4.01 (s, 3H), 3.97 (s, 3H), 3.72 - 3.79 (m, 4H), 3.16 - 3.26 (m, 1H), 2.35 - 2.45 (m, 4H). |
| 44 | 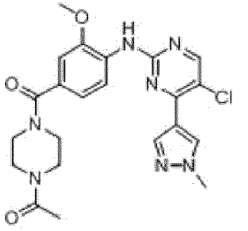 | ¹H NMR (400 MHz, Chloroform-d) δ 8.54 (d, J = 8.6 Hz, 1H), 8.34 (s, 1H), 8.33 (s, 1H), 8.28 (s, 1H), 7.85 (s, 1H), 7.04 (t, J = 3.2 Hz, 2H), 3.98 (s, 3H), 3.94 (s, 3H), 3.41 - 3.82 (m, 8H), 2.12 (s, 3H). |
| 45 | 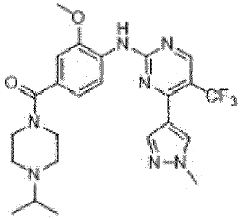 | ¹H NMR (300 MHz, CDCl3) δ 8.66 (s, 1H), 8.55 (d, J = 9.0 Hz, 1H), 8.19 (s, 1H), 8.04 (s, 1H), 8.03 (s, 1H), 7.08 (s, 1H), 7.05 (d, J = 9.0 Hz, 1H), 4.00 (s, 3H), 3.98 (s, 3H), 3.94 - 3.99 (m, 4H), 2.74 (m, 1H), 2.52 - 2.58 (m, 4H), 1.07 (d, J = 6.0 Hz, 6H). |
| 46 | 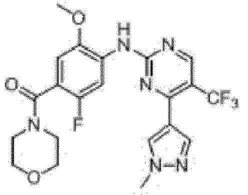 | ¹H NMR (300 MHz, 3hloroform-d) δ 8.69 (s, 1H), 8.47 (d, J = 11.9 Hz, 1H), 8.18 (s, 1H), 8.09 (s, 1H), 8.04 (s, 1H), 6.96 (d, J = 5.9 Hz, 1H), 4.01 (s, 3H), 3.96 (s, 3H), 3.41 - 3.86 (m, 8H). |
| 47 | 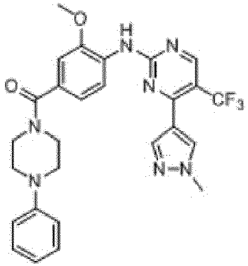 | ¹H NMR (300 MHz, CDCl3) δ 8.65 (s, 1H), 8.57 (d, J = 9.0 Hz, 1H), 8.18 (s, 1H), 3.06 (s, 1H), 8.02 (s, 1H), 7.45 - 7.48 (m, 2H), 7.08 - 7.12 (m, 2H), 6.91 - 6.95 (m, 3H), 3.98 (s, 3H), 3.92 (s, 3H), 3.78 - 3.86 (m, 4H), 3.17 - 3.25 (m, 4H). |
| 48 | 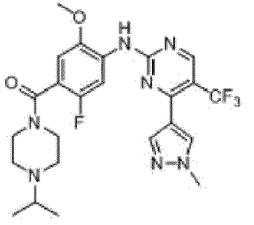 | ¹H NMR(300 MHz, CDCl3) δ 8.68 (s, 1H), 8.46 (d, J = 12.0 Hz, 1H), 8.18 (s, 1H), 8.08 (s, 1H), 8.04 (s, 1H), 6.94 (d, J = 6.0 Hz, 1H), 4.00 (s, 3H), 3.94 (s, 3H). |
| 49 | 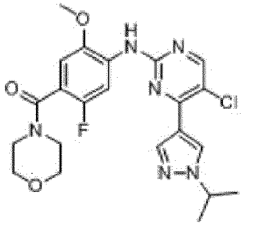 | ¹H NMR(300 MHz, CDCl3) δ 8.46 (d, J = 12.0 Hz, 1H), 8.40 (s, 1H), 8.39 (s, 1H), 8.36 (s, 1H), 7.90 (s, NH), 6.94 (d, J = 6.0 Hz, 1H), 4.59 (m, 1H), 3.94 (s, 3H), 3.80 (s, 4H), 3.67 - 3.71 (m, 2H), 3.43 - 3.47 (m, 2H), 1.60 (d, J = 9.0 Hz, 6H). |
| 50 | 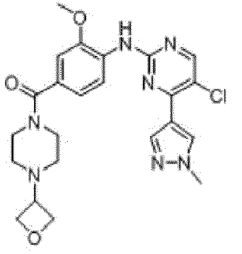 | ¹H NMR (300 MHz, Chloroform-d) δ 8.54 (d, J = 8.7 Hz, 1H), 8.33 - 8.39 (m, 2H), 8.30 (s, 1H), 7.84 (s, 1H), 7.01 - 7.08 (m, 2H), 4.65 (dt, J = 16.8, 6.4 Hz, 4H), 4.00 (s, 3H), 3.95 (s, 3H), 3.60 - 3.82 (m, 4H), 3.52 (p, J = 6.4 Hz, 1H), 2.28 - 2.42 (m, 4H). |
| 51 | 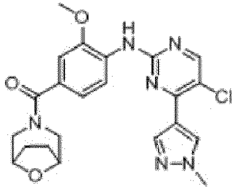 | ¹H NMR (300 MHz, Chloroform-d) δ 8.52 (d, J = 8.7 Hz, 1H), 8.37 - 8.32 (m, 2H), 8.28 (s, 1H), 7.83 (s, 1H), 6.98 - 7.05 (m, 2H), 4.18 - 4.49 (m, 2H), 3.98 (s, 3H), 3.94 (s, 3H), 2.99 - 3.79 (m, 4H), 1.68 - 1.99 (m, 4H). |
| 52 | 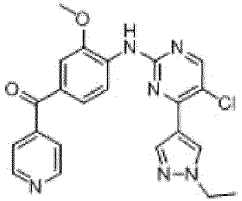 | ¹H NMR (300 MHz, Chloroform-d) δ 8.78 - 8.83 (m, 2H), 8.67 (d, J = 8.5 Hz, 1H), 8.41 (s, 1H), 8.38 (d, J = 0.7 Hz, 1H), 8.34 (d, J = 0.7 Hz, 1H), 8.09 (s, 1H), 7.55 - 7.60 (m, 3H), 7.41 (dd, J = 8.5, 1.9 Hz, 1H), 4.27 (q, J = 7.3 Hz, 2H), 4.03 (s, 3H), 1.57 (t, J = 7.3 Hz, 3H). |
| 53 | 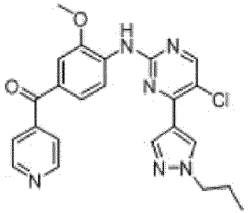 | ¹H NMR (300 MHz, Chloroform-d) δ 8.77 - 8.85 (m, 2H), 8.67 (d, J = 8.5 Hz, 1H), 8.41 (s, 1H), 8.38 (s, 1H), 8.32 (s, 1H), 8.09 (s, 1H), 7.54 - 7.61 (m, 3H), 7.40 (dd, J = 8.5, 1.9 Hz, 1H), 4.17 (t, J = 7.1 Hz, 2H), 4.02 (s, 3H), 1.97 (h, J = 7.4 Hz, 2H), 0.97 (t, J = 7.4 Hz, 3H). |
| 54 | 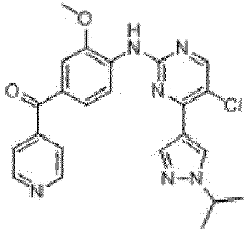 | ¹H NMR (300 MHz, Chloroform-*d*) δ 8.78 - 8.85 (m, 2H), 8.68 (d, J = 8.5 Hz, 1H), 8.40 (d, J = 6.2 Hz, 2H), 8.36 (s, 1H), 8.09 (s, 1H), 7.55 - 7.60 (m, 3H), 7.40 (dd, J = 8.5, 1.9 Hz, 1H), 4.59 (m, J = 6.6 Hz, 1H), 4.03 (s, 3H), 1.59 (d, J = 6.7 Hz, 6H). |
| 55 | 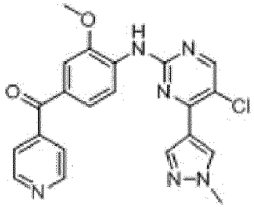 | ¹H NMR (400 MHz, Chloroform-d) δ 8.81 (d, J = 5.0 Hz, 2H), 8.67 (d, J = 8.4 Hz, 1H), 8.39 (d, J = 18.9 Hz, 2H), 8.32 (s, 1H), 8.10 (s, 1H), 7.58 (d, J = 5.0 Hz, 2H), 7.57 (s, 1H), 7.41 (d, J = 8.4 Hz, 1H), 4.03 (s, 3H), 4.01 (s, 3H). |
| 56 | 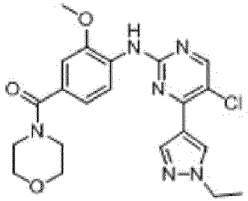 | ¹H NMR (300 MHz, Chloroform-d) δ 8.56 (d, J = 8.3 Hz, 1H), 8.33 - 8.40 (m, 3H), 7.86 (s, 1H), 7.02 - 7.08 (m, 2H), 4.27 (q, J = 7.3 Hz, 2H), 3.96 (s, 3H), 3.60 - 3.81 (m, 8H), 1.57 (t, J = 7.3 Hz, 3H). |
| 57 | 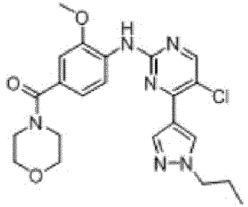 | ¹H NMR (300 MHz, Chloroform-d) δ 8.56 (d, J = 8.6 Hz, 1H), 8.36 - 8.40 (m, 2H), 8.32 (s, 1H), 7.85 (s, 1H), 7.03 - 7.10 (m, 2H), 4.17 (t, J = 7.2 Hz, 2H), 3.96 (s, 2H), 3.63 - 3.77 (m, 8H), 1.97 (m, J = 7.2 Hz, 2H), 0.97 (t, J = 7.4 Hz, 3H). |
| 58 | 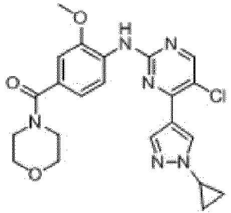 | ¹H NMR (400 MHz, Chloroform-d) δ 8.55 (d, J = 8.2 Hz, 1H), 8.38 (s, 1H), 8.37 (s, 1H), 8.34 (s, 1H), 7.85(s, 1H), 7.06 (d, J = 8.2 Hz, 1H), 7.05 (dd, J = 8.2, 1.8 Hz, 1H) 3.96 (s, 3H), 3.60 - 3.81 (m, 8H), 1.20 -1.26 (m, 2H), 1.06 - 1.14 (m, 2H). |
| 59 | 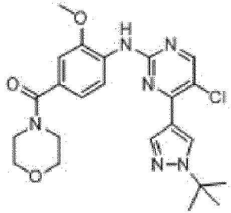 | ¹H NMR (300 MHz, Chloroform-d) δ 8.60 (d, J = 8.2 Hz, 1H), 8.46 (d, J = 0.7 Hz, 1H), 8.43 (d, J = 0.7 Hz,1H), 8.39 (s, 1H), 7.88 (s, 1H), 7.09 (d, J = 1.8 Hz,1H), 7.05 (dd, J = 8.2, 1.8 Hz, 1H), 3.98 (s, 3H),3.64 - 3.80 (m, 8H), 1.69 (s, 9H). |
| 60 | 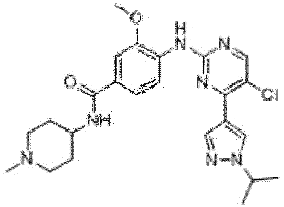 | ¹H NMR (300 MHz, Chloroform-*d*) δ 8.59 (d, *J*= 8.4 Hz, 1H), 8.39 (s, 1H), 8.38 (s, 1H), 8.35 (s, 1H), 7.90(s, 1H), 7.47 (d, *J*= 1.9 Hz, 1H), 7.29 (dd, *J*= 8.4,1.9 Hz, 1H), 5.97 (d, *J*= 8.0 Hz, 1H), 4.52 - 4.66 (m,1H), 4.00 (s, 3H), 2.78 - 2.93 (m, 2H), 2.33 (s, 3H),2.13 - 2.26 (m, 3H), 2.00 - 2.14 (m, 3H), 1.59 (d, *J*= 6.7 Hz, 6H). |
| 61 | 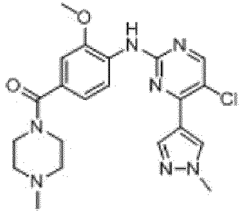 | ¹H NMR (300 MHz, Chloroform-*d*) δ 8.54 (d, *J*= 8.8 Hz, 1H), 8.36 (s, 2H), 8.30 (s, 1H), 7.85 (s, 1H), 7.06(dd, *J*= 8.8, 1.9 Hz, 1H), 7.05 (d, *J*= 1.9 Hz, 1H), 4.00(s, 3H), 3.95 (s, 3H), 3.55 - 3.80 (m, 4H), 2.55 -2.38 (m, 4H), 2.34 (s, 3H). |
| 62 | 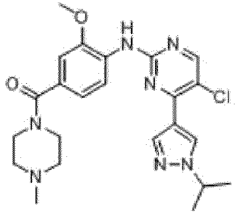 | ¹H NMR (300 MHz, Chloroform-*d*) δ 8.55 (d, *J*= 8.7 Hz, 1H), 8.39 (s, 1H), 8.37 (s, 1H), 8.35 (s, 1H), 7.85(s, 1H), 7.05 (d, *J*= 1.9 Hz, 1H), 7.04 (dd, *J*=8.7, 1.9 Hz, 1H), 4.50 - 4.65 (m, 1H), 3.95 (s, 3H), 3.50 -3.85 (m, 4H), 2.37 - 2.52 (m, 4H), 2.34 (s, 3H), 1.59 (d, *J*= 6.7 Hz, 6H). |
| 63 | 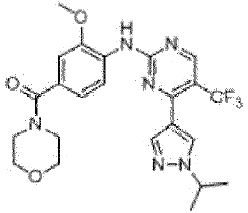 | ¹H NMR (300 MHz, Chloroform-*d*) δ 8.66 (s, 1H), 8.59 (d, *J*= 8.2 Hz, 1H), 8.20 (s, 1H), 8.09 (s, 1H), 8.05(s, 1H), 7.07 (d, *J*= 1.9 Hz, 1H), 7.05 (dd, *J*=8.2, 1.9 Hz, 1H), 4.57 (m, *J*= 6.7 Hz, 1H), 3.97 (s, 3H), 3.60 -3.80 (m, 8H), 1.58 (d, *J*= 6.7 Hz, 6H). |
| 64 | 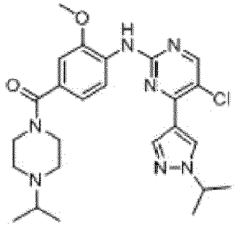 | ¹H NMR (400 MHz, Chloroform-*d*) δ 8.56 (d, *J*= 8.2 Hz, 1H), 8.40 (s, 1H), 8.37 (s, 1H), 8.35 (s, 1H), 7.85(s, 1H), 7.06 (d, , *J*= 1.8 Hz, 1H), 7.05 (dd, , *J*= 8.2, 1.8 Hz, 1H), 4.53 - 4.65 (m, 1H), 3.96 (s, 3H),3.88 - 3.48 (m, 4H), 2.69 - 2.79 (m, 1H), 2.45 - 2.65(m, 4H), 1.59 (d, *J*= 6.7 Hz, 6H), 1.07 (d, *J*= 6.5 Hz, 6H). |
| 65 | 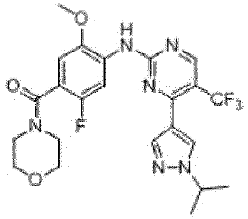 | ¹H NMR (300 MHz, Chloroform-*d*) δ 8.67 (s, 1H), 8.48 (d, *J*= 12.1 Hz, 1H), 8.18 (s, 1H), 8.11 (s, 1H), 8.08 (s, 1H), 6.95 (d, *J*= 5.9 Hz, 1H), 4.50 - 4.65 (m, 1H),3.94 (s, 3H), 3.75 - 3.85 (m, 4H), 3.60 - 3.70 (m,2H), 3.40 - 3.50 (m, 2H), 1.58 (d, *J*= 6.7 Hz, 6H). |
| 66 | 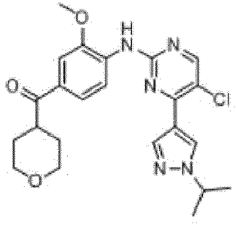 | ¹H NMR (300 MHz, Chloroform-*d*) δ 8.67 (d, *J*= 8.6 Hz, 1H), 8.41 (s, 1H), 8.40 (s, 1H), 8.37 (s, 1H), 8.02(s, 1H), 7.65 (dd, *J*= 8.6, 1.8 Hz, 1H), 7.57 (d, *J*= 1.8 Hz, 1H), 4.52 - 4.67 (m, 1H), 4.03 - 4.12 (m, 2H),4.00 (s, 3H), 3.47 - 3.65 (m, 3H), 1.67 - 2.02 (m,5H), 1.60 (d, *J*= 6.7 Hz, 6H). |
| 67 | 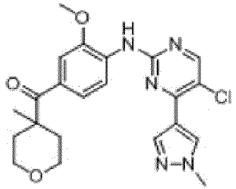 | ¹H NMR (300 MHz, Chloroform-*d*) δ 8.57 (d, *J*= 8.6 Hz, 1H), 8.39 (s, 1H), 8.38 (s, 1H), 8.32 (s, 1H), 7.97(s, 1H), 7.63 (dd, *J*= 8.6, 1.9 Hz, 1H), 7.45 (d, *J*= 1.9 Hz, 1H), 4.02 (s, 3H), 3.99 (s, 3H), 3.73 - 3.82(m, 2H), 3.47 - 3.57 (m, 2H), 2.32 - 2.43 (m, 2H),1.65 - 1.77 (m, 2H), 1.54 (s, 3H). |
| 68 | 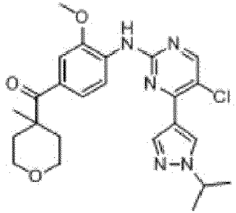 | ¹H NMR (400 MHz, Chloroform-*d*) δ 8.59 (d, *J*= 8.4 Hz, 1H), 8.41 (s, 1H), 8.39 (s, 1H), 8.36 (s, 1H), 7.97(s, 1H), 7.63 (dd, *J*= 8.4, 2.0 Hz, 1H), 7.45 (d, *J*= 2.0 Hz, 1H), 4.53 - 4.66 (m, 1H), 3.99 (s, 3H), 3.72 -3.83 (m, 2H), 3.48 - 3.59 (m, 2H), 2.33 - 2.43 (m,2H), 1.67 - 1.77 (m, 2H), 1.60 (d, *J*= 6.6 Hz, 6H),1.54 (s, 3H). |
| 69 | 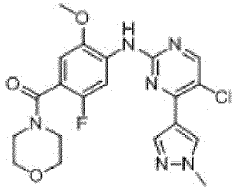 | ¹H NMR(300 MHz, CDCl3) δ 8.42 (d, J = 12.0 Hz, 1H),8.37 (s, 1H), 8.35 (s, 1H), 8.30 (s, 1H), 7.88 (s,NH), 6.92 (d, J = 6.0 Hz, 1H), 4.00 (s, 3H), 3.93 (s, 3H), 3.78 - 3.82 (m, 4H), 3.66 - 3.70 (m, 2H), 3.42 -3.46 (m, 2H). |
| 70 | 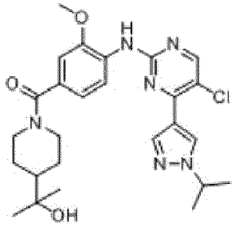 | ¹H NMR (300 MHz, CDCl3) δ 8.53 (d, J = 8.7 Hz, 1H),8.37 (s, 1H), 8.34 (s, 1H), 8.33 (s, 1H), 7.83 (s,1H), 7.00 - 7.06 (m, 2H), 4.56 (m, 1H), 3.93 (s, 3H),2.83 (br s, 2H), 1.58 - 1.96 (m, 3H), 1.51 - 1.57 (m,8H), 1.22 - 1.43 (m, 3H), 1.19 (s, 6H). |
| 71 | 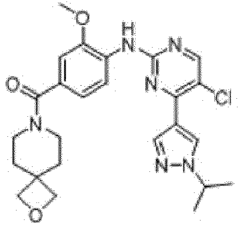 | ¹H NMR (300 MHz, CDCl3) δ 8.54 (d, J = 8.7 Hz, 1H),8.37 (s, 1H), 8.35 (s, 1H), 8.34 (s, 1H), 7.84 (s,1H), 6.98 - 7.04 (m, 2H), 4.57 (m, 1H), 4.47 (s, 4H),3.93 (s, 3H), 3.54 (s, 4H), 1.84 - 1.97 (m, 4H), 1.57 (d, *J* = 6.7 Hz, 6H). |
| 72 | 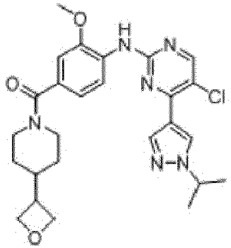 | ¹H NMR(300 MHz, CDCl3) d 7.92(s, 1H), 7.36-7.53(m,7H), 7.30(s, 1H), 6.54(s, 1H), 4.83(t, *J* = 6.6 Hz,1H), 4.54(s, 1H), 4.10-4.17(m, 1H), 3.59-3.67(m, 2H),2.74-2.86 (m, 2H), 1.37(d, *J* = 6.6 Hz, 3H). |
| 73 | 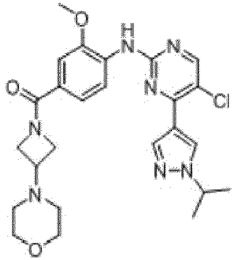 | ¹H NMR (300 MHz, CDCl3) δ 8.56 (d, J = 8.4 Hz, 1H),8.38 (s, 1H), 8.36 (s, 1H), 8.34 (s, 1H), 7.90 (s,1H), 7.33 (d, J = 1.8 Hz, 1H), 7.21 (dd, J = 8.4, 1.8Hz, 1H), 4.57 (m, 1H), 4.00 - 4.40 (m, 4H), 3.96 (s,3H), 3.74 (t, J = 4.6 Hz, 4H), 3.19 (m, 1H), 2.38 -2.40 (m, 4H), 1.58 (d, J = 6.7 Hz, 6H). |
| 74 | 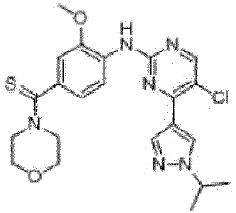 | ¹H NMR(300 MHz, CDCl3) δ 8.51 (d, J = 8.4 Hz, 1H),8.38 (s, 1H), 8.36 (s, 1H), 8.34 (s, 1H), 7.82 (s,1H), 7.01 (d, J = 1.9 Hz, 1H), 6.89 (dd, J = 8.4, 1.9Hz, 1H), 4.58 (m, 1H), 4.44 (s, 2H), 3.95 (s, 3H),3.86 - 3.90 (m, 2H), 3.62 - 3.80 (m, 4H), 1.58 (d, J =6.6 Hz, 6H). |
| 75 | 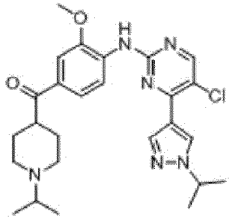 | ¹H NMR (300 MHz, Chloroform-*d*) δ 8.64 (d, *J*= 8.5 Hz, 1H), 8.40 (s, 1H), 8.38 (s, 1H), 8.35 (s, 1H), 8.00(s, 1H), 7.63 (dd, *J*= 8.5, 1.8 Hz, 1H), 7.55 (d, *J*= 1.8 Hz, 1H), 4.59 (m, 1H), 3.98 (s, 3H), 2.94 - 3.05(m, 2H), 2.73 (m, 1H), 2.30 - 2.44 (m, 2H), 1.82 -1.99 (m, 4H), 1.59 (d, *J*= 6.7 Hz, 6H), 1.10 (d, *J*= 6.6 Hz, 6H). |
| 76 | 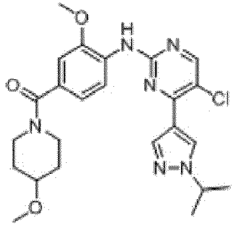 | ¹H NMR (300 MHz, Chloroform-*d*) δ 8.53 (d, *J*= 8.7 Hz, 1H), 8.38 (s, 1H), 8.36 (s, 1H), 8.35 (s, 1H), 7.87(s, 1H), 6.98 - 7.08 (m, 2H), 4.58 (m, 1H), 3.74 -4.06 (m, 5H), 3.29 - 3.53 (m, 6H), 1.80 - 1.99 (m,2H), 1.52 - 1.71 (m, 2H),1.58 (d, *J*= 6.6 Hz, 6H). |
| 77 | 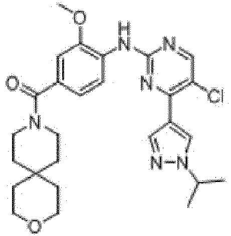 | ¹H NMR (300 MHz, Chloroform-*d*) δ 8.54 (d, *J*= 8.7 Hz, 1H), 8.39 (s, 1H), 8.36 (s, 1H), 8.35 (s, 1H), 7.86(s, 1H), 7.01 - 7.06 (m, 2H), 4.58 (m, 1H), 3.94 (s,3H), 3.48 - 3.76 (m, 8H), 1.49 - 1.68 (m, 8H),1.58 (d, *J*= 6.6 Hz, 6H). |
| 78 | 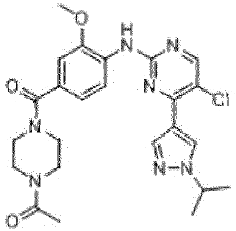 | ¹H NMR (300 MHz, Chloroform-*d*) δ 8.58 (d, *J*= 8.1 Hz, 1H), 8.39 (s, 1H), 8.37 (s, 1H), 8.35 (s, 1H), 7.89(s, 1H), 7.02 - 7.08 (m, 2H), 4.58 (m, 1H), 3.96 (s,3H), 3.59 - 3.76 (m, 6H), 3.47 - 3.56 (m, 2H), 2.14 (s, 3H), 1.58 (d, *J*= 6.6 Hz, 6H). |
| 79 | 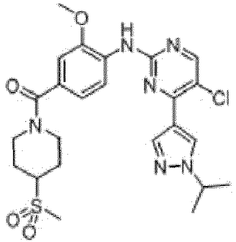 | ¹H NMR (300 MHz, Chloroform-*d*) δ 8.56 (d, *J*= 8.7 Hz, 1H), 8.38 (s, 1H), 8.36 (s, 1H), 8.35 (s, 1H), 7.88(s, 1H), 7.00 - 7.06 (m, 2H), 4.57 (m, 1H), 4.30 -4.68 (m, 2H) 3.95 (s, 3H), 3.10 (m, 1H), 2.91 - 3.04(m, 2H), 2.88 (s, 3H), 2.12 - 2.26 (m, 2H), 1.74 -1.94 (m, 2H), 1.58 (d, *J*= 6.7 Hz, 6H). |
| 80 | 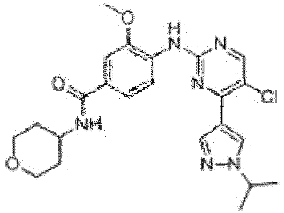 | ¹H NMR (300 MHz, CDCl3) δ 8.57 (d, J = 8.4 Hz, 1H),8.37 (s, 1H), 8.36 (s, 1H), 8.34 (s, 1H), 7.89 (s,1H), 7.46 (d, J = 1.9 Hz, 1H), 7.29 (dd, J = 8.4, 1.9Hz, 1H), 6.07 (d, J = 7.8 Hz, 1H), 4.58 (m, 1H), 4.20(m, 1H), 3.98 - 4.03 (m, 5H), 3.50 - 3.59 (m, 2H),1.97 - 2.05 (m, 2H), 1.57 - 1.59 (m, 8H). |
| 81 | 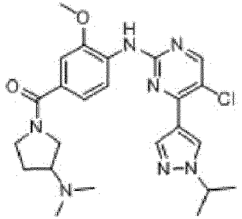 | ¹H NMR (300 MHz, CDCl3) δ 8.55 (d, J = 8.4 Hz, 1H),8.39 (s, 1H), 8.36 (s, 1H), 8.34 (s, 1H), 7.87 (s,1H), 7.17 - 7.20 (m, 2H), 4.58 (m, 1H), 3.95 (s, 3H),3.63 - 3.90 (m, 3H), 3.42 (m, 1H), 2.71 (m, 1H), 2.31(s, 3H), 2.23 (s, 4H), 1.74 - 2.02 (m, 3H), 1.58 (d, J= 6.7 Hz, 6H). |
| 82 | 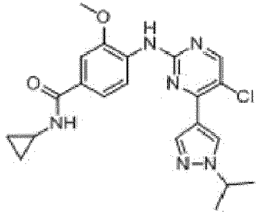 | ¹H NMR (300 MHz, CDCl3) δ 8.55 (d, J = 8.4 Hz, 1H),8.37 (s, 1H), 8.37 (s, 1H), 8.34 (s, 1H), 7.89 (s,1H), 7.47 (d, J = 1.9 Hz, 1H), 7.23 (d, J = 1.9 Hz,1H), 6.26 (br s, 1H), 4.58 (m, 1H), 3.98 (s, 3H), 2.91(m, 1H), 1.59 (d, J = 6.7 Hz, 6H), 0.85 - 0.91 (m,2H), 0.59 - 0.67 (m, 2H). |
| 83 | 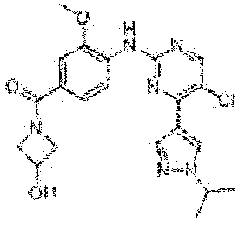 | ¹H NMR (300 MHz, CDCl3) δ 8.55 (d, J = 8.5 Hz, 1H),8.38 (s, 1H), 8.36 (s, 1H), 8.34 (s, 1H), 7.90 (s,1H), 7.32 (d, J = 1.8 Hz, 1H), 7.20 (dd, J = 8.5, 1.8Hz, 1H), 4.73 (br s, 1H), 4.57 (m, 3H), 4.02 - 4.32(m, 2H), 3.96 (s, 3H), 2.95 (m, 1H), 1.59 (d, J = 6.7 Hz, 6H). |
| 84 | 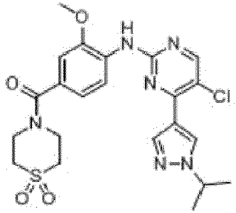 | ¹H NMR (300 MHz, Chloroform-*d*) δ 8.60 (dd, *J*= 8.8, 2.2 Hz, 1H), 8.37 (s, 1H), 8.36 (s, 1H), 8.34 (s, 1H),7.89 (s, 1H), 6.98 - 7.11 (m, 2H), 4.57 (m, 1H), 4.06- 4.22 (m, 4H), 3.96 (s, 3H), 2.97 - 3.21 (m, 4H),1.57 (d, *J*= 6.7 Hz, 6H). |
| 85 | 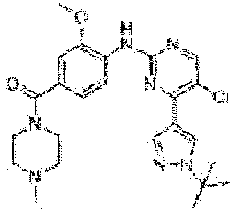 | ¹H NMR (300 MHz, Chloroform-*d*) δ 8.57 (d, *J*= 8.6 Hz, 1H), 8.43 (s, 1H), 8.41 (s, 1H), 8.36 (s, 1H), 7.85(s, 1H), 7.01 - 7.08 (m, 2H), 3.95 (s, 3H), 3.58 -3.83 (m, 4H), 2.41 - 2.54 (m, 4H), 2.36 (s, 3H), 1.67 (s, 9H). |
| 86 | 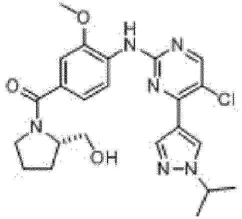 | ¹H NMR (300 MHz, CDCl3) δ 8.57 (d, J = 8.2 Hz, 1H),8.39 (s, 1H), 8.37 (s, 1H), 8.35 (s, 1H), 7.88 (s,1H), 7.14 - 7.21 (m, 2H), 5.03 (br s, 1H), 4.58 (m,1H), 4.44 (m, 1H), 3.96 (s, 3H), 3.65 - 3.89 (m, 3H),3.57 (m, 1H), 2.18 (m, 1H), 1.88 (m, 1H), 1.64 - 1.81 (m, 3H), 1.59 (d, J = 6.7 Hz, 6H). |
| 87 | 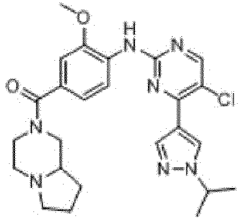 | ¹H NMR (300 MHz, CDCl3) δ 8.55 (d, J = 8.7 Hz, 1H),8.39 (s, 1H), 8.36 (s, 1H), 8.34 (s, 1H), 7.85 (s,1H), 7.01 - 7.08 (m, 2H), 4.53 - 4.55 (m, 2H), 3.95(s, 3H), 3.09 - 3.15 (m, 3H), 2.14 - 2.23 (m, 2H),1.95 - 2.01 (m, 2H), 1.75 - 1.90 (m, 3H), 1.58 (d, J =6.7 Hz, 6H), 1.42 (m, 1H). |
| 88 | 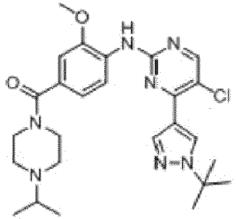 | ¹H NMR (300 MHz, Chloroform-*d*) δ 8.56 (d, *J*= 8.1 Hz, 1H), 8.43 (d, *J*= 0.7 Hz, 1H), 8.41 (d, *J*= 0.7 Hz, 1H),8.36 (s, 1H), 7.84 (s, 1H), 7.01 - 7.07 (m, 2H), 3.95(s, 3H), 3.57 - 3.86 (m, 4H), 2.76 (m, 1H), 2.48 -2.64 (m, 4H), 1.66 (s, 9H), 1.08 (d, *J*= 6.5 Hz, 6H). |
| 89 | 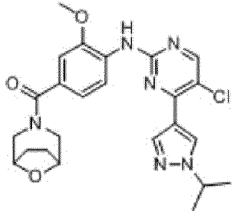 | ¹H NMR (300 MHz, Chloroform-*d*) δ 8.54 (d, *J* = 8.7 Hz, 1H), 8.38 (s, 1H), 8.36 (s, 1H), 8.35 (s, 1H), 7.86(s, 1H), 6.99 - 7.04 (m, 2H), 4.58 (m, 1H), 4.22 -4.47 (m, 2H), 3.95 (s, 3H), 3.07 - 3.78 (m, 3H), 1.67- 2.06 (m, 4H), 1.58 (d, *J*= 6.7 Hz, 6H). |
| 90 | 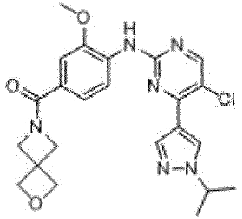 | ¹H NMR (300 MHz, Chloroform-*d*) δ 8.57 (d, *J*= 8.4 Hz, 1H), 8.38 (s, 1H), 8.36 (s, 1H), 8.35 (s, 1H), 7.91(s, 1H), 7.31 (d, *J*= 1.8 Hz, 1H), 7.19 (dd, *J*= 8.4,1.8 Hz, 1H), 4.74 - 4.88 (m, 4H), 4.58 (m, 1H), 4.27 -4.56 (m, 4H), 3.96 (s, 3H), 1.58 (d, *J*= 6.7 Hz, 6H). |
| 91 | 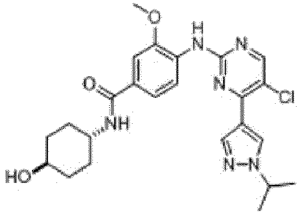 | ¹H NMR (300 MHz, Chloroform-*d*) δ 8.56 (d, *J*= 8.4 Hz, 1H), 8.37 (s, 1H), 8.36 (s, 1H), 8.35 (s, 1H), 7.91(s, 1H), 7.46 (d, *J*= 1.9 Hz, 1H), 7.26 (dd, *J*= 8.4,1.9 Hz, 1H), 5.94 (d, *J*= 7.9 Hz, 1H), 4.58 (m, 1H),3.98 (s, 3H), 3.96 (m, 1H), 3.67 (m, 1H), 2.09 - 2.19(m, 2H), 1.98 - 2.09 (m, 2H), 1.74 (br s, 1H), 1.59(d, *J*= 6.7 Hz, 6H), 1.41 - 1.53 (m, 2H), 1.27 - 1.41(m, 2H). |
| 92 | 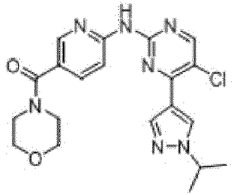 | ¹H NMR (300 MHz, Chloroform-*d*) δ 9.03 (br s, 1H),8.46 - 8.53 (m, 3H), 8.38 (d, *J*= 0.7 Hz, 1H), 8.36 (d, *J*= 0.7 Hz, 1H), 7.82 (dd, *J*= 8.7, 2.3 Hz, 1H), 4.59(m, 1H), 3.60 - 3.80 (m, 8H), 1.59 (d, *J*= 6.7 Hz, 6H). |
| 93 | 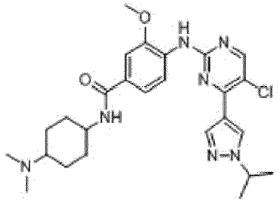 | ¹H NMR (300 MHz, Chloroform-*d*) δ 8.58 (d, *J*= 8.4 Hz, 1H), 8.40 (s, 1H), 8.38 (s, 1H), 8.35 (s, 1H), 7.90(s, 1H), 7.48 (d, *J*= 1.8 Hz, 1H), 7.36 (dd, *J*= 8.4,1.8 Hz, 1H), 6.32 (s, 1H), 4.61 (m, 1H), 4.27 (s, 1H),4.00 (s, 3H), 2.45 (s, 6H), 2.38 (m, 1H), 1.88 - 2.05 (m, 2H), 1.64 - 1.87 (m, 6H), 1.60 (d, *J*= 6.7 Hz, 6H). |
| 94 | 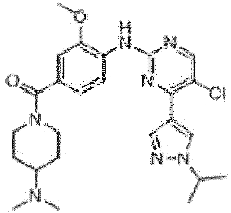 | ¹H NMR (300 MHz, Chloroform-*d*) δ 8.55 (d, *J*= 8.7 Hz, 1H), 8.39 (s, 1H), 8.36 (s, 1H), 8.35 (s, 1H), 7.84(s, 1H), 6.99 - 7.10 (m, 2H), 4.58 (m, 1H), 4.18 -4.53 (m, 2H), 3.95 (s, 3H), 2.83 - 3.03 (m, 2H), 2.46(m, 1H), 2.34 (s, 6H), 1.83 - 1.98 (m, 2H), 1.59 (d, *J*= 6.7 Hz, 6H), 1.41 - 1.58 (m, 2H). |
| 95 | 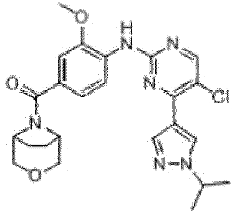 | ¹H NMR (300 MHz, Chloroform-*d*) δ 8.56 (d, *J*= 8.2 Hz, 1H), 8.38 (s, 1H), 8.36 (s, 1H), 8.34 (s, 1H), 7.88(s, 1H), 7.16 (d, *J*= 1.7 Hz, 1H), 7.12 (dd, *J*= 8.2,1.7 Hz, 1H), 4.67 (m, 1H), 4.58 (m, 1H), 4.20 (m, 1H),3.96 (s, 3H), 3.74 - 3.90 (m, 2H), 3.59 - 3.73 (m,2H), 1.86 - 2.09 (m, 4H), 1.58 (d, *J*= 6.7 Hz, 6H). |
| 96 | 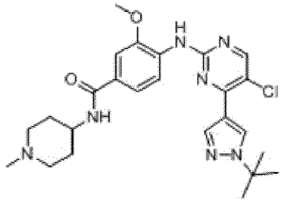 | ¹H NMR (300 MHz, CDCl3) δ 8.59 (d, *J*= 8.4 Hz, 1H),8.42 (s, 1H), 8.40 (s, 1H), 8.36 (s, 1H), 7.89 (s,1H), 7.46 (d, *J*= 1.9 Hz, 1H), 7.29 (d, *J*= 1.9 Hz, 1H),6.00 (d, *J*= 8.0 Hz, 1H), 3.99 - 4.05 (m, 4H), 2.86 (d, *J*= 11.8 Hz, 2H), 2.33 (s, 3H), 2.13 - 2.28 (m, 3H),2.04 (s, 3H), 1.67 (s, 9H). |
| 97 | 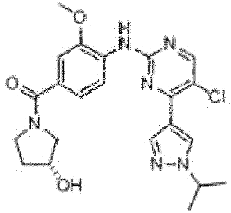 | MS *m*/*z* : 458.2 [M+1] |
| 98 | 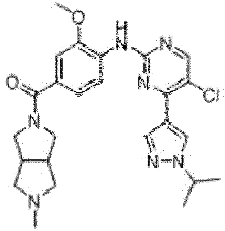 | ¹H NMR(300 MHz, CDCl3) d 7.92(s, 1H), 7.36-7.53(m, 7H), 7.30(s, 1H), 6.54(s, 1H), 4.83 (t, *J* = 6.6 Hz, 1H), 4.54 (s, 1H), 4.10-4.17 (m, 1H), 3.59-3.67 (m, 2H), 2.74-2.86 (m, 2H), 1.37(d, *J* = 6.6 Hz, 3H). |
| 99 | 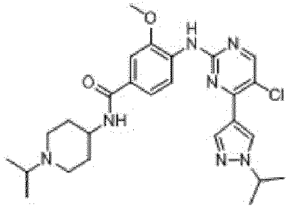 | 1H NMR (300 MHz, CDCl3) δ 8.58 (d, J = 8.4 Hz, 1H),8.39 (s, 1H), 8.37 (s, 1H), 8.35 (s, 1H), 7.90 (s,1H), 7.47 (d, J = 1.9 Hz, 1H), 7.29 (m, 1H), 5.99 (d,J = 8.0 Hz, 1H), 4.59 (m, 1H), 3.99 - 4.02 (m, 4H),2.84 - 2.93 (m, 2H), 2.80 (m, 1H), 2.37 (t, J = 11.3Hz, 2H), 2.07 - 2.11 (m, 2H), 1.58 - 1.64 (m, 8H),1.09 (d, J = 6.5 Hz, 6H). |
| 100 | 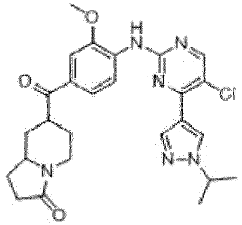 | ¹H NMR (300 MHz, Chloroform-*d*) δ 8.57 (d, *J*= 8.3 Hz, 1H), 8.38 (s, 1H), 8.36 (s, 1H), 8.35 (s, 1H), 7.90(s, 1H), 7.00 - 7.08 (m, 2H), 4.57 (m, 1H), 4.18 -4.45 (m, 2H), 4.07 (m, 1H), 3.95 (s, 3H), 3.62 (m,1H), 2.77 - 3.03 (m, 2H), 2.63 (m, 1H), 2.37 - 2.48(m, 2H), 2.21 (m, 1H), 1.57 (d, *J*= 6.7 Hz, 6H). |
| 101 | 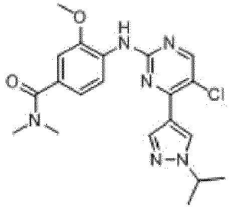 | ¹H NMR (300 MHz, CDCl3) δ 8.54 (d, *J*= 8.7 Hz, 1H),8.39 (s, 1H), 8.36 (s, 1H), 8.34 (s, 1H), 7.84 (s,1H), 7.05 - 7.08 (m, 2H), 4.58 (m, 1H), 3.95 (s, 3H),3.09 (s, 6H), 1.58 (d, *J*= 6.6 Hz, 6H). |
| 102 | 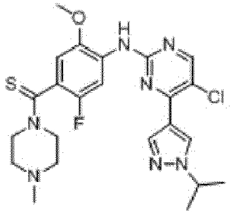 | ¹H NMR (300 MHz, Chloroform-*d*) δ 8.42 (d, *J*= 12.5 Hz, 1H), 8.38 - 8.39 (m, 2H), 8.36 (d, *J*= 0.7 Hz, 1H),7.87 (s, 1H), 6.98 (d, *J*= 6.4 Hz, 1H), 4.59 (m, 1H),4.32 - 4.66 (m, 2H), 3.94 (s, 3H), 3.65 - 3.92 (m,2H), 2.57 - 2.91 (m, 4H), 2.48 (s, 3H), 1.59 (d, *J*= 6.7 Hz, 6H). |
| 103 | 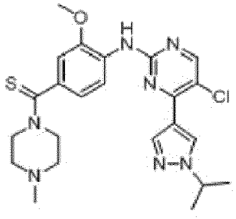 | ¹H NMR (300 MHz, Chloroform-*d*) δ 8.51 (d, *J*= 8.4 Hz, 1H), 8.38 (s, 1H), 8.36 (s, 1H), 8.34 (s, 1H), 7.82(s, 1H), 7.00 (d, *J*= 1.9 Hz, 1H), 6.91 (dd, *J*= 8.4,1.9 Hz, 1H), 4.57 (m, 1H), 4.33 - 4.64 (m, 2H). 3.95(s, 3H), 3.73 - 3.87 (m, 2H), 2.62 - 2.81 (m, 2H),2.46 - 2.60 (m, 2H), 2.42 (s, 3H), 1.59 (d, *J*= 6.7 Hz,6H). |
| 104 | 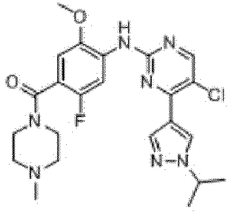 | ¹H NMR (400 MHz, Chloroform-*d*) δ 8.43 (d, *J*= 12.2 Hz, 1H), 8.37 (s, 1H), 8.36 (s, 1H), 8.34 (s, 1H), 7.86(s, 1H), 6.89 (d, *J*= 5.9 Hz, 1H), 4.57 (m, 1H), 3.91(s, 3H), 3.76 - 3.85 (m, 2H), 3.43 (d, *J*= 6.0 Hz, 2H),2.49 (t, *J*= 5.1 Hz, 2H), 2.39 (d, *J*= 5.0 Hz, 2H), 2.32 (s, 3H), 1.57 (d, *J*= 6.7 Hz, 6H). |
| 105 | 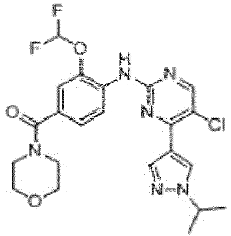 | ¹H NMR (300 MHz, CDCl3) δ 8.67 (d, J = 9.1 Hz, 1H),8.39 (s, 1H), 8.37 (s, 1H), 8.36 (s, 1H), 7.67 (s,1H), 7.28 - 7.33 (m, 2H), 6.63 (t, J = 72.9 Hz, 1H),4.59 (m, 1H), 3.50 - 3.75 (m, 8H), 1.59 (d, J = 6.7 Hz, 6H). |
| 106 | 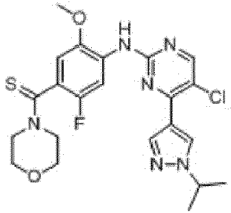 | ¹H NMR (300 MHz, CDCl3) δ 8.48 (s, 1H), 8.38 - 8.43 (m, 2H), 8.34 (d, J = 12.2 Hz, 1H), 7.00 (d, J = 6.4Hz, 1H), 4.60 (p, J = 6.7 Hz, 1H), 4.45 (q, J = 4.9Hz, 2H), 3.95 (s, 3H), 3.89 (t, J = 4.9 Hz, 2H), 3.71- 3.83 (m, 2H), 3.53 - 3.70 (m, 2H), 1.59 (d, J = 6.7 Hz, 6H). |
| 107 | 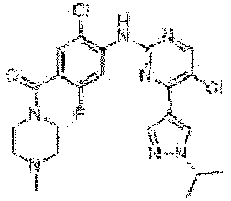 | ¹H NMR (300 MHz, Chloroform-*d*) δ 8.49 (dd, *J*= 8.8, 1.4 Hz, 1H), 8.40 (s, 1H), 8.36 (d, *J*= 0.7 Hz, 1H),8.34 (d, *J*= 0.7 Hz, 1H), 7.68 (s, 1H), 7.33 (dd, *J*=8.8, 7.2 Hz, 1H), 4.58 (m, 1H), 3.78 - 3.90 (m, 2H),3.35 - 3.48 (m, 2H), 2.48 - 2.58 (m, 2H), 2.39 - 2.47(m, 2H), 2.35 (s, 3H), 1.58 (d, *J*= 6.7 Hz, 6H). |
| 108 | 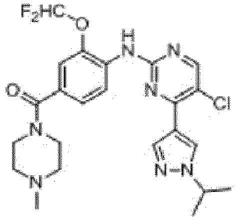 | ¹H NMR (300 MHz, CDCl3) δ 8.64 - 8.71 (m, 1H), 8.39(s, 1H), 8.37 (d, *J*= 0.7 Hz, 1H), 8.35 (d, *J*= 0.7 Hz,1H), 7.63 (s, 1H), 7.30 - 7.33 (m, 2H), 6.63 (t, *J*= 73.0 Hz, 1H), 4.59 (m, 1H), 3.73 - 3.90 (m, 4H), 2.41- 2.60 (m, 4H), 2.39 (s, 3H), 1.59 (d, *J*= 6.7 Hz, 6H). |
| 109 | 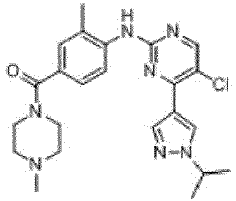 | ¹H NMR (300 MHz, CDCl3) δ 8.34 (s, 1H), 8.33 (s, 1H),8.31 (s, 1H), 8.24 (m, 1H), 8.12 (m, 1H), 7.32 (m,1H), 7.03 (br s, 1H), 4.57 (m, 1H), 3.60 - 3.79 (m,4H), 2.45 - 2.50 (m, 4H), 2.36 (s, 6H), 1.57 (d, *J*=6. 7 Hz, 6H). |
| 110 | 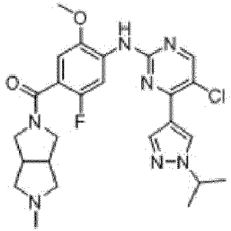 | ¹H NMR (500 MHz, Chloroform-*d*) δ 8.45 (d, *J*= 12.3 Hz, 1H), 8.38 (d, *J*= 0.7 Hz, 1H), 8.38 (s, 1H), 8.35 (d, *J*= 0.7 Hz, 1H), 7.89 (d, *J*= 1.4 Hz, 1H), 6.90 (d, *J*= 5.9 Hz, 1H), 4.88 (m, 1H), 4.58 (m, 1H), 3.93 (s, 3H),3.86 (m, 1H), 2.99 - 3.18 (m, 2H), 2.80 (m, 1H), 2.62(s, 6H), 2.19 (m, 1H), 2.10 (m, 1H), 1.62 - 1.74 (m, 2H), 1.58 (d, *J*= 6.7 Hz, 6H). |
| 111 | 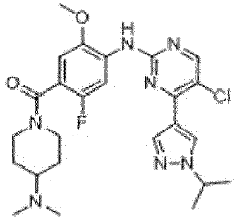 | ¹H NMR (500 MHz, Chloroform-*d*) δ 8.45 (d, *J*= 12.3 Hz, 1H), 8.38 (d, *J*= 0.7 Hz, 1H), 8.38 (s, 1H), 8.35 (d, *J*= 0.7 Hz, 1H), 7.89 (d, *J*= 1.4 Hz, 1H), 6.90 (d, *J*= 5.9 Hz, 1H), 4.88 (m, 1H), 4.58 (m, 1H), 3.93 (s, 3H),3.86 (m, 1H), 2.99 - 3.18 (m, 2H), 2.80 (m, 1H), 2.62(s, 6H), 2.19 (m, 1H), 2.10 (m, 1H), 1.62 - 1.74 (m, 2H), 1.58 (d, *J*= 6.7 Hz, 6H). |
| 112 | 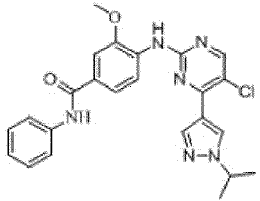 | ¹H NMR (300 MHz, CDCl3) δ 8.60 (d, *J*= 8.5 Hz, 1H),8.43 (s, 1H), 8.41 (s, 1H), 8.38 (s, 1H), 8.24 (s,1H), 7.88 (s, 1H), 7.64 - 7.71 (m, 2H), 7.56 (d, *J*=1.9 Hz, 1H), 7.35 - 7.47 (m, 3H), 7.15 (m, 1H), 4.60(m. 1H), 4.02 (s, 3H), 1.60 (d, *J*= 6.7 Hz, 6H). |
| 113 | 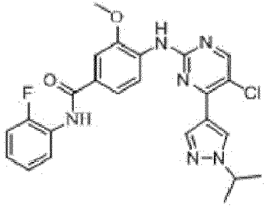 | ¹H NMR (300 MHz, MeOD) δ 8.62 (d, *J*= 8.5 Hz, 1H),8.57 (d, *J*= 0.7 Hz, 1H), 8.45 (s, 1H), 8.36 (d, *J*= 0.7Hz, 1H), 7.74 (m, 1H), 7.68 (dd, *J*= 8.5, 2.0 Hz, 1H),7.63 (d, *J*= 2.0 Hz, 1H), 7.17 - 7.28 (m, 3H), 4.66 (m,1H), 4.04 (s, 3H), 1.57 (d, *J*= 6.7 Hz, 6H). |
| 114 | 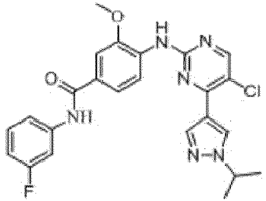 | ¹H NMR (300 MHz, MeOD) δ 8.61 (d, *J*= 8.5 Hz, 1H),8.56 (d, *J*= 0.7 Hz, 1H), 8.44 (s, 1H), 8.34 (d, *J*= 0.7Hz, 1H), 7.62 - 7.70 (m, 2H), 7.60 (d, *J*= 2.0 Hz, 1H),7.46 (dd, *J*= 8.5, 2.0, 1H), 7.35 (m, 1H), 6.86 (m,1H), 4.65 (m, 1H), 4.04 (s, 3H), 1.57 (d, *J*= 6.7 Hz, 6H). |
| 115 | 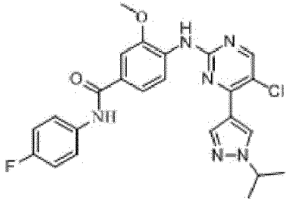 | ¹H NMR (300 MHz, MeOD) δ 8.60 (d, *J*= 8.5 Hz, 1H),8.57 (d, *J*= 0.8 Hz, 1H), 8.44 (s, 1H), 8.35 (d, *J*= 0.8Hz, 1H), 7.62 - 7.73 (m, 3H), 7.60 (d, *J*= 2.0 Hz, 1H),7.07 - 7.13 (m, 2H), 4.65 (m, 1H), 4.04 (s, 3H), 1.57 (d, *J*= 6.7 Hz, 6H). |
| 116 | 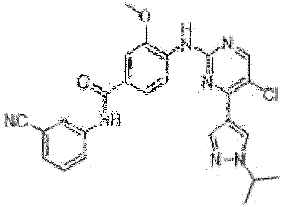 | ¹H NMR (300 MHz, MeOD) δ 8.35 - 8.42 (m, 2H), 8.17 -8.22 (m, 2H), 8.10 (d, *J*= 1.9 Hz, 1H), 7.97 (s, 1H),7.89 (m, 1H), 7.35 - 7.50 (m, 4H), 4.58 (m, 1H), 3.92(s, 3H), 2.99 (s, 3H), 2.86 (s, 3H), 1.54 (d, *J*= 6.7 Hz, 6H). |
| 117 | 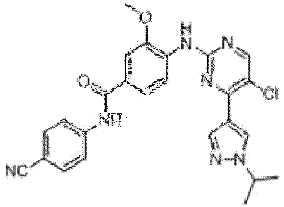 | ¹H NMR (300 MHz, CDCl3) δ 8.64 (d, *J*= 8.5 Hz, 1H),8.36 - 8.46 (m, 3H), 8.13 (d, *J*= 19.7 Hz, 2H), 7.83(d, *J*= 8.7 Hz, 2H), 7.63 - 7.72 (m, 2H), 7.54 (d, *J*= 1.9 Hz, 1H), 7.45 (m, 1H), 4.60 (m, 1H), 4.02 (s, 3H),1.60 (d, *J*= 6.7 Hz, 6H). |
| 118 | 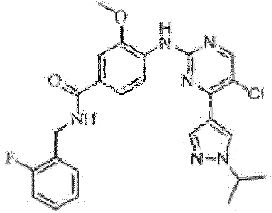 | ¹H NMR (300 MHz, MeOD) δ 8.31 - 8.40 (m, 2H), 8.10 -8.20 (m, 2H), 7.34 - 7.48 (m, 3H), 7.26 (m, 1H), 7.01- 7.16 (m, 2H), 4.46 - 4.64 (m, 3H), 3.84 (d, *J*= 2.2Hz, 3H), 1.50 (dd, *J*= 6.8, 2.2 Hz, 6H). |
| 119 | 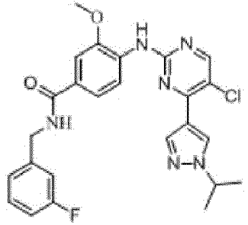 | ¹H NMR (300 MHz, MeOD) δ 8.48 - 8.54 (m, 2H), 8.37(s, 1H), 8.30 (s, 1H), 7.48 - 7.57 (m, 2H), 7.34 (m,1H), 7.18 (m, 1H), 7.11 m, 1H), 6.98 (m, 1H), 4.55 -4.68 (m, 3H), 3.97 (s, 3H), 1.55 (d, *J*= 6.7 Hz, 6H). |
| 120 | 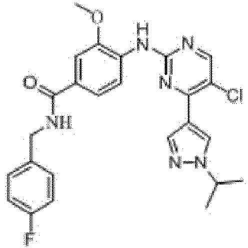 | ¹H NMR (300 MHz, MeOD) δ 8.50 - 8.59 (m, 2H), 8.42(s, 1H), 8.33 (s, 1H), 7.50 - 7.59 (m, 2H), 7.34 -7.45 (m, 2H), 7.06 (t, *J*= 8.8 Hz, 2H), 4.65 (m, 1H),4.56 (s, 2H), 4.00 (s, 3H), 1.56 (d, *J*= 6.7 Hz, 6H). |
| 121 | 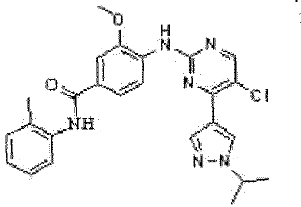 | ¹H NMR (300 MHz, MeOD) δ 8.57 (d, *J*= 8.5 Hz, 1H),8.52 (d, *J*= 0.7 Hz, 1H), 8.37 (s, 1H), 8.31 (d, *J*= 0.7Hz, 1H), 7.65 (dd, *J*= 8.5, 2.0 Hz, 1H), 7.59 (d, *J*=2.0 Hz, 1H), 7.16 - 7.37 (m, 4H), 4.63 (m, 1H), 4.00(s, 3H), 2.32 (s, 3H), 1.55 (d, *J*= 6.7 Hz, 6H). |
| 122 | 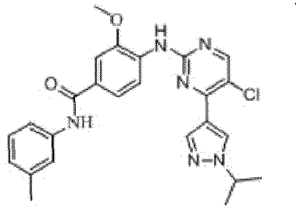 | ¹H NMR (300 MHz, MeOD) δ 8.48 - 8.54 (m, 2H), 8.33(s, 1H), 8.28 (d, *J*= 0.7 Hz, 1H), 7.58 (dd, *J*= 8.5,2.0 Hz, 1H), 7.45 - 7.54 (m, 3H), 7.22 (t, *J*= 7.8 Hz,1H), 6.96 (m, 1H), 4.61 (m, 1H), 3.98 (s, 3H), 2.35(d, *J*= 0.8 Hz, 3H), 1.55 (d, *J*= 6.7 Hz, 6H). |
| 123 | 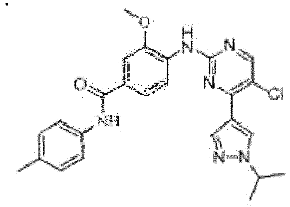 | ¹H NMR (300 MHz, MeOD) δ 8.32 - 8.37 (m, 2H), 8.15(d, *J*= 0.7 Hz, 1H), 8.14 (s, 1H), 7.52 (d, *J*= 2.0 Hz,1H), 7.50 (d, *J*= 2.0 Hz, 1H), 7.45 (dd, *J*= 8.5, 2.0Hz, 1H), 7.39 (d, *J*= 2.0 Hz, 1H), 7.12 (m, 1H), 7.08 -7.11 (m, 1H), 4.53 (m, 1H), 3.86 (s, 3H), 2.29 (s,3H), 1.50 (d, *J*= 6.7 Hz, 6H). |
| 124 | 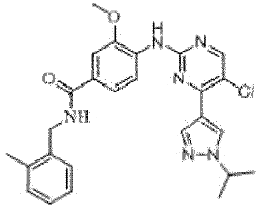 | ¹H NMR (300 MHz, MeOD) δ 8.50 - 8.52 (m, 2H), 8.36(s, 1H), 8.30 (d, *J*= 0.7 Hz, 1H), 7.49 - 7.57 (m, 2H),7.28 - 7.30 (m, 1H), 7.13 - 7.20 (m, 3H), 4.56 - 4.68(m, 3H), 3.97 (s, 3H), 2.38 (s, 3H), 1.55 (d, *J*= 6.7 Hz, 6H). |
| 125 | 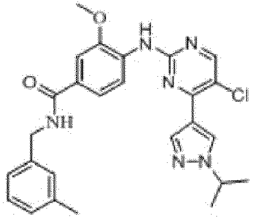 | ¹H NMR (300 MHz, MeOD) δ 8.49 - 8.59 (m, 2H), 8.42(s, 1H), 8.34 (d, *J*= 0.7 Hz, 1H), 7.51 - 7.60 (m, 2H),7.04 - 7.26 (m, 4H), 4.54 - 4.73 (m, 4H), 4.01 (s,3H), 2.35 (d, *J*= 0.8 Hz, 3H), 1.57 (d, *J*= 6.7 Hz, 6H). |
| 126 | 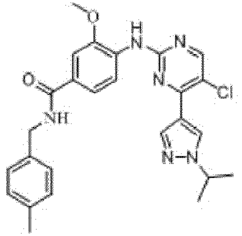 | ¹H NMR (300 MHz, MeOD) δ 8.36 - 8.44 (m, 2H), 8.21(d, *J*= 1.1 Hz, 2H), 7.38 - 7.48 (m, 2H), 7.20 - 7.28(m, 2H), 7.08 - 7.16 (m, 2H), 4.48 - 4.62 (m, 3H),3.88 (s, 3H), 2.30 (s, 3H), 1.51 (d, *J*= 6.7 Hz, 6H). |
| 127 | 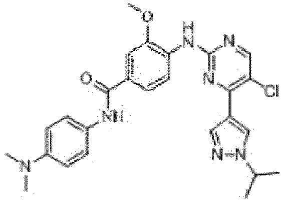 | ¹H NMR (300 MHz, MeOD) δ 8.54 - 8.60 (m, 2H), 8.42(s, 1H), 8.34 (d, *J*= 0.7 Hz, 1H), 7.56 - 7.65 (m, 2H),7.46 - 7.52 (m, 2H), 6.81 (d, *J*= 9.1 Hz, 2H), 4.65 (m,1H), 4.03 (s, 3H), 2.93 (s, 6H), 1.57 (d, *J*= 6.7 Hz,6H). |
| 128 | 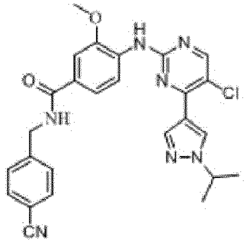 | ¹H NMR (300 MHz, MeOD) δ 8.47 - 8.52 (m, 2H), 8.33(s, 1H), 8.27 (d, *J*= 0.7 Hz, 1H), 7.66 - 7.72 (m, 2H),7.47 - 7.56 (m, 4H), 4.54 - 4.69 (m, 3H), 3.95 (s,3H), 1.54 (d, *J*= 6.7 Hz, 6H). |
| 129 | 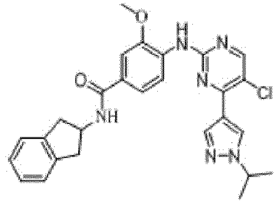 | ¹H NMR (400 MHz, MeOD) δ 8.60 - 8.50 (m, 2H), 8.43(s, 1H), 8.33 (s, 1H), 7.58 - 7.51 (m, 2H), 7.27 -7.19 (m, 2H), 7.19 - 7.13 (m, 2H), 4.65 (p, *J* = 6.7Hz, 1H), 4.01 (s, 3H), 3.39 - 3.33 (m, 2H), 3.03 (dd, *J* = 15.8, 6.9 Hz, 2H), 1.56 (d, *J* = 6.7 Hz, 6H). |
| 130 | 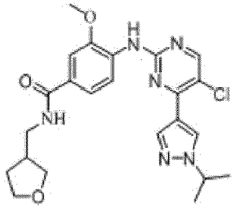 | ¹H NMR (300 MHz, MeOD) δ 8.45 - 8.50 (m, 2H), 8.32(s, 1H), 8.27 (d, *J*= 0.7 Hz, 1H), 7.44 - 7.46 (m, 2H),4.61 (m, 1H), 3.96 (s, 3H), 3.70 - 3.93 (m, 3H), 3.62(m, 1H), 3.38 - 3.42 (m, 2H), 2.60 (m, 1H), 2.08 (m, 1H), 1.72 (m, 1H), 1.55 (d, *J*= 6.7 Hz, 6H) |
| 131 | 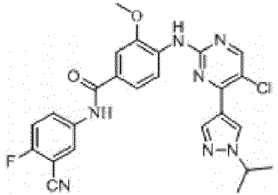 | ¹H NMR (300 MHz, MeOD) δ 8.61 (d, *J*= 8.5 Hz, 1H),8.57 (d, *J*= 0.7 Hz, 1H), 8.44 (s, 1H), 8.34 (d, *J*= 0.7Hz, 1H), 8.18 (m, 1H), 8.00 (m, 1H), 7.59 - 7.69 (m,2H), 7.36 (t, *J*= 9.0 Hz, 1H), 4.65 (m, 1H), 4.05 (s,3H), 1.57 (d, *J*= 6.7 Hz, 6H). |
| 132 | 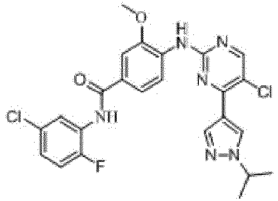 | ¹H NMR (300 MHz, MeOD) δ 8.60 (d, *J*= 8.5 Hz, 1H),8.56 (d, *J*= 0.7 Hz, 1H), 8.42 (s, 1H), 8.34 (d, *J*= 0.7Hz, 1H), 7.93 (m, 1H), 7.57 - 7.68 (m, 2H), 7.17 -7.27 (m, 2H), 4.65 (m, 1H), 4.03 (s, 3H), 1.57 (d, *J*= 6.7 Hz, 6H). |
| 133 | 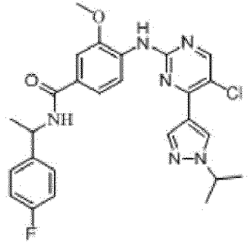 | ¹H NMR (300 MHz, MeOD) δ 8.41 - 8.49 (m, 2H), 8.31(s, 1H), 8.27 (d, *J*= 0.7 Hz, 1H), 7.51 (m, 1H), 7.40 -7.46 (m, 3H), 7.01 - 7.10 (m, 2H), 5.25 (q, *J*= 7.1 Hz,1H), 4.60 (m, 1H), 3.93 (s, 3H), 1.57 (d, *J*= 7.1 Hz,3H), 1.54 (d, *J*= 6.7 Hz, 6H). |
| 134 | 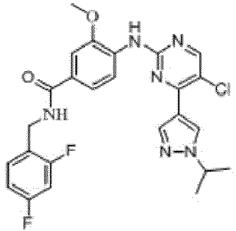 | ¹H NMR (300 MHz, MeOD) δ 8.46 - 8.48 (m, 2H), 8.30 (s, 1H), 8.26 (d, *J*= 0.7 Hz, 1H), 7.37 - 7.53(m, 3H),6.88 - 7.02 (m, 2H), 4.56 - 4.65 (m, 3H), 3.93 (s,3H), 1.54 (d, *J*= 6.7 Hz, 6H). |
| 135 | 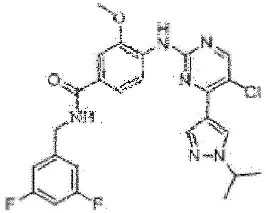 | ¹H NMR (300 MHz, MeOD) δ 8.55 - 8.57 (m, 2H), 8.41(s, 1H), 8.33 (d, *J*= 0.7 Hz, 1H), 7.51 - 7.60 (m, 2H),6.93 - 7.01 (m, 2H), 6.83 (m, 1H), 4.56 - 4.70 (m,3H), 4.00 (s, 3H), 1.56 (d, *J*= 6.7 Hz, 6H). |
| 136 | 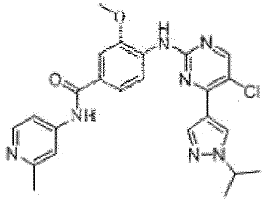 | ¹H NMR (300 MHz, Chloroform-*d*) δ 8.81 (d, *J*= 8.7 Hz, 1H), 8.75 (dd, *J*= 4.5, 1.4 Hz, 1H), 8.47 (dd, *J*= 8.4,1.4 Hz, 1H), 8.44 (s, 1H), 8.42 (d, *J*= 0.7 Hz, 1H),8.39 (d, *J*= 0.7 Hz, 1H), 8.24 (s, 1H), 8.05 (dd, *J*=8.6, 1.9 Hz, 1H), 7.73 (d, *J*= 1.9 Hz, 1H), 7.47 (dd, *J*= 8.4, 4.5 Hz, 1H), 4.61 (m, 1H), 4.05 (s, 3H), 2.80 (s, 3H), 1.60 (d, *J*= 6.7 Hz, 6H). |
| 137 | 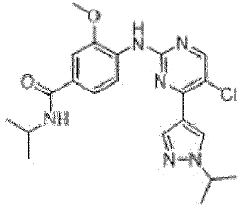 | ¹H NMR (300 MHz, Methanol-*d*4) δ 8.34 - 8.40 (m, 2H),8.18 (s, 1H), 8.18 (s, 1H), 7.37 - 7.44 (m, 2H), 4.56(m, 1H), 4.22 (m, 1H), 3.90 (s, 3H), 1.52 (d, *J*= 6.7 Hz, 6H), 1.27 (d, *J*= 6.6 Hz, 6H). |
| 138 | 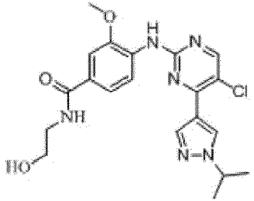 | ¹H NMR (500 MHz, Methanol-*d*4) δ 8.43 (s, 1H), 8.41 (d, *J*= 8.3 Hz, 1H), 8.25 (s, 1H), 7.41 - 7.45 (m, 2H),4.59 (m, 1H), 3.92 (s, 3H), 3.74 (t, *J*= 5.9 Hz, 2H),3.52 (t, *J*= 5.9 Hz, 2H), 1.54 (d, *J*= 6.7 Hz, 6H). |
| 139 | 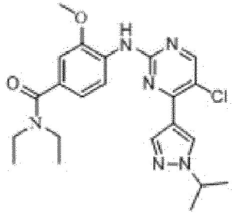 | ¹H NMR (300 MHz, Methanol-*d*4) δ 8.52 (d, *J*= 0.7 Hz,1H), 8.48 (d, *J*= 8.7 Hz, 1H), 8.37 (s, 1H), 8.30 (d, *J*= 0.7 Hz, 1H), 7.00 - 7.06 (m, 2H), 4.63 (m, 1H),3.96 (s, 3H), 3.34 - 3.62 (m, 4H), 1.55 (d, *J*= 6.7 Hz,6H), 1.16 - 1.31 (m, 6H). |
| 140 | 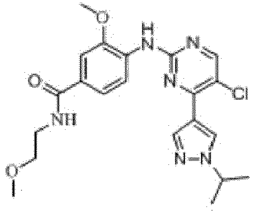 | ¹H NMR (300 MHz, Methanol-*d*4) δ 8.34 - 8.40 (m, 2H),8.18 (s, 2H), 7.36 - 7.41 (m, 2H), 4.57 (m, 1H), 3.89(s, 3H), 3.55 - 3.59 (m, 4H), 3.40 (s, 3H), 1.53 (d, *J*= 6.7 Hz, 6H). |
| 141 | 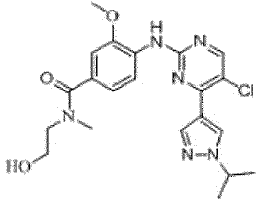 | ¹H NMR (300 MHz, Methanol-*d*4) δ 8.47 (d, *J*= 0.7 Hz,1H), 8.44 (d, *J*= 8.2 Hz, 1H), 8.30 (s, 1H), 8.26 (d,*J*= 0.7 Hz, 1H), 7.13 (s, 1H), 7.09 (dd, *J*= 8.2, 1.8Hz, 1H), 4.61 (m, 1H), 3.94 (s, 3H), 3.47 - 3.88 (m,4H), 3.13 (s, 3H), 1.53 (d, *J*= 6.7 Hz, 6H). |
| 142 | 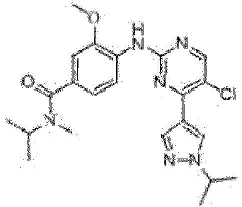 | ¹H NMR (300 MHz, Methanol-*d*4) δ 8.37 - 8.50 (m, 2H),8.28 (s, 1H), 8.25 (s, 1H), 6.93 - 7.09 (m, 2H), 4.60(m, 1H), 3.93 (s, 3H), 2.92 (s, 3H), 1.53 (d, *J*= 6.7 Hz, 6H), 1.22 (d, *J*= 6.7 Hz, 6H). |
| 143 | 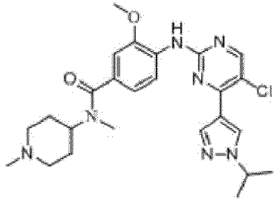 | ¹H NMR (300 MHz, Methanol-*d*4) δ 8.46 - 8.52 (m, 2H),8.34 (s, 1H), 8.29 (s, 1H), 7.02 - 7.09 (m, 2H), 4.63(m, 1H), 3.96 (s, 3H), 3.73 (m, 1H), 2.94 - 3.13 (m,2H), 2.96 (s, 1H), 2.35 (s, 3H), 1.73 - 2.09 (m, 6H),1.56 (d, *J*= 6.7 Hz, 6H). |
| 144 | 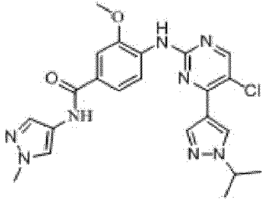 | ¹H NMR (300 MHz, Methanol-*d*4) δ 8.30 (d, *J*= 0.7 Hz,1H), 8.27 (d, *J*= 8.5 Hz, 1H), 8.11 (d, *J*= 0.7 Hz, 1H),8.08 (s, 1H), 7.91 (s, 1H), 7.59 (s, 1H), 7.30 - 7.40(m, 2H), 4.52 (m, 1H), 3.83 (s, 6H), 1.50 (d, *J*= 6.7 Hz, 6H). |
| 145 | 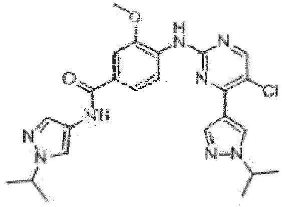 | ¹H NMR (300 MHz, Methanol-*d*4) δ 8.41 - 8.46 (m, 2H),8.25 (s, 1H), 8.22 (d, *J*= 0.7 Hz, 1H), 8.04 (d, *J*= 0.8Hz, 1H), 7.67 (d, *J*= 0.8 Hz, 1H), 7.51 (dd, *J*= 8.5,2.0 Hz, 1H), 7.46 (d, *J*= 2.0 Hz, 1H), 4.58 (m, 1H),4.50 (m, 1H), 3.93 (s, 3H), 1.53 (d, *J*= 10.1 Hz, 6H),1.50 (d, *J*= 6.7 Hz, 6H). |
| 146 | 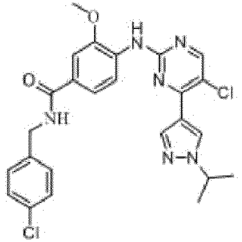 | ¹H NMR (300 MHz, Chloroform-*d*) δ 8.54 (d, *J*= 8.0 Hz, 1H), 8.36 (s, 1H), 8.34 (s, 2H), 7.96 (s, 1H), 7.49(s, 1H), 7.23 - 7.38 (m, 5H), 6.62 (s, 1H), 4.61 (d,*J*= 6.0 Hz, 2H), 4.56 (m, 1H), 3.96 (s, 3H), 1.57 (d,*J*= 6.6 Hz, 6H). |
| 147 | 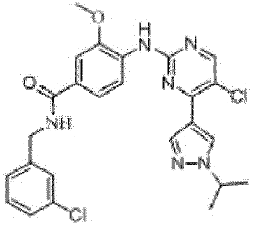 | ¹H NMR (300 MHz, Chloroform-*d*) δ 8.54 (d, *J*= 8.5 Hz, 1H), 8.35 (s, 1H), 8.33 (s, 2H), 7.94 (s, 1H), 7.49 (d, *J*= 1.9 Hz, 1H), 7.30 - 7.37 (m, 2H), 7.17 - 7.27(m, 3H), 6.71 (t, *J*= 6.0 Hz, 1H), 4.62 (d, *J*= 6.0 Hz,2H), 4.56 (m, 1H), 3.95 (s, 3H), 1.57 (d, *J*= 6.7 Hz, 6H). |
| 148 | 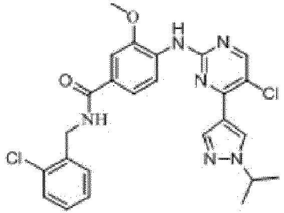 | ¹H NMR (300 MHz, Chloroform-*d*) δ 8.55 (d, *J*= 8.5 Hz, 1H), 8.36 (s, 1H), 8.34 (s, 2H), 7.94 (s, 1H), 7.43 -7.51 (m, 2H), 7.30 - 7.40 (m, 2H), 7.19 - 7.28 (m,2H), 6.75 (t, *J*= 6.0 Hz, 1H), 4.72 (d, *J*= 6.0 Hz, 2H),4.57 (m, 1H), 3.95 (s, 3H), 1.57 (d, *J*= 6.7 Hz, 6H). |
| 149 | 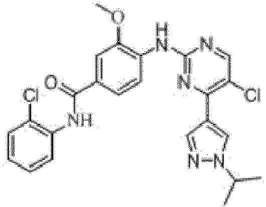 | ¹H NMR (300 MHz, Chloroform-*d*) δ 8.67 (d, *J*= 8.5 Hz, 1H), 8.58 (dd, *J*= 8.3, 1.5 Hz, 1H), 8.49 (s, 1H), 8.43(s, 1H), 8.42 (s, 1H), 8.40 (s, 1H), 8.19 (s, 1H),7.60 (d, *J*= 2.0 Hz, 1H), 7.51 (dd, *J*= 8.5, 2.0 Hz, 1H), 7.43 (dd, *J*= 8.0, 1.5 Hz, 1H), 7.35 (m, 1H), 7.07(m, 1H), 4.60 (m, 1H), 4.04 (s, 3H), 1.60 (d, *J*= 6.7 Hz, 6H). |
| 150 | 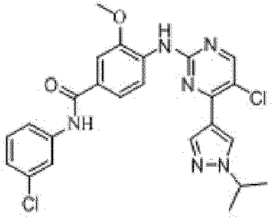 | ¹H NMR (300 MHz, Chloroform-*d*) δ 8.57 (d, *J*= 8.4 Hz, 1H), 8.37 (d, *J*= 0.7 Hz, 1H), 8.37 (d, *J*= 0.7 Hz, 1H),8.34 (s, 1H), 8.07 (s, 1H), 8.04 (s, 1H), 7.81 (t, *J*=2.0 Hz, 1H), 7.53 (ddd, *J*= 8.0, 2.0, 1.0 Hz, 1H), 7.50(d, *J*= 1.9 Hz, 1H), 7.41 (dd, *J*= 8.4, 1.9 Hz, 1H),7.28 (m, 1H), 7.11 (ddd, *J*= 8.0, 2.0, 1.0 Hz, 1H),4.59 (m, 1H), 3.97 (s, 3H), 1.59 (d, *J*= 6.7 Hz, 6H). |
| 151 | 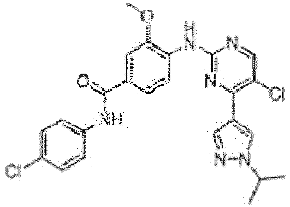 | ¹H NMR (300 MHz, Chloroform-*d*) δ 8.59 (d, *J*= 8.4 Hz, 1H), 8.38 (s, 1H), 8.37 (s, 1H), 8.36 (s, 1H), 8.04(s, 1H), 8.01 (s, 1H), 7.60 - 7.66 (m, 2H), 7.51 (d,*J*= 2.0 Hz, 1H), 7.42 (dd, *J*= 8.4, 2.0 Hz, 1H), 7.30 -7.37 (m, 2H), 4.59 (m, 1H), 3.98 (s, 3H), 1.59 (d, *J*=6.7 Hz, 6H). |
| 152 | 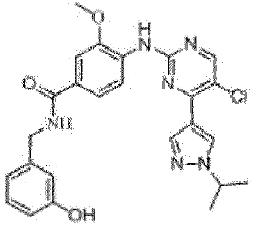 | ¹H NMR (300 MHz, DMSO-*d*6) δ 9.31 (s, 1H), 8.91 (t, *J*=6.0 Hz, 1H), 8.63 (s, 1H), 8.55 (s, 1H), 8.33 (d, *J*= 8.3 Hz, 1H), 8.25 - 8.31 (m, 2H), 7.56 - 7.65 (m, 2H),7.11 (t, *J***=** 8.0 Hz, 1H), 6.72 - 6.77 (m, 2H), 6.62(ddd, *J***=** 8.1, 2.4, 1.1 Hz, 1H), 4.67 (m, 1H), 4.42 (d,*J*= 5.9 Hz, 2H), 3.94 (s, 3H), 1.48 (d, *J*= 6.6 Hz, 6H). |
| 153 | 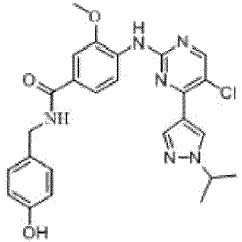 | ¹H NMR (300 MHz, Chloroform-*d*) δ 8.48 (d, *J*= 8.4 Hz, 1H), 8.34 (s, 1H), 8.31 (s, 1H), 8.29 (s, 1H), 7.88(s, 1H), 7.44 (d, *J*= 1.8 Hz, 1H), 7.31 (dd, *J*= 8.4,1.8 Hz, 1H), 7.13 - 7.20 (m, 2H), 6.78 - 6.87 (m, 2H),6.74 (t, *J*= 5.5 Hz, 1H), 4.55 (m, 1H),4.52 (d, *J*= 6.0Hz, 2H),3.87 (s, 3H),1.55 (d, *J*= 6.7 Hz, 6H). |
| 154 | 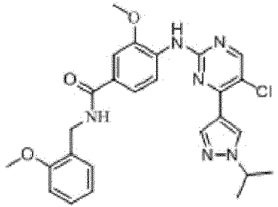 | ¹H NMR (400 MHz, MeOD) δ 8.59 - 8.53 (m, 2H), 8.45(s, 1H), 8.35 (s, 1H), 7.59 - 7.53 (m, 2H), 7.31 -7.21 (m, 2H), 6.98 (d, *J* = 8.1 Hz, 1H), 6.92 (td, *J* =7.5, 1.1 Hz, 1H), 4.65 (p, *J* = 6.7 Hz, 1H), 4.59 (s,2H), 4.02 (s, 3H), 3.89 (s, 3H), 1.56 (d, *J* = 6.7 Hz,6H). |
| 155 | 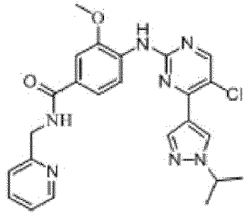 | ¹H NMR (300 MHz, Methanol-d4) δ 8.50 (ddd, *J*= 5.0, 1.8, 0.9 Hz, 1H), 8.46 (d, *J*= 9 Hz, 1H), 8.44 (s, 1H),8.27 (s, 1H), 8.24 (s, 1H), 7.81 (td, *J*= 7.7, 1.8 Hz,1H), 7.52 (dd, *J*= 8.5, 2.0 Hz, 1H), 7.48 (d, *J*= 1.9Hz, 1H), 7.44 (dt, *J*= 7.9, 1.0 Hz, 1H), 7.31 (ddd, *J*=7.6, 5.0, 1.2 Hz, 1H), 4.69 (s, 2H), 4.59 (m, 1H),3.92 (s, 3H), 1.53 (d, *J*= 6.7 Hz, 6H). |
| 156 | 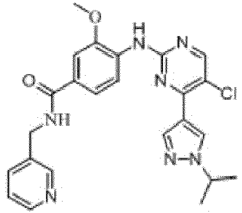 | ¹H NMR (300 MHz, Methanol-*d*4) δ 8.54 - 8.58 (m, 1H),8.41 (dd, *J*= 4.9, 1.6 Hz, 1H), 8.26 - 8.31 (m, 2H),8.10 (s, 1H), 8.05 (s, 1H), 7.30 - 7.42 (m, 3H), 4.56(s, 2H), 4.50 (m, 1H), 3.79 (s, 3H), 1.48 (d, *J*= 6.7 Hz, 6H). |
| 157 | 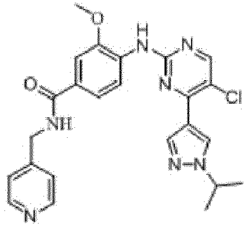 | ¹H NMR (300 MHz, Methanol-d4) δ 8.43 - 8.49 (m, 2H),8.35 (d, *J*= 8.6 Hz, 1H), 8.32 (s, 1H) 8.12 (s, 1H),8.10 (s, 1H), 7.35 - 7.45 (m, 4H), 4.58 (s, 2H), 4.52(m, 1H), 3.83 (s, 3H), 1.49 (d, *J*= 6.7 Hz, 6H). |
| 158 | 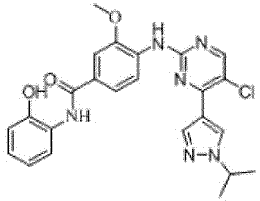 | ¹H NMR (400 MHz, DMSO-*d*6) δ 9.96 (s, 0.5H), 8.72 - 8.14 (m, 5H), 7.87 (dd, *J* = 8.6, 1.9 Hz, 0.5H), 7.77 -7.26 (m, 4H), 7.06 - 6.72 (m, 1H), 4.83 - 4.52 (m,1H), 4.05 - 3.70 (m, 3H), 1.54 - 1.38 (m, 6H). |
| 159 | 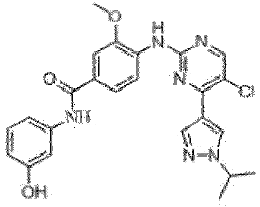 | ¹H NMR (400 MHz, MeOD) δ 8.65 (d, *J* = 8.6 Hz, 1H),8.57 (s, 1H), 8.46 (s, 1H), 8.36 (s, 1H), 7.84 (dd, *J*= 8.6, 1.9 Hz, 1H), 7.69 (d, *J* = 1.8 Hz, 1H), 7.14 (t,*J* = 8.0 Hz, 1H), 6.68 - 6.45 (m, 3H), 4.66 (p, *J* = 6.7Hz, 1H), 4.03 (s, 3H), 1.57 (d, *J* = 6.7 Hz, 6H). |
| 160 | 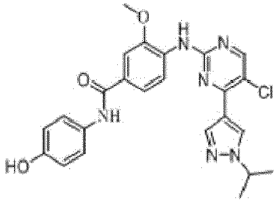 | ¹H NMR (300 MHz, MeOD) δ 8.65 - 8.58 (m, 2H), 8.46(s, 1H), 8.37 (d, *J* = 0.7 Hz, 1H), 7.70 - 7.60 (m,2H), 7.48 (d, *J* = 8.8 Hz, 2H), 6.82 (d, *J* = 8.9 Hz,2H), 4.67 (p, *J* = 6.7 Hz, 1H), 4.06 (s, 3H), 1.59 (d*,J* = 6.7 Hz, 6H). |
| 161 | 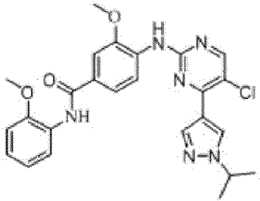 | ¹H NMR (300 MHz, MeOD) δ 8.63 (d, *J* = 9.0 Hz, 1H),8.58 (d, *J* = 0.7 Hz, 1H), 8.46 (s, 1H), 8.37 (d, *J* =0.7 Hz, 1H), 8.00 (dd, *J* = 7.9, 1.6 Hz, 1H), 7.65 -7.55 (m, 2H), 7.18 (ddd, *J* = 8.3, 7.4, 1.6 Hz, 1H),7.08 (dd, *J* = 8.3, 1.5 Hz, 1H), 6.99 (td, *J* = 7.6, 1.5Hz, 1H), 4.67 (p, *J* = 6.7 Hz, 1H), 4.05 (s, 3H), 3.94 (s, 3H), 1.57 (d, *J* = 6.7 Hz, 6H). |
| 162 | 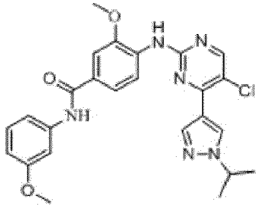 | ¹H NMR (300 MHz, MeOD) δ 8.66 - 8.55 (m, 2H), 8.45(s, 1H), 8.36 (s, 1H), 7.66 (dd, *J* = 8.5, 2.0 Hz, 1H),7.61 (d, *J* = 2.0 Hz, 1H), 7.44 - 7.38 (m, 1H), 7.29 -7.22 (m, 2H), 6.77 - 6.66 (m, 1H), 4.66 (p, *J* = 6.7Hz, 1H), 4.05 (s, 3H), 3.82 (s, 3H), 1.57 (d, *J* = 6.7 Hz, 6H). |
| 163 | 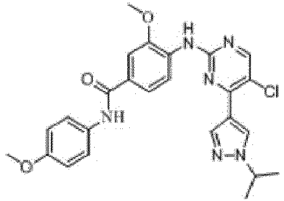 | ¹H NMR (400 MHz, CDCl3) δ 8.59 (d, *J* = 8.4 Hz, 1H),8.44 - 8.40 (m, 2H), 8.38 (s, 1H), 8.18 (s, 1H), 7.78(s, 1H), 7.60 - 7.52 (m, 3H), 7.43 (dd, *J* = 8.5, 1.9Hz, 1H), 6.92 (d, *J* = 9.0 Hz, 2H), 4.60 (p, *J* = 6.7Hz, 1H), 4.01 (s, 3H), 3.82 (s, 3H), 1.60 (d, *J* = 6.7 Hz, 6H). |
| 164 | 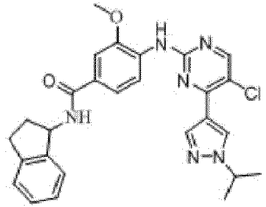 | ¹H NMR (400 MHz, MeOD) δ 8.60 -8.55 (m, 2H), 8.47 (s,1H), 8.37 (s, 1H), 7.63 -7.55 (m, 2H), 7.38 - 7.19 (m,4H), 5.70 (t, *J* = 7.9 Hz, 1H), 4.67 (hept, *J* = 6.5 Hz,1H), 4.04 (s, 3H), 3.19 - 3.04 (m, 1H), 3.04 - 2.86(m, 1H), 2.70 - 2.55 (m, 1H), 2.14 - 1.94 (m, 1H),1.58 (d, *J* = 6.7 Hz, 6H). |
| 165 | 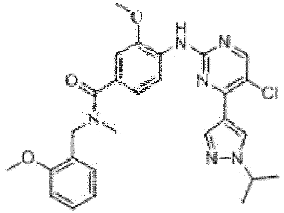 | ¹H NMR (400 MHz, CDCl3) δ 8.25 - 8.17 (m, 3H), 7.54 (d, *J* = 1.6 Hz, 1H), 7.37 (dd, *J* = 7.3, 1.8 Hz, 1H),7.32 - 7.26 (m, 3H), 6.99 - 6.89 (m, 2H), 6.67 (t, *J* =5.9 Hz, 1H), 4.65 (d, *J* = 5.8 Hz, 2H), 4.52 (h, *J* =6.6 Hz, 1H), 3.90 (s, 3H), 3.82 (s, 3H), 3.44 (s, 3H),1.54 (d, *J* = 6.7 Hz, 6H). |
| 166 | 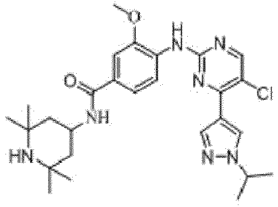 | ¹H NMR (300 MHz, CDCl3) δ 8.56 (d, *J*= 8.4 Hz, 1H),8.32 - 8.37 (m, 3H), 7.88 (s, 1H), 7.47 (d, *J*= 1.9 Hz,1H), 7.29 (dd, *J*= 8.4, 2.0 Hz, 1H), 6.02 (d, *J*= 7.8Hz, 1H), 4.40 - 4.65 (m, 2H), 3.97 (s, 3H), 2.03 (dd,*J*= 12.6, 3.7 Hz, 2H), 1.58 (d, *J*= 6.7 Hz, 6H), 1.35 (s, 6H), 1.24 (s, 9H). |
| 167 | 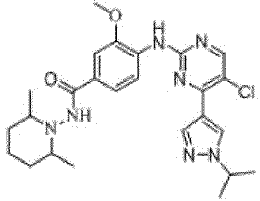 | ¹H NMR (300 MHz, MeOD) δ 8.60 (t, *J*= 4.1 Hz, 2H),8.48 (s, 1H), 8.38 (s, 1H), 7.54 (d, *J*= 9.5 Hz, 2H),4.68 (m, 1H), 4.05 (s, 3H), 2.65 - 2.69 (s, 3H), 1.73- 1.77 (m, 3H), 1.60 (s, 3H), 1.58 (s, 3H), 1.13 (d,*J*= 6.2 Hz, 6H). |
| 168 | 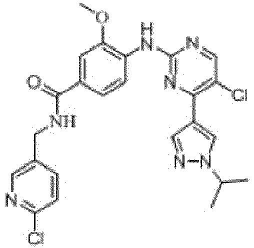 | ¹H NMR (300 MHz, MeOD) δ 8.48 - 8.50 (m, 2H), 8.38(dd, *J*= 2.5, 0.7 Hz, 1H), 8.34 (s, 1H), 8.28 (d, *J*=0.7 Hz, 1H), 7.83 (dd, *J*= 8.4, 2.5 Hz, 1H), 7.46 -7.52 (m, 2H), 7.43 (dd, *J*= 8.4, 0.7 Hz, 1H), 4.55 -4.67 (m, 3H), 3.95 (s, 3H), 1.55 (d, *J*= 6.7 Hz, 6H). |
| 169 | 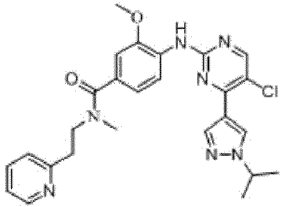 | ¹H NMR (300 MHz, MeOD) δ 8.22 - 8.55 (m, 5H), 7.70 (m, 1H), 7.34 - 7.42 (m, 2H), 6.80 - 6.90 (m, 2H),4.61 (m, 1H), 3.86 - 3.98 (m, 5H), 3.03 - 3.22 (m,5H), 1.53 (d, *J*= 6.7 Hz, 6H). |
| 170 | 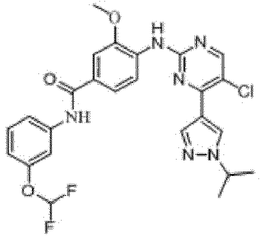 | ¹H NMR (300 MHz, MeOD) δ 8.31 - 8.40 (m, 2H), 8.11 -8.19 (m, 2H), 7.64 (t, *J*= 2.2 Hz, 1H), 7.25 - 7.55 (m,4H), 6.85 (m, 1H), 4.54 (m, 1H), 3.88 (s, 3H), 1.51 (d, *J*= 6.7 Hz, 6H). |
| 171 | 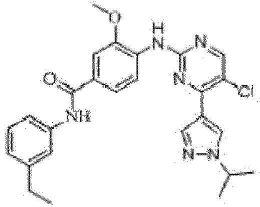 | ¹H NMR (300 MHz, MeOD) δ 8.59 (d, *J*= 8.5 Hz, 1H),8.56 (d, *J*= 0.7 Hz, 1H), 8.43 (s, 1H), 8.34 (d, *J*= 0.7Hz, 1H), 7.65 (dd, *J*= 8.5, 2.0 Hz, 1H), 7.60 (d, *J*=1.9 Hz, 1H), 7.49 - 7.57 (m, 2H), 7.27 (t, *J*= 7.8 Hz,1H), 7.00 (dt, *J*= 7.9, 1.2 Hz, 1H), 4.65 (m, 1H), 4.04(s, 3H), 2.67 (q, *J*= 7.6 Hz, 2H), 1.57 (d, *J*= 6.7 Hz, 6H), 1.27 (t, *J*= 7.6 Hz, 3H). |
| 172 | 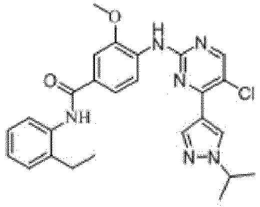 | ¹H NMR (300 MHz, MeOD) δ 8.58 (d, *J*= 8.5 Hz, 1H),8.52 (d, *J*= 0.7 Hz, 1H), 8.37 (s, 1H), 8.32 (d, *J*= 0.7Hz, 1H), 7.66 (dd, *J*= 8.5, 2.0 Hz, 1H), 7.60 (d, *J*=1.9 Hz, 1H), 7.22 - 7.37 (m, 4H), 4.60 (m, 1H), 4.00(s, 3H), 2.71 (q, *J*= 7.6 Hz, 2H), 1.56 (d, *J*= 6.7 Hz,6H), 1.18 - 1.26 (m, 3H). |
| 173 | 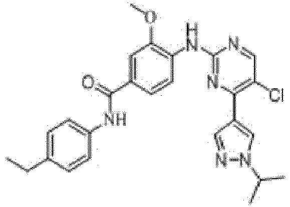 | ¹H NMR (300 MHz, MeOD) δ 8.45 (dd, *J*= 9.1, 2.0 Hz,2H), 8.20 - 8.29 (m, 2H), 7.44 - 7.60 (m, 4H), 7.16(dd, *J*= 8.5, 2.4 Hz, 2H), 4.58 (m, 1H), 3.94 (s, 3H),2.54 - 2.68 (m, 2H), 1.48 - 1.58 (m, 6H), 1.20 - 1.25 (m, 3H). |
| 174 | 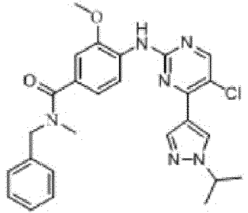 | ¹H NMR (300 MHz, MeOD) δ 8.40 - 8.50 (m, 2H), 8.36(s, 1H), 8.29 (s, 1H), 7.35 (dd, *J*= 20.3, 6.5 Hz, 5H),7.12 (dd, *J*= 8.3, 1.7 Hz, 2H), 4.55 - 4.80 (m, 3H),3.87 (d, *J*= 55.9 Hz, 3H), 3.02 (s, 3H), 1.54 (d, *J*=6.7 Hz, 6H). |
| 175 | 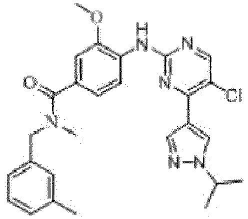 | ¹H NMR (300 MHz, Methanol-*d*4) δ 8.42 - 8.54 (m, 2H),8.36 (s, 1H), 8.29 (s, 1H), 7.27 (t, *J*= 7.5 Hz, 1H) ,6.98 - 7.22 (m, 5H), 4.68 (s, 2H), 4.62 (m, 1H), 3.72(d, *J*= 55.0 Hz, 3H), 3.01 (s, 3H), 2.35 (s, 3H), 1.54 (d, *J*= 6.7 Hz, 6H). |
| 176 | 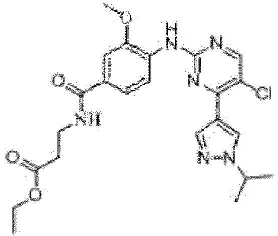 | ¹H NMR (300 MHz, Methanol-*d*4) δ 8.30 8.37 (m, 2H),8.16 (s, 1H), 8.15 (s, 1H), 7.31 7.37 (m, 2H), 4.56(m, 1H), 4.16 (q, *J*= 7.1 Hz, 2H), 3.87 (s, 3H), 3.63(t, *J*= 6.9 Hz, 2H), 2.80 (s, 3H), 2.66 (t, *J*= 6.9 Hz,2H), 1.52 (d, *J*= 6.7 Hz, 6H), 1.26 (t, *J*= 7.1 Hz, 3H). |
| 177 | 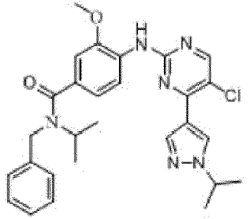 | ¹H NMR (500 MHz, DMSO-d6) δ 8.60 (s, 1H), 8.52 (s,1H), 8.20 - 8.32 (m, 3H), 7.29 - 7.39 (m, 4H), 7.23(m, 1H), 7.04 - 7.13 (m, 2H), 4.66 (m, 1H), 4.59 (s,2H), 4.19 (s, 1H), 3.88 (s, 3H), 1.47 (d, *J*= 6.6 Hz,6H), 1.12 (d, *J*= 6.3 Hz, 6H). |
| 178 | 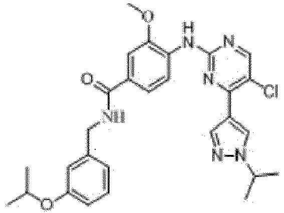 | ¹H NMR (300 MHz, Methanol-*d*4) δ 8.39 - 8.43 (m, 2H),8.21 (s, 1H), 8.20 (s, 1H), 7.50 (dd, *J*= 8.5, 2.0 Hz,1H), 7.44 (d, *J*= 2.0 Hz, 1H), 7.38 (m, 1H), 7.18 -7.22 (m, 2H), 6.66 (m, 1H), 4.51 - 4.63 (m, 2H), 3.90(s, 3H), 1.52 (d, *J*= 6.7 Hz, 6H), 1.32 (d, *J*= 6.1 Hz, 6H). |
| 179 | 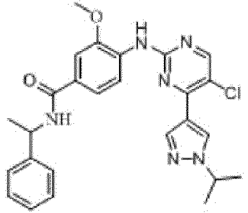 | ¹H NMR (300 MHz, Methanol-*d*4) δ 8.47 - 8.51 (m, 2H),8.34 (s, 1H), 8.29 (s, 1H), 7.53 (dd, *J*= 8.5, 2.0 Hz,1H), 7.47 (d, *J*= 2.0 Hz, 1H), 7.40 - 7.45 (m, 2H),7.29 - 7.37 (m, 2H), 7.23 (m, 1H), 4.61 (m, 1H), 3.94(s, 3H), 1.59 (d, *J*= 7.1 Hz, 3H), 1.55 (d, *J*= 6.7 Hz, 6H). |
| 180 | 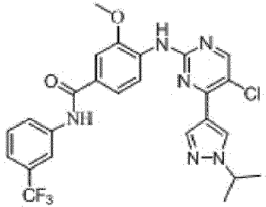 | ¹H NMR (500 MHz, Methanol-d4) δ 8.39 - 8.43 (m, 2H),8.22 (s, 1H), 8.20 (s, 1H), 8.13 (d, *J*= 2.0 Hz, 1H),7.90 (dt, *J*= 8.1, 1.3 Hz, 1H), 7.46 - 7.53 (m, 2H) , 7.45 (d, *J*= 2.0 Hz, 1H), 7.37 (d, *J*= 7.7 Hz, 1H), 4.57(m, 1H), 3.92 (s, 3H), 1.53 (d, *J*= 6.7 Hz, 6H). |
| 181 | 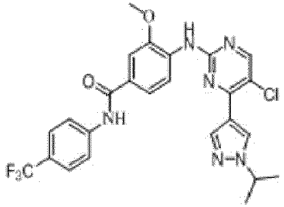 | ¹H NMR (500 MHz, Methanol-*d*4) δ 8.56 (d, *J*= 8.5 Hz,1H), 8.52 (s, 1H), 8.38 (s, 1H), 8.31 (s, 1H), 7.92(s, 1H), 7.90 (s, 1H), 7.60 - 7.65 (m, 3H), 7.57 (d,*J*= 1.9 Hz, 1H), 4.613 (m, 1H), 4.01 (s, 3H), 1.56 (d, *J*= 6.7 Hz, 6H). |
| 182 | 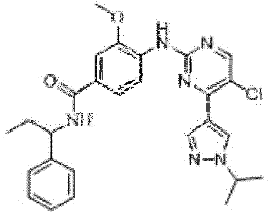 | ¹H NMR (300 MHz, Methanol-*d*4) δ 8.45 - 8.51 (m, 2H),8.32 (s, 1H), 8.28 (s, 1H), 7.52 (dd, *J*= 8.5, 1.9 Hz,1H), 7.38 - 7.46 (m, 3H), 7.29 - 7.37 (m, 2H), 7.23(m, 1H), 5.00 (m, 1H), 4.60 (m, 1H), 3.92 (s, 3H),1.86 - 2.02 (m, 3H), 1.54 (d, *J*= 6.7 Hz, 6H), 1.00 (t,*J*= 7.3 Hz, 3H). |
| 183 | 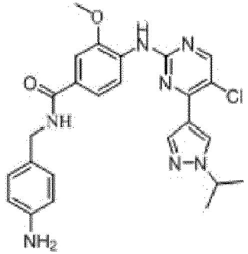 | ¹H NMR (300 MHz, Methanol-*d*4) δ 8.33 - 8.41 (m, 2H),8.16 - 8.21 (m, 2H), 7.34 - 7.44 (m, 2H), 7.08 - 7.17(m, 2H), 6.65 - 6.75 (m, 2H), 4.55 (m, 1H), 4.43 (s,2H), 3.86 (s, 3H), 1.51 (d, *J*= 6.7 Hz, 6H). |
| 184 | 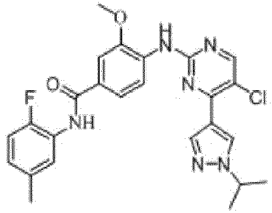 | ¹H NMR (300 MHz, MeOD) δ 8.45 (d, *J*= 8.5 Hz, 1H),8.42 (d, *J*= 0.7 Hz, 1H), 8.24 (s, 1H), 8.22 (d, *J*= 0.7Hz, 1H), 7.44 - 7.60 (m, 3H), 6.97 - 7.09 (m, 2H),4.58 (m, 1H), 3.93 (s, 3H), 2.32 (d, *J*= 0.9 Hz, 3H),1.53 (d, *J*= 6.7 Hz, 6H). |
| 185 | 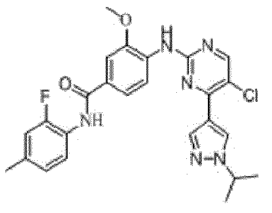 | ¹H NMR (300 MHz, MeOD) δ 8.60 (d, *J*= 8.5 Hz, 1H),8.56 (s, 1H), 8.43 (d, *J*= 0.7 Hz, 1H), 8.34 (s, 1H),7.50 - 7.68 (m, 3H), 6.99 - 7.09 (m, 2H), 4.65 (m,1H), 4.03 (s, 3H), 2.37 (s, 3H), 1.56 (dd, *J*= 6.7, 0.7 Hz, 6H). |
| 186 | 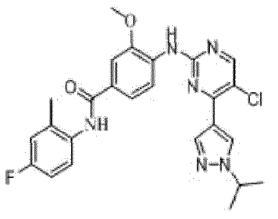 | ¹H NMR (300 MHz, MeOD) δ 8.64 (d, *J*= 8.5 Hz, 1H),8.59 (s, 1H), 8.47 (s, 1H), 8.37 (s, 1H), 7.32 (dd, *J*=8.7, 5.4 Hz, 1H), 6.92 - 7.11 (m, 2H), 4.61 - 4.72 (m,2H), 4.05 (s, 3H), 2.31 (s, 3H), 1.57 (d, *J*= 6.7 Hz, 6H). |
| 187 | 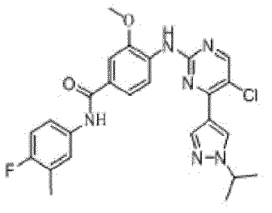 | ¹H NMR (300 MHz, MeOD) δ 8.54 (d, *J*= 8.5 Hz, 1H),8.52 (d, *J*= 0.7 Hz, 1H), 8.37 (s, 1H), 8.30 (d, *J*= 0.7Hz, 1H), 7.52 - 7.62 (m, 3H), 7.32 (dd, *J*= 8.3, 2.1Hz, 1H), 7.11 - 7.22 (m, 1H), 4.63 (m, 1H), 4.01 (s,3H), 2.23 (d, *J*= 1.8 Hz, 3H), 1.56 (d, *J*= 6.7 Hz, 6H). |
| 188 | 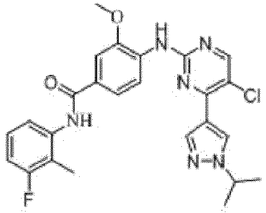 | ¹H NMR (300 MHz, MeOD) δ 8.64 (d, *J*= 8.5 Hz, 1H),8.58 (s, 1H), 8.46 (s, 1H), 8.36 (s, 1H), 7.70 (dd, *J*=8.5, 1.9 Hz, 1H), 7.64 (d, *J*= 2.0 Hz, 1H), 7.16 - 7.29(m, 2H), 7.02 (t, *J*= 9.0 Hz, 1H), 4.65 (q, *J*= 6.7 Hz,2H), 4.05 (s, 3H), 2.21 (d, *J*= 2.2 Hz, 3H), 1.57 (d,*J*= 6.7 Hz, 6H). |
| 189 | 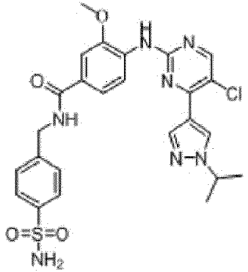 | ¹H NMR (300 MHz, MeOD) δ 8.53 - 8.62 (m, 2H), 8.47 (d, J = 7.5 Hz, 1H), 8.30 (m, 1H), 7.88 (d, J = 8.4Hz, 2H), 7.48 - 7.62 (m, 4H), 4.66 (d, J = 3.2 Hz,2H), 4.60 (d, J = 6.6 Hz, 1H), 4.02 (s, 3H), 1.57 (d, J = 6.7 Hz, 6H). |
| 190 | 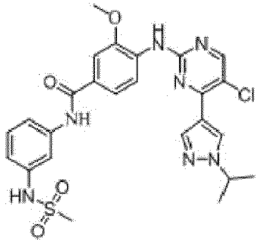 | ¹H NMR (300 MHz, MeOD) δ 8.43 - 8.48 (m, 2H), 8.27(s, 1H), 8.24 (d, *J*= 0.7 Hz, 1H), 7.76 (t, *J*= 2.1 Hz,1H), 7.54 (dd, *J*= 8.5, 2.0 Hz, 1H), 7.48 (d, *J*= 2.0Hz, 1H), 7.42 (ddd, *J*= 8.1, 2.0, 1.0 Hz, 1H), 7.30 (t,*J*= 8.1 Hz, 1H), 7.01 (ddd, *J*= 8.0, 2.2, 1.0 Hz, 1H),4.60 (m, 1H), 3.94 (s, 3H), 3.02 (s, 3H), 1.54 (d, *J*=6.7 Hz, 6H). |
| 191 | 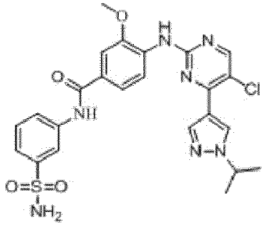 | ¹H NMR (300 MHz, MeOD) δ 8.64 (d, *J*= 8.5 Hz, 1H),8.59 (s, 1H), 8.47 (s, 1H), 8.37 (d, *J*= 1.5 Hz, 2H),7.92 (ddd, *J*= 8.0, 2.2, 1.1 Hz, 1H), 7.64 - 7.73 (m,3H), 7.55 (t, *J*= 8.0 Hz, 1H), 4.65 (m, 1H), 4.07 (s,3H), 1.57 (d, *J*= 6.7 Hz, 6H). |
| 192 | 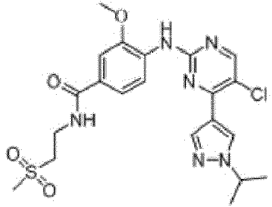 | ¹H NMR (300 MHz, CDCl3) δ 8.55 (d, *J*= 8.4 Hz, 1H),8.35 - 8.46 (m, 3H), 8.18 (s, 1H), 7.45 (d, *J*= 1.9 Hz,1H), 7.37 (dd, *J*= 8.4, 2.0 Hz, 1H), 7.00 (t, *J*= 6.0Hz, 1H), 4.55 - 4.67 (m, 1H), 3.94 - 4.07 (m, 6H),3.38 (t, *J*= 5.8 Hz, 2H), 3.01 (s, 4H), 1.59 (d, *J*= 6.7 Hz, 6H). |
| 193 | 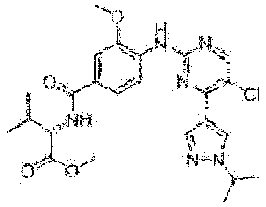 | ¹H NMR (300 MHz, hloroform-*d*) δ 8.58 (d, *J*= 8.4 Hz, 1H), 8.38 (d, *J*= 0.7 Hz, 1H), 8.35 (s, 1H), 8.34 (d,*J*= 0.7 Hz, 1H), 7.96 (s, 1H), 7.47 (d, *J*= 1.9 Hz, 1H),7.38 (dd, *J*= 8.4, 1.9 Hz, 1H), 6.65 (d, *J*= 8.6 Hz,1H), 4.77 (dd, *J*= 8.6, 5.0 Hz, 1H), 4.57 (m, 1H), 3.97(s, 3H), 3.78 (s, 3H), 2.28 (m, 1H), 1.58 (d, *J*= 6.7Hz, 6H), 1.01 (dd, *J*= 6.9, 5.8 Hz, 6H). |
| 194 | 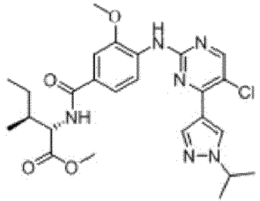 | ¹H NMR (300 MHz, Chloroform-*d*) δ 8.57 (d, *J*= 8.4 Hz, 1H), 8.37 (s, 1H), 8.34 (s, 1H), 8.33 (s, 1H), 7.94(s, 1H), 7.46 (d, *J*= 1.9 Hz, 1H), 7.36 (dd, *J*= 8.4,1.9 Hz, 1H), 6.69 (d, *J*= 8.4 Hz, 1H), 4.80 (dd, *J*=8.4, 5.0 Hz, 1H), 4.57 (m, 1H), 3.95 (s, 3H), 3.77 (s,3H), 2.01 (m, 1H), 1.57 (d, *J*= 6.7 Hz, 6H), 1.52 (m,1H), 1.23 (m, 1H), 0.96 (t, *J*= 7.2 Hz, 6H). |
| 195 | 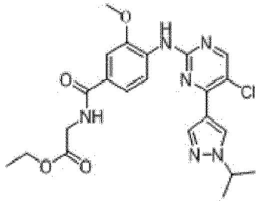 | ¹H NMR (300 MHz, Chloroform-*d*) δ 8.51 (d, *J*= 8.4 Hz, 1H), 8.36 (s, 1H), 8.34 (s, 1H), 8.33 (s, 1H), 7.97(s, 1H), 7.44 (d, *J*= 1.9 Hz, 1H), 7.39 (dd, *J*= 8.4,1.9 Hz, 1H), 6.85 (t, *J*= 5.2 Hz, 1H), 4.59 (m, 1H),4.20 - 4.31 (m, 4H), 3.94 (s, 3H), 1.58 (d, *J=* 6.7 Hz,6H), 1.31 (t, *J*= 7.1 Hz, 3H). |
| 196 | 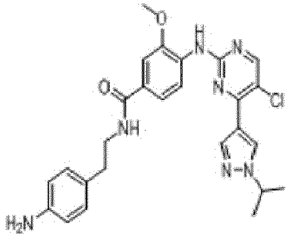 | ¹H NMR (300 MHz, Methanol-*d*4) δ 8.54 (d, *J*= 0.7 Hz,1H), 8.50 (m, 1H), 8.39 (s, 1H), 8.32 (d, *J*= 0.7 Hz,1H), 7.44 (d, *J*= 7.4 Hz, 2H), 6.99 - 7.05 (m, 2H), 6.66 - 6.73 (m, 2H), 4.64 (m, 1H), 3.98 (s, 3H), 3.49- 3.58 (m, 2H), 2.80 (t, *J*= 7.4 Hz, 2H), 1.56 (d, *J*=6.7 Hz, 6H). |
| 197 | 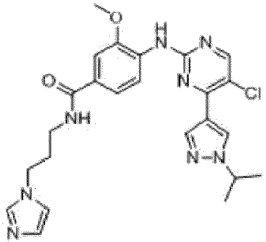 | ¹H NMR (300 MHz, Chloroform-*d*) δ 8.50 (d, *J*= 8.5 Hz, 1H), 8.32 (s, 1H), 8.30 (s, 1H), 8.29 (s, 1H), 7.85 (s, 1H), 7.59 (s, 1H), 7.4 (d, *J*= 1.8 Hz, 1H), 7.37(dd, *J*= 8.5, 1.8 Hz, 1H), 7.22 (s, 1H), 7.00 (d, *J*=12.5 Hz, 2H), 4.54 (m, 1H), 4.04 (t, *J* = 6.8 Hz, 2H),3.90 (s, 3H), 3.43 (q, *J*= 6.4 Hz, 2H), 2.04 - 2.16 (m,2H), 1.55 (d, *J*= 6.7 Hz, 6H). |
| 198 | 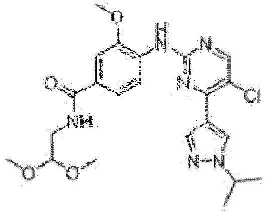 | ¹H NMR (300 MHz, Methanol-d4) δ 8.34 - 8.40 (m, 2H),8.18 (s, 1H), 8.18 (s, 1H), 7.37 - 7.44 (m, 2H), 4.56(m, 1H), 4.22 (m, 1H), 3.90 (s, 3H), 1.52 (d, *J*= 6.7 Hz, 6H), 1.27 (d, *J*= 6.6 Hz, 6H). |
| 199 | 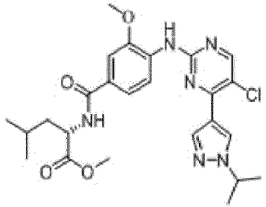 | ¹H NMR (300 MHz, Chloroform-*d*) δ 8.54 (d, *J*= 8.5 Hz, 1H), 8.36 (s, 1H), 8.33 (s, 1H), 8.33 (s, 1H), 7.92(s, 1H), 7.44 (d, *J*= 1.9 Hz, 1H), 7.36 (dd, *J*= 8.5,1.9 Hz, 1H), 6.68 (d, *J*= 8.2 Hz, 1H), 4.85 (m, 1H),4.57 (m, 1H), 3.93 (s, 3H), 3.77 (s, 3H), 1.64 - 1.83(m, 3H), 1.57 (d, *J*= 6.7 Hz, 6H), 0.98 (dd, *J*= 6.0,4.0 Hz, 6H). |
| 200 | 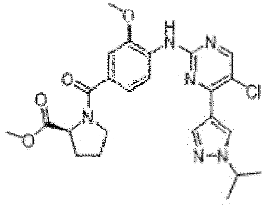 | ¹H NMR (300 MHz, Chloroform-*d*) δ 8.50 (d, *J*= 8.4 Hz, 1H), 8.37 (s, 1H), 8.35 (s, 1H), 8.34 (s, 1H), 7.91(s, 1H), 7.18 - 7.27 (m, 2H), 4.66 (m, 1H), 4.58 (m,1H), 3.92 (s, 3H), 3.76 (s, 3H), 3.72 (m, 2H), 2.31(m, 1H), 1.84 - 2.09 (m, 4H), 1.57 (d, *J*= 6.7 Hz, 6H). |
| 201 | 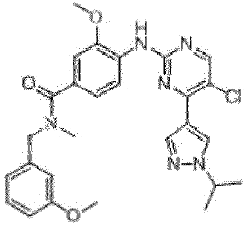 | ¹H NMR (300 MHz, MeOD) δ 8.39 (s, 1H), 8.17 (s, 1H),8.13 (s, 1H), 7.59 (d, *J* = 1.9 Hz, 1H), 7.52 (dd, *J* =8.1, 1.9 Hz, 1H), 7.34 (d, *J* = 8.1 Hz, 1H), 7.31 (d, *J*= 8.7 Hz, 2H), 6.93 - 6.87 (m, 2H), 4.66 - 4.52 (m,3H), 3.82 (s, 3H), 3.78 (s, 3H), 3.44 (s, 3H), 1.51 (d, *J* = 6.7 Hz, 6H). |
| 202 | 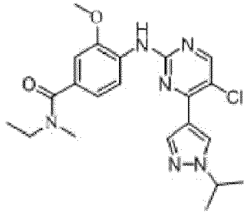 | ¹H NMR (300 MHz, MeOD) δ 8.60 - 8.46 (m, 2H), 8.42 (s, 1H), 8.33 (s,1H), 7.07 (d, *J* = 6.2 Hz, 2H), 4.65(p, *J* = 6.7 Hz, 1H), 3.98 (s, 3H), 3.70 - 3.36 (m,2H), 3.07 (s, 3H), 1.23 (m, 3H). |
| 203 | 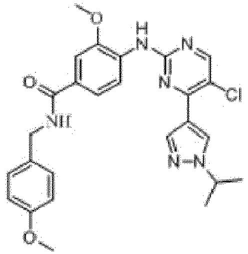 | ¹H NMR (400 MHz, MeOD) δ 8.53 (d, *J* = 7.5 Hz, 2H),8.40 (s, 1H), 8.32 (s, 1H), 7.58 - 7.46 (m, 2H), 7.30(d, *J* = 8.7 Hz, 2H), 6.89 (d, *J* = 8.7 Hz, 2H), 4.64(hept, *J* = 6.8 Hz, 1H), 4.52 (s, 2H), 3.99 (s, 3H),3.77 (s, 3H), 1.56 (d, *J* = 6.6 Hz, 6H). |
| 204 | 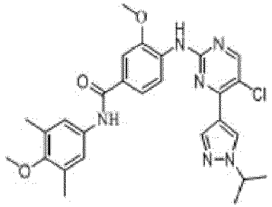 | ¹H NMR (300 MHz, Methanol-d4) δ 8.47 (s, 1H), 8.46 (d, *J*= 8.5 Hz, 1H), 8.30 (s, 1H), 8.25 (s, 1H), 7.53(dd, *J*= 8.5, 1.9 Hz, 1H), 7.49 (d, *J*= 1.9 Hz, 1H),7.30 (s, 2H), 4.56 (s, 1H), 3.96 (s, 3H), 3.70 (s,3H), 2.26 (s, 6H), 1.54 (d, *J*= 6.7 Hz, 6H). |
| 205 | 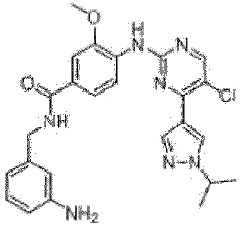 | ¹H NMR (300 MHz, Methanol-d4) δ 8.32 (dd, *J*= 4.6, 3.9 Hz, 2H), 8.14 (s, 1H), 8.11 (s, 1H), 7.39 (m, 1H),7.36 (m, 1H), 7.06 (t, *J*= 2.2 Hz, 1H), 6.74 (t, *J*= 2.0Hz, 1H), 6.69 (m, 1H), 6.62 (m, 1H), 4.51 (m, 1H),4.46 (s, 2H), 3.81 (s, 3H), 1.49 (d, *J*= 6.7 Hz, 6H). |
| 206 | 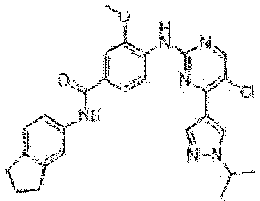 | ¹H NMR (300 MHz, MeOD) δ 8.63 - 8.55 (m, 2H), 8.45(s, 1H), 8.36 (s, 1H), 7.71 - 7.53 (m, 3H), 7.38 (dd,*J* = 8.0, 2.0 Hz, 1H), 7.19 (d, *J* = 8.1 Hz, 1H), 4.66(p, *J* = 6.7 Hz, 1H), 4.04 (s, 3H), 2.91 (q, *J* = 7.8Hz, 4H), 2.10 (p, *J* = 7.4 Hz, 2H), 1.57 (d, *J* = 6.7 Hz, 6H). |
| 207 | 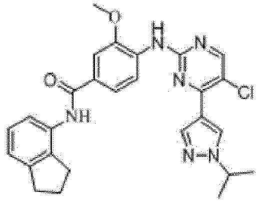 | ¹H NMR (300 MHz, MeOD) δ 8.67 - 8.56 (m, 2H), 8.47(s, 1H), 8.37 (s, 1H), 7.71 - 7.61 (m, 2H), 7.26 -7.13 (m, 3H), 4.67 (p, *J* = 6.7 Hz, 1H), 4.05 (s, 3H),3.04 - 2.85 (m, 4H), 2.16 - 2.04 (m, 2H), 1.57 (d, *J* =6.7 Hz, 6H). |
| 208 | 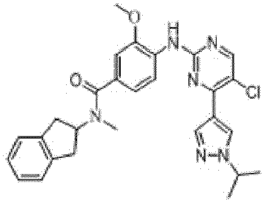 | ¹H NMR (300 MHz, MeOD) δ 8.40 (s, 1H), 8.17 (s, 1H),8.12 (s, 1H), 7.61 - 7.49 (m, 2H), 7.33 (d, *J* = 8.1Hz, 1H), 7.29 - 7.13 (m, 4H), 4.93 - 4.77 (m, 1H),4.59 (p, *J* = 6.6 Hz, 1H), 3.82 (s, 3H), 3.44 (s, 3H),3.37 (dd, *J* = 15.9, 7.8 Hz, 2H), 3.04 (dd, *J* = 15. 8, 6. 8 Hz, 2H), 1.51 (d, *J =* 6.7 Hz, 6H). |
| 209 | 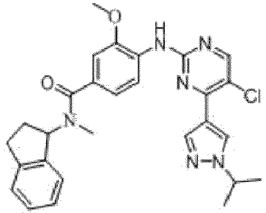 | ¹H NMR (400 MHz, MeOD) δ 8.39 (s, 1H), 8.18 (s, 1H),8.13 (s, 1H), 7.62 (d, *J* = 1.9 Hz, 1H), 7.55 (dd, *J* =8.1, 1.9 Hz, 1H), 7.37 - 7.17 (m, 5H), 5.69 (t, *J* =7.8 Hz, 1H), 4.59 (p, *J* = 6.7 Hz, 1H), 3.83 (s, 3H),3.45 (s, 3H), 3.17 - 3.04 (m, 1H), 2.98 - 2.88 (m,1H), 2.66 - 2.55 (m, 1H), 2.11 - 2.00 (m, 1H), 1.52 (d, *J* = 6.7 Hz, 6H). |
| 210 | 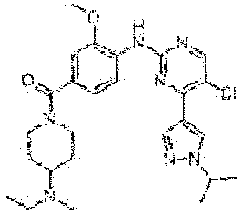 | ¹H NMR (300 MHz, MeOD) δ 8.57 - 8.46 (m, 2H), 8.42(s, 1H), 8.34 (s, 1H), 7.11 - 7.06 (m, 2H), 4.73 -4.48 (m, 2H), 3.98 (s, 3H), 3.27 - 2.67 (m, 4H), 2.63(q, *J* = 7.1 Hz, 2H), 2.30 (s, 3H), 1.89 (br s, 2H),1.61 - 1.41 (m, 8H), 1.11 (t, *J* = 7.2 Hz, 2H). |
| 211 | 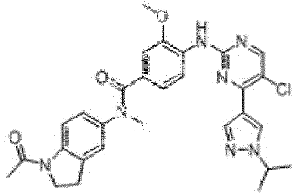 | ¹H NMR (400 MHz, DMSO-*d*6) δ 10.18 (s, 1H), 8.52 (s,1H), 8.31 (s, 1H), 8.08 (br s, 01), 8.01 (d, J = 8.7Hz, 1H), 7.72 (s, 1H), 7.65 - 7.57 (m, 2H), 7.48 (dd,*J* = 8.7, 2.2 Hz, 1H), 7.42 (d, *J* = 8.0 Hz, 1H), 4.63(hept, *J* = 6.6 Hz, 1H), 4.11 (t, *J* = 8.5 Hz, 2H), 3.81(s, 3H), 3.42 (s, 2H), 3.17 (t, *J* = 8.5 Hz, 2H), 2.15 (s, 3H), 1.45 (d, *J =* 6.6 Hz, 6H). |
| 212 | 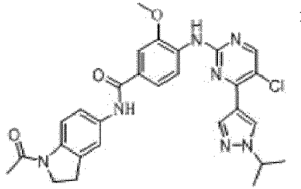 | ¹H NMR (300 MHz, DMSO) δ 10.05 (s, 1H), 8.64 (s, 1H),8.57 (s, 1H), 8.45 - 8.23 (m, 3H), 8.04 - 7.95 (m,1H), 7.74 - 7.64 (m, 2H), 7.62 (d, *J* = 1.9 Hz, 1H) , 7.47 (d, *J* = 8.5 Hz, 1H), 4.68 (p, *J* = 6.6 Hz, 1H),4.10 (t, *J* = 8.5 Hz, 2H), 3.98 (s, 3H), 3.16 (t, *J* =8.5 Hz, 2H), 2.15 (s, 3H), 1.48 (d, *J* = 6.6 Hz, 6H) |
| 213 | 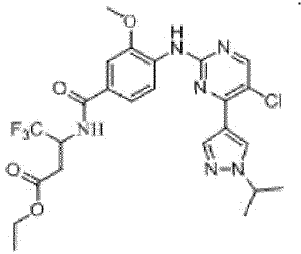 | ¹H NMR (300 MHz, MeOD) δ 8.63 - 8.54 (m, 2H), 8.44(s, 1H), 8.34 (s, 1H), 7.58 - 7.50 (m, 2H), 5.43 -5.21 (m, 1H), 4.65 (p, J = 6.7 Hz, 1H), 4.29 - 4.05(m, 2H), 4.02 (s, 3H), 3.03 - 2.83 (m, 2H), 1.57 (d, *J*= 6. 7 Hz, 6H), 1.22 (t, *J* = 7.1 Hz, 3H). |
| 214 | 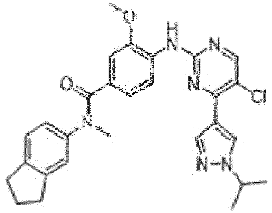 | ¹H NMR (300 MHz, Acetone-d6) δ 9.44 (s, 1H), 8.45 (s, 1H), 8.22 (s, 1H), 8.11 (s, 1H), 7.75 (s, 1H), 7.69 (d, *J* = 1.9 Hz, 1H), 7.63 (dd, *J* = 8.1, 1.9 Hz, 1H) ,7.60 - 7.53 (m, 1H), 7.40 (d, *J* = 8.0 Hz, 1H), 7.18 (d, *J* = 8.1 Hz, 1H), 4.62 (p, *J* = 6.6 Hz, 1H), 3.85(s, 3H), 3.46 (s, 3H), 2.88 (dt, *J* = 10.5, 7.4 Hz,4H), 2.14 - 2.00 (m, 4H), 1.50 (d, *J* = 6.7 Hz, 6H). |
| 215 | 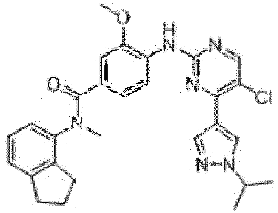 | ¹H NMR (300 MHz, DMSO-d6) δ 9.95 (s, 1H), 8.52 (s,1H), 8.31 (s, 1H), 8.09 (br s, 1H), 7.68 - 7.57 (m,2H), 7.42 (d, *J* = 8.0 Hz, 1H), 7.30 - 7.21 (m, 1H),7.21 - 7.08 (m, 2H), 4.63 (p, J = 6.7 Hz, 1H), 3.81(s, 3H), 3.42 (s, 3H), 2.89 (dt, *J* = 20.5, 7.4 Hz,4H), 2.01 (p, *J* = 7.5 Hz, 2H), 1.45 (d, *J* = 6.6 Hz,6H). |
| 216 | 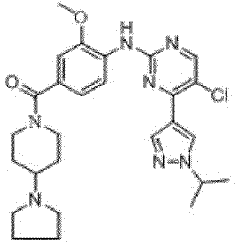 | ¹H NMR (300 MHz, Chloroform-d) δ 8.49 (d, *J*= 8.2 Hz, 1H), 8.34 (s, 1H), 8.31 (s, 1H), 8.30 (s, 1H), 7.80(s, 1H), 6.96 - 7.02 (m, 2H), 4.54 (m, 1H), 3.90 (s,3H), 2.84 - 3.03 (m, 2H), 2.65 - 2.74 (m, 4H), 2.41(m, 1H), 1.90 - 1.99 (m, 2H), 1.78 - 1.86 (m, 4H),1.56 - 1.69 (m, 2H), 1.54 (d, *J*= 6.7 Hz, 6H). |
| 217 | 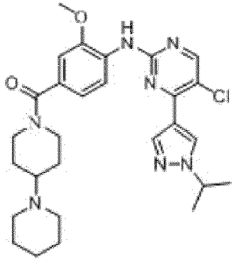 | ¹H NMR (300 MHz, Chloroform-*d*) δ 8.47 (d, *J*= 8.7 Hz, 1H), 8.29 (s, 1H), 8.27 (s, 1H), 8.25 (s, 1H), 7.76(s, 1H), 6.91 - 6.98 (m, 2H), 4.52 (m, 1H), 3.86 (s,3H), 2.47 - 3.05 (m, 8H), 1.73 - 1.95 (m, 2H), 1.45 -1.63 (m, 6H), 1.50 (d, *J*= 6.7 Hz, 6H), 1.33 - 1.45 (m,3H). |
| Comparati ve Compound 1 | 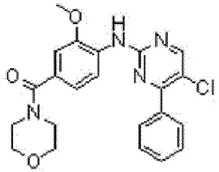 | ¹H NMR(300 MHz, CDCl3) δ 8.60 (d, J = 6.0 Hz, 1H),8.51 (s, 1H), 8.02 (s, 1H), 7.90 (m, 2H), 7.55 (m, 3H), 7.08 (s, 1H), 7.02 (d, J = 6.0 Hz, 1H), 3.97 (s,3H), 3.72 (m, 8H), 3.11 (d, J = 6.0 Hz. 3H) |
| Comparati ve Compound 2 | 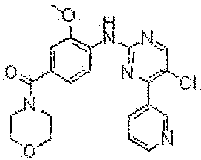 | ¹H NMR (300 MHz, CDCl3) δ 9.20 (s, 1H), 8.77 (s, 1H),8.53 (s, 1H), 8.51 (d, J = 8.2 Hz, 1H), 8.38 (d, J =7.6 Hz, 1H), 8.04 (s, 1H), 7.59 (d, J = 9.5 Hz, 1H),7.08 (s, 1H), 7.02 (d, J = 8.2 Hz, 1H), 3.97 (s, 3H),3.60 - 3.79 (m, 8H). |
| Comparati ve Compound 3 | 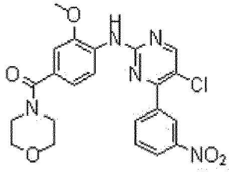 | ¹H NMR (300 MHz, CDCl3) δ 8.81 (t, J = 2.3 Hz, 1H),8.55 (s, 1H), 8.51 (d, J = 8.3 Hz, 1H), 8.38 (ddd, J =8.3, 2.3, 1.0 Hz, 1H), 8.27 (dt, J = 8.0, 1.4 Hz, 1H),8.03 (s, 1H), 7.71 (t, J = 8.0 Hz, 1H), 7.08 (d, J =1.8 Hz, 1H), 7.03 (dd, J = 8.3, 1.8 Hz, 1H), 3.97 (s,3H), 3.60 - 3.81 (m, 8H). |
| Comparati ve Compound 4 | 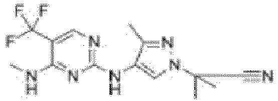 | DNL-151 (GNE-0877), Oral drug for Parkinson's disease |
| Comparati ve Compound 5 | 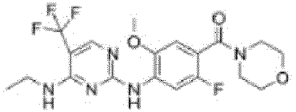 | DNL-201(GNE-7915), Oral drug for Parkinson's disease |

### Comparative Compound 1. (4-((5-chloro-4-phenylpyrimidin-2-yl)amino)-3-methoxyphenyl)(morpholino)methanone

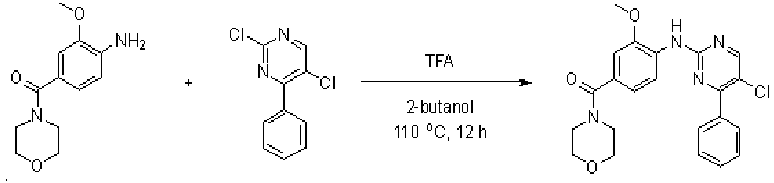

Into a 5-mL microwave vial were added 2,5-dichloro-4-phenylpyrimidin-4-amine (206 mg, 0.94 mmol), (4-amino-3-methoxyphenyl)(morpholino)methanone (200 mg, 0.85 mmol), anhydrous 2-BuOH (1.8 mL), and TFA (0.2 mL) which were then heated at 110°C for 12 hours. After completion of the reaction, MC and a saturated aqueous NaHCO₃ solution were added. The organic layer thus formed was dried (Mg₂SO₄), filtered, and concentrated. The crude product was dissolved in DCM, followed by isolation through column chromatography to afford the title compound as a white solid (110 mg) (yield 31%).

¹H NMR (300 MHz, CDCl3) δ 8.60 (d, J = 6.0 Hz, 1H), 8.51 (s, 1H), 8.02 (s, 1H), 7.90 (m, 2H), 7.55 (m, 3 H), 7.08 (s, 1H), 7.02 (d, J = 6.0 Hz, 1H), 3.97 (s, 3H), 3.72 (m, 8H), 3.11 (d, J = 6.0 Hz. 3H)

### Comparative Compound 2. (4-((5-chloro-4-(pyridin-3-yl)pyrimidin-2-yl)amino)-3-methoxyphenyl)(morpholino)methanone

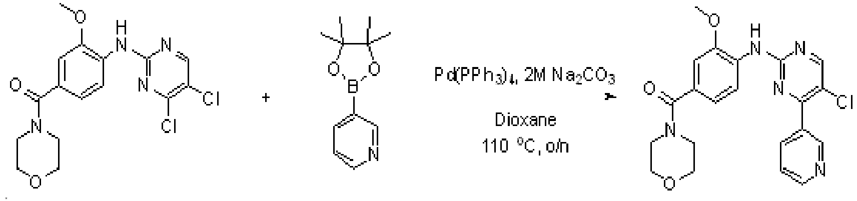

To a 5-mL microwave vial were added (4-((4,5-dichloropyrimidin-2-yl)amino)-3-methoxyphenyl)(morpholino)methanone (100 mg, 0.26 mmol), 3-(4,4,5,5-tetramethyl-1,3,2-dioxaborolan-2-yl)pyridine (48.5 mg, 0.236 mmol), 2M Na₂CO₃ solution (0.35 mL, 0.7 mmol), and dioxane (2 mL) which were then degassed twice. Subsequently, Pd(PPh₃)₄ (23 mg, 0.02 mmol) was added and degassing was conducted one more time. The mixture was stirred at 110°C for 12 hours. Following filtration through a Celite filter, ethyl acetate and water were added. The organic layer thus formed was dried (Mg₂SO₄), filtered, and concentrated. The crude product was dissolved in DCM, followed by isolation through column chromatography to afford the title compound as a white solid (67.3mg, 0.158 mmol) (yield 67%) .

¹H NMR (300 MHz, CDCl3) δ 9.20 (s, 1H), 8.77 (s, 1H), 8.53 (s, 1H), 8.51 (d, J = 8.2 Hz, 1H), 8.38 (d, J = 7.6 Hz, 1H), 8.04 (s, 1H), 7.59 (d, J = 9.5 Hz, 1H), 7.08 (s, 1H), 7.02 (d, J = 8.2 Hz, 1H), 3.97 (s, 3H), 3.60 - 3.79 (m, 8H).

### Comparative Compound 3. (4-((5-chloro-4-(3-nitrophenyl)pyrimidin-2-yl)amino)-3-methoxyphenyl)(morpholino)methanone

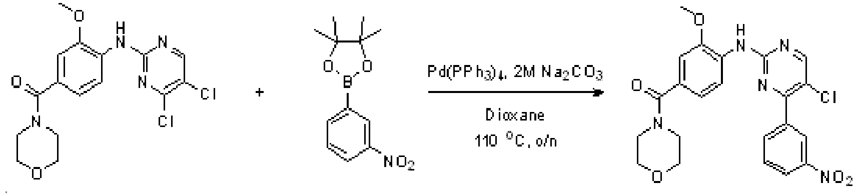

To a 5-mL microwave vial were added (4-((4,5-dichloropyrimidin-2-yl)amino)-3-methoxyphenyl)(morpholino)methanone (100 mg, 0.26 mmol), 4,4,5,5-tetramethyl-2-(3-nitrophenyl)-1,3,2-dioxaborolane (58.8 mg, 0.236 mmol), 2M Na₂CO₃ solution (0.35 mL, 0.7 mmol), and dioxane (2 mL) which were degassed twice. Subsequently, Pd(PPh₃)₄ (23 mg, 0.02 mmol) was added and degassing was conducted one more time. The mixture was stirred at 110°C for 12 hours. Following filtration through a Celite filter, ethyl acetate and water were added. The organic layer thus formed was dried (Mg₂SO₄), filtered, and concentrated. The crude product was dissolved in DCM, followed by isolation through column chromatography to afford the title compound as a white solid (65.3 mg, 0.139 mmol) (yield 59%).

¹H NMR (300 MHz, CDCl₃) δ 8.81 (t, J = 2.3 Hz, 1H), 8.55 (s, 1H), 8.51 (d, J = 8.3 Hz, 1H), 8.38 (ddd, J = 8.3, 2.3, 1.0 Hz, 1H), 8.27 (dt, J = 8.0, 1.4 Hz, 1H), 8.03 (s, 1H), 7.71 (t, J = 8.0 Hz, 1H), 7.08 (d, J = 1.8 Hz, 1H), 7.03 (dd, J = 8.3, 1.8 Hz, 1H), 3.97 (s, 3H), 3.60 - 3.81 (m, 8H).

### <EXAMPLE 2. Identification of Inhibitory Activity against LRRK2>

Representative compounds synthesized in Example 1 were assayed for inhibitory activity against wild-type LRRK2 and mutant LRRK2 (G2019S), and the results are summarized in Tables 2 and 3. In brief, a T7 phase strain carrying the wild-type kinase consisting of amino acid residues from H970 to E2527 of human-derived LRRK2 (Accession no. NP_940980.3) or a T7 phase strain carrying the G2019S mutation kinase was infected into E. coli strain BL21 which was then grown until log phase. After infection with T7 phases (multiplicity of infection = 0.4), the cells were cultured at 32°C for 90-150 minutes while shaking. Then, E. coli lysates were centrifuged (6,000 x g) and the supernatant was filtered through a 0.2-um filter. The kinases were produced in HEK 293 cells. In this regard, DNA was tagged to obtain DNA-tagged kinases for qPCR detection. For use in analyzing the kinases, affinity resins were prepared by treating streptavidin-coated magnetic beads with biotinylated small molecule ligands at room temperature for 30 minutes. Then, the liganded beads were blocked with an excess of biotin and washed with a blocking buffer (SeaBlock (Pierce), 1 % BSA, 0.05 % Tween 20, 1 mM DTT) to remove unbound ligands and thus to reduce the binding of non-specific phages. Subsequently, the prepared kinase, the ligand-affinity beads, and each of the compounds (10, 50, or 100 nM; used concentrations in Table 2, below) were mixed in 1x binding buffer (20 % SeaBlock, 0.17x PBS, 0.05 % Tween 20, 6 mM DTT) to form a final volume of 0.02 ml in each well of polypropylene 384-well plates, followed by incubation at room temperature while shaking. After one hour of incubation, the affinity beads were washed with a wash buffer (1x PBS, 0.05 % Tween 20), resuspended in an elution buffer (1x PBS, 0.05 % Tween 20, 0.5 µM non-biotinylated affinity ligand), and incubated at room temperature for 30 minutes while shaking. Afterwards, concentrations of wild-type LRRK2 and mutant LRRK2 (G2019S) in the eluted samples were quantitated by qPCR. Activity values are expressed as percentages relative to the control (% of ctrl). The compounds used in the activity assay were prepared into 40x stocks in 100% DMSO. The stocks were directly diluted during analysis procedures.

Assay results are summarized in Tables 2 and 3, below. Table 2 includes inhibitory activities of the representative compounds of the present disclosure at 100 nM against wild-type LRRK2 and mutant LRRK2 (G2019S) while Table 3 includes inhibitory activities of the representative compounds at 10 nM and 50 nM against wild-type LRRK2. (+++ less than 10%; ++ less than 30%; + less than 50%; - 50% or higher)

**TABLE 2**

| Compound No. | Remaining kinase activity(%) at 100 nM | |
|---|---|---|
| | LRRK2 (WT) | LRRK2(G2019Smutant) |
| Compound 1 | ++ | ++ |
| Compound 2 | +++ | +++ |
| Compound 4 | ++ | ++ |
| Compound 5 | +++ | +++ |
| Compound 6 | + | ++ |
| Compound 7 | +++ | +++ |
| Compound 10 | ++ | +++ |
| Compound 15 | + | ++ |
| Compound 16 | ++ | +++ |
| Compound 17 | ++ | + |
| Compound 18 | ++ | ++ |
| Compound 19 | +++ | +++ |
| Compound 20 | +++ | +++ |
| Compound 21 | ++ | ++ |
| Compound 22 | ++ | + |
| Compound 23 | + | + |
| Compound 24 | ++ | + |
| Compound 25 | ++ | + |
| Compound 26 | + | + |
| Compound 27 | +++ | +++ |
| Compound 28 | ++ | ++ |
| Compound 29 | + | ++ |
| Compound 31 | +++ | ++ |
| Compound 32 | + | ++ |
| Compound 33 | ++ | + |
| Compound 34 | +++ | ++ |
| Compound 35 | ++ | +++ |
| Compound 37 | +++ | +++ |
| Compound 38 | +++ | +++ |
| Compound 39 | + | +++ |
| Compound 40 | ++ | ++ |
| Compound 41 | ++ | + |
| Compound 42 | ++ | + |
| Compound 43 | +++ | ++ |
| Compound 44 | +++ | ++ |
| Compound 45 | ++ | ++ |
| Compound 46 | +++ | +++ |
| Compound 49 | +++ | +++ |
| Compound 50 | +++ | +++ |
| Compound 51 | ++ | ++ |
| Compound 54 | + | ++ |
| Compound 56 | +++ | +++ |
| Compound 57 | +++ | +++ |
| Compound 58 | ++ | +++ |
| Compound 59 | +++ | +++ |
| Comparative Compound 1 | - | - |
| Comparative Compound 2 | - | - |
| Comparative Compound 3 | - | - |

**TABLE 3**

| Compound No. | LRRK2(WT) Remaining kinase activity(%) | |
|---|---|---|
| | at 10 nM | at 50 nM |
| Compound 37 | + | ++ |
| Compound 60 | + | +++ |
| Compound 61 | + | ++ |
| Compound 62 | ++ | +++ |
| Compound 64 | ++ | +++ |
| Compound 70 | + | ++ |
| Compound 71 | + | ++ |
| Compound 72 | + | +++ |
| Compound 73 | + | ++ |
| Compound 74 | + | +++ |
| Compound 76 | + | ++ |
| Compound 77 | + | ++ |
| Compound 78 | + | +++ |
| Compound 79 | + | +++ |
| Compound 80 | + | ++ |
| Compound 81 | + | ++ |
| Compound 82 | + | ++ |
| Compound 84 | + | ++ |
| Compound 85 | + | ++ |
| Compound 87 | + | ++ |
| Compound 88 | + | +++ |
| Compound 89 | - | ++ |
| Compound 90 | - | ++ |
| Compound 91 | - | ++ |
| Compound 93 | + | +++ |
| Compound 94 | ++ | +++ |
| Compound 95 | - | ++ |
| Compound 96 | - | ++ |
| Compound 98 | ++ | +++ |
| Compound 99 | + | ++ |
| Compound 100 | + | ++ |
| Compound 101 | - | + |
| Compound 102 | + | +++ |
| Compound 103 | ++ | +++ |
| Compound 104 | - | ++ |
| Compound 105 | + | ++ |
| Compound 106 | - | ++ |
| Compound 108 | - | ++ |
| Compound 109 | - | ++ |
| Compound 110 | + | +++ |
| Compound 111 | + | +++ |
| Compound 112 | + | ++ |
| Compound 126 | - | + |
| Compound 127 | + | ++ |
| Compound 128 | - | + |
| Compound 129 | - | + |
| Compound 130 | - | ++ |
| Compound 138 | - | + |
| Compound 140 | - | ++ |
| Compound 141 | - | ++ |
| Compound 151 | - | + |
| Compound 152 | - | ++ |
| Compound 154 | - | ++ |
| Compound 166 | - | ++ |
| Compound 167 | - | ++ |
| Compound 168 | - | + |
| Compound 176 | - | + |
| Compound 179 | - | + |
| Compound 182 | - | + |
| Compound 183 | - | ++ |
| Compound 189 | - | + |
| Compound 190 | - | + |
| Compound 191 | - | ++ |
| Compound 192 | - | ++ |
| Compound 197 | - | + |
| Compound 198 | - | + |
| Compound 200 | - | + |
| Compound 202 | - | + |
| Compound 203 | - | + |
| Compound 204 | - | + |
| Compound 205 | - | + |
| Compound 210 | + | +++ |
| Compound 212 | - | + |
| Compound 216 | + | +++ |
| Compound 217 | ++ | +++ |

As can be seen in Tables 2 and 3, the pyrimidine derivatives of the present disclosure were identified to have inhibitory activity against LRRK2. At a concentration of 50 nM, the pyrimidine derivative compounds of Chemical Formula 1 wherein the substituent R₄ is a pyrazole and R₅ is a halogen or a haloalkyl exhibited very high inhibitory activity against LRRK2, compared to the other compounds.

### <EXAMPLE 3. Blood-Brain Barrier (BBB) Penetration>

Test substances were each orally administered at a dose of 10 mg/kg. Three hours later, the animals were sacrificed and blood samples were immediately taken from the heart and centrifuged to obtain plasma samples. In order to remove the blood remaining in the body, 20 ml of saline containing 10 U/ml heparin were perfused through the heart, after which the brain was excised. The brain was homogenized together with three volumes of a PBS buffer. For quantitative analysis in the prepared plasma and brain tissue, advantage was taken of LC-MS/MS (Agilent, Santa Clara, CA, USA). Separation in LC was conducted with a mobile phase consisting of 80% of acetonitrile and 20% of water containing 0.1% formic acid. For each substance, the eluting buffer was run for a total of 3 minutes at a flow rate of 0.3 ml/min. Each substance was quantitated using a multiple reaction monitoring method (for comparative compounds 4 and 5, the oral Parkinson's disease drugs DNL-151 and DNL-201 were used as LRRK2 protein inhibitors, respectively).

Three hours after oral administration, compound 37, representative of the compounds of the present disclosure, was measured to have 1.64- and 2.77-fold higher effective concentrations in the brain tissue, compared to comparative compound-4 (DNL-151) and comparative compound-5 (DNL-201), respectively.

The compound of the present disclosure was shown to be effective for treatment of neurodegenerative diseases including Parkinson's disease, and cancer.

From the result, it is understood that the pyrimidine derivative compounds of Chemical Formula 1 wherein the substituent R₄ is a pyrazole and R₅ is a halogen or a haloalkyl can be highly useful for preventing or treating neurodegenerative diseases and cancers as they effectively cross the blood-brain barrier, as well as inhibiting LRRK2 protein activity.

### <PREPARATION EXAMPLE 1. Preparation of Powder Formulation>

Compound 37 of the present disclosure ((4-((5-chloro-4-(1-isopropyl-1H-pyrazol-4-yl)pyrimidin-2-yl)amino)-3-methoxyphenyl)(morpholino)methanone) (2 g) and lactose (1 g) were mixed and loaded into an airtight pouch to prepare a powder formulation.

### <PREPARATION EXAMPLE 2. Preparation of Tablet Formulation>

Compound 37 of the present disclosure ((4-((5-chloro-4-(1-isopropyl-1H-pyrazol-4-yl)pyrimidin-2-yl)amino)-3-methoxyphenyl)(morpholino)methanone) (100 mg), microcrystalline cellulose (100 mg), lactose hydrate (60 mg), low-substituted hydroxypropyl cellulose (20 mg), and magnesium stearate (2 mg) were mixed and compressed into a tablet, using a typical tableting method.

### <PREPARATION EXAMPLE 3. Preparation of Capsule Formulation>

Compound 37 of the present disclosure ((4-((5-chloro-4-(1-isopropyl-1H-pyrazol-4-yl)pyrimidin-2-yl)amino)-3-methoxyphenyl)(morpholino)methanone) (100 mg), microcrystalline cellulose (100 mg), lactose hydrate (60 mg), low-substituted hydroxypropyl cellulose (20 mg), and magnesium stearate (2 mg) were mixed and loaded into a gelatin capsule according to a typical method to prepare a capsule formulation.

### <PREPARATION EXAMPLE 4. Preparation of Pill Formulation>

Compound 37 of the present disclosure ((4-((5-chloro-4-(1-isopropyl-1H-pyrazol-4-yl)pyrimidin-2-yl)amino)-3-methoxyphenyl)(morpholino)methanone) (90 mg), glutinous rice starch (5 mg), and purified water (5 mg) were mixed together with a small amount of anti-hydroscopic additives including dextrin, maltodextrin, corn starch, and microcrystalline cellulose (MCSS) and prepared into a pill (100 mg) according to a typical procedure.

### <PREPARATION EXAMPLE 5. Preparation of Injection Formulation>

Compound 37 of the present disclosure ((4-((5-chloro-4-(1-isopropyl-1H-pyrazol-4-yl)pyrimidin-2-yl)amino)-3-methoxyphenyl)(morpholino)methanone) (10 mg) was mixed with a suitable amount of sterile distilled water for injection, and a suitable amount of a pH adjustor per one ampoule (2 ml) and prepared into an injection formulation according to a typical method.

## Claims

1. A compound represented by the following Chemical Formula 1, an optical isomer thereof, or a pharmaceutically acceptable salt thereof: 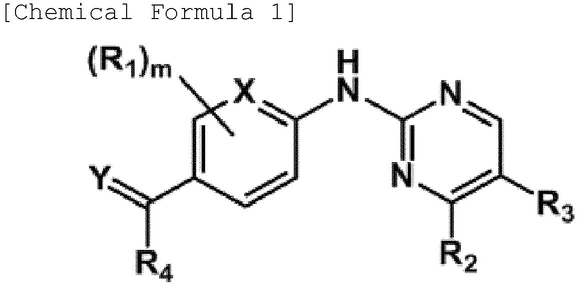 wherein,
R₁ is each independently selected from the group consisting of a hydrogen, a halogen, a hydroxy, a cyano, an amino, an amide, nitro, a C₁-C₆ alkyl, C₁-C₆ alkoxy, and a halo C₁-C₆ alkoxy;
R₂ is a substituted or unsubstituted pyrazole, wherein the substituted pyrazole has as a substituent thereon at least one radical selected from the group consisting of a halogen, a hydroxy, a cyano, an amino, an amide, a nitro, a C₁-C₆ alkylsulfonyl, an acetyl, a methyloxycarbonyl, a substituted or unsubstituted C₁-C₆ alkyl, a substituted or unsubstituted benzyl, a substituted or unsubstituted C₃-C₁₂ cycloalkyl, a substituted or unsubstituted C₃-C₁₂ heterocycloalkyl, a substituted or unsubstituted C₄-C₁₂ aryl, and a substituted or unsubstituted C₄-C₁₂ hetaroaryl,
wherein the substituted alkyl, the substituted benzyl, the substituted C₃-C₁₂ cycloalkyl, the substituted C₄-C₁₂ aryl, and the substituted C₄-C₁₂ hetaroaryl have each as a substituent thereon at least one radical selected from the group consisting of a halogen, a hydroxy, a cyano, an amino, an amide, a nitro, a C₁-C₆ alkyl, a C₁-C₆ alkoxy, a C₃-C₁₂ heterocycloalkyl, a C₄-C₁₂ an aryl-C₁-C₆ alkoxy, and a - C (=O) O-alkyl (C₁-C₆) ;
R₃ is a halogen or a halo C₁-C₆ alkyl;
R₄ is represented by any one of B-1 to B-29, below, 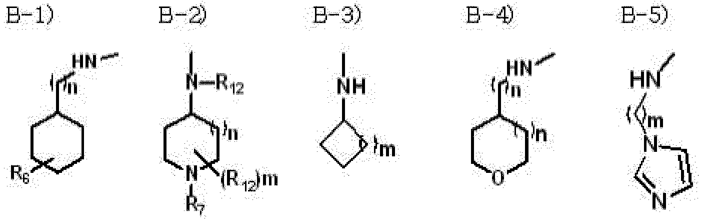 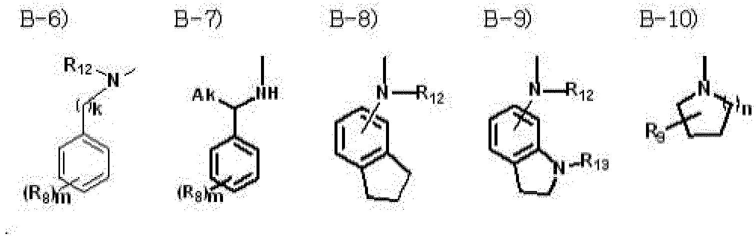 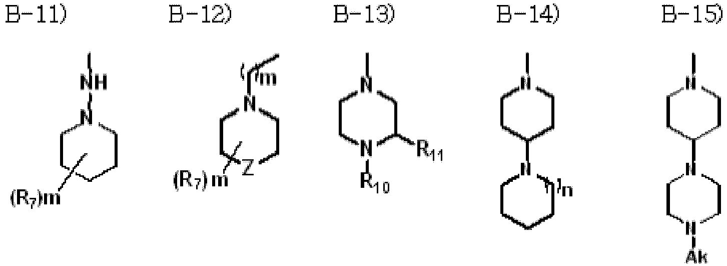 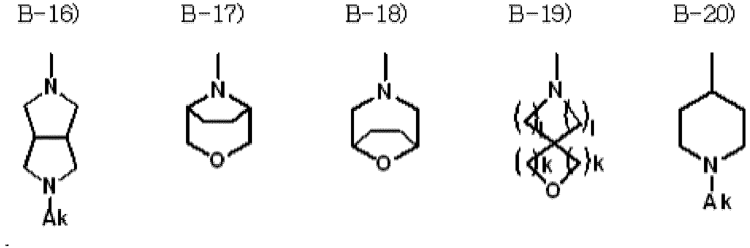 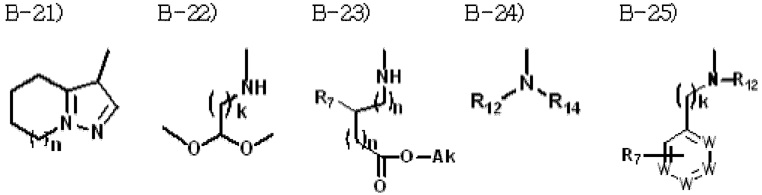 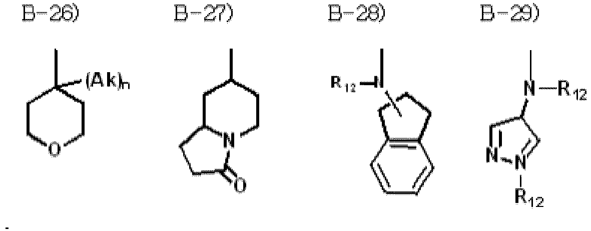 wherein,
R₆ is selected from the group consisting of a hydrogen, a halogen, a hydroxy, a C₁-C₆ alkyl, a C₁-C₆ hydroxyalkyl, a C₁-C₆ alkylsulfonate, and a C₁-C₆ alkylamino;
R₇ is each independently selected from the group consisting of a hydrogen, a halogen, a halo C₁-C₆ alkyl, and a C₁-C₆ alkyl;
R₈ is each independently selected from the group consisting of a hydrogen, a halogen, an amino, a hydroxy, a cyano, a C₁-C₆ dialkylamino, an aminosulfonyl, a C₁-C₆ alkylsulfonamino, a C₁-C₆ alkyl, a halo C₁-C₆ alkyl, a halo C₁-C₆ alkyloxy, and a C₁-C₆ alkoxy;
R₉ is selected from the group consisting of a hydrogen, a halogen, a hydroxy, a hydroxy C₁-C₆ alkyl, a C₁-C₆ alkylsulfonyl, a C₁-C₆ alkylacetyl, a C₁-C₆ alkyloxycarbonyl, a C₁-C₆ alkylamino, a C₁-C₆ alkoxy, a morpholine, a piperidine, a pyrrolidine, an oxetanyl, a C₁-C₆ alkyl-substituted piperidine, and an acetate;
R₁₀ is selected from the group consisting of a hydrogen, a C₁-C₆ alkyl, a phenyl, an oxetanyl, and an acetate;
R₁₁ is a hydrogen or bonds to R₁₀ to form a saturated or unsaturated C₅-C₆ heterocycloalkyl;
R₁₂ is a hydrogen or a C₁-C₆ alkyl;
R₁₃ is a hydrogen, a C₁-C₆ alkylcarbonyl, or a C₁-C₆ alkylacetyl;
R₁₄ is a hydrogen, a C₁-C₆ alkyl, a hydroxy C₁-C₆ alkyl, a C₁-C₆ alkoxy C₁-C₆ alkyl, a C₁-C₆ alkyloxycarbonyl C₁-C₆ alkyl, or a methylsulfonyl C₁-C₆ alkyl;
Ak is a C₁-C₆ alkyl;
k is each independently 0, 1, or 2 (with a provision that all k's are non-zero simultaneously);
1 is 1 or 2;
m is 0, 1, 2, 3, or 4;
n is 0 or 1,
W is each independently N;
Z is O or SO₂;
X is C, CH, or N; and
Y is selected from the group consisting of O and S.

2. The compound, the optical isomer thereof, or the pharmaceutically acceptable salt thereof according to claim 1, wherein the compound represented by Chemical Formula 1 is selected from the group consisting of:
(4-((5-chloro-4-(1-methyl-1H-pyrazol-4-yl)pyrimidin-2-yl)amino)-3-methoxyphenyl)(morpholino)methanone (Compound 1);
(4-((5-chloro-4-(1-methyl-1H-pyrazol-4-yl)pyrimidin-2-yl)amino)-3-methoxyphenyl)(4-morpholinopiperidin-1-yl)methanone (Compound 2);
1-(4-(5-chloro-2-((2-methoxy-4-(morpholine-4-carbonyl)phenyl)amino)pyrimidin-4-yl)-1H-pyrazol-1-yl)ethan-1-one (Compound 3);
(4-((5-chloro-4-(1-(2-hydroxyethyl)-1H-pyrazol-4-yl)pyrimidin-2-yl)amino)-3-methoxyphenyl)(morpholino)methanone (Compound 4);
3-(4-(5-chloro-2-((2-methoxy-4-(morpholine-4-carbonyl)phenyl)amino)pyrimidin-4-yl)-1H-pyrazol-1-yl)propanenitrile (Compound 5);
2-(4-(5-chloro-2-((2-methoxy-4-(morpholine-4-carbonyl)phenyl)amino)pyrimidin-4-yl)-1H-pyrazol-1-yl)acetonitrile (Compound 6);
3-(4-(5-chloro-2-((2-methoxy-4-(morpholine-4-carbonyl)phenyl)amino)pyrimidin-4-yl)-1H-pyrazol-1-yl)butanenitrile (Compound 7);
(4-((5-chloro-4-(1-(2-morpholinoethyl)-1H-pyrazol-4-yl)pyrimidin-2-yl)amino)-3-methoxyphenyl)(morpholino)methanone (Compound 8);
methyl 2-(4-(5-chloro-2-((2-methoxy-4-(morpholine-4-carbonyl)phenyl)amino)pyrimidin-4-yl)-1H-pyrazol-1-yl)acetate (Compound 9);
(4-((5-chloro-4-(1-(methylsulfonyl)-1H-pyrazol-4-yl)pyrimidin-2-yl)amino)-3-methoxyphenyl)(morpholino)methanone (Compound 10);
(4-((5-chloro-4-(1-(pyridin-2-yl)-1H-pyrazol-4-yl)pyrimidin-2-yl)amino)-3-methoxyphenyl)(morpholino)methanone (Compound 11);
(4-((5-chloro-4-(1-(pyridin-3-yl)-1H-pyrazol-4-yl)pyrimidin-2-yl)amino)-3-methoxyphenyl)(morpholino)methanone (Compound 12);
(4-((5-chloro-4-(1-(pyridin-4-yl)-1H-pyrazol-4-yl)pyrimidin-2-yl)amino)-3-methoxyphenyl)(morpholino)methanone (Compound 13);
(4-((5-chloro-4-(1-phenyl-1H-pyrazol-4-yl)pyrimidin-2-yl)amino)-3-methoxyphenyl)(morpholino)methanone (Compound 14);
(4-((5-chloro-4-(1-(difluoromethyl)-1H-pyrazol-4-yl)pyrimidin-2-yl)amino)-3-methoxyphenyl)(morpholino)methanone (Compound 15);
(4-((5-chloro-4-(1-(methoxymethyl)-1H-pyrazol-4-yl)pyrimidin-2-yl)amino)-3-methoxyphenyl)(morpholino)methanone (Compound 16);
(4-((5-chloro-4-(1-(4-methoxybenzyl)-1H-pyrazol-4-yl)pyrimidin-2-yl)amino)-3-methoxyphenyl)(morpholino)methanone (Compound 17);
(4-((5-chloro-4-(1H-pyrazol-4-yl)pyrimidin-2-yl)amino)-3-methoxyphenyl)(morpholino)methanone (Compound 18);
(4-((5-chloro-4-(1-methyl-1H-pyrazol-4-yl)pyrimidin-2-yl)amino)-3-methoxyphenyl)(4-(4-methylpiperazin-1-yl)piperidin-1-yl)methanone (Compound 19) ;
(4-((5-chloro-4-(1-(tetrahydro-2H-pyran-4-yl)-1H-pyrazol-4-yl)pyrimidin-2-yl)amino)-3-methoxyphenyl)(morpholino)methanone (Compound 20);
4-((5-chloro-4-(1-methyl-1H-pyrazol-4-yl)pyrimidin-2-yl)amino)-3-methoxy-N-(2-methoxyethyl)-N-methylbenzamide (Compound 21);
(4-((5-chloro-4-(1-methyl-1H-pyrazol-4-yl)pyrimidin-2-yl)amino)-3-methoxyphenyl)(pyrrolidin-1-yl)methanone (Compound 22);
4-((5-chloro-4-(1-methyl-1H-pyrazol-4-yl)pyrimidin-2-yl)amino)-N-ethyl-3-methoxy-N-(2-methoxyethyl)benzamide(Compound 23);
4-((5-chloro-4-(1-methyl-1H-pyrazol-4-yl)pyrimidin-2-yl)amino)-N-(1-hydroxy-2-methylpropan-2-yl)-3-methoxybenzamide (Compound 24);
N-(tert-butyl)-4-((5-chloro-4-(1-methyl-1H-pyrazol-4-yl)pyrimidin-2-yl)amino)-3-methoxybenzamide(Compound 25);
4-((5-chloro-4-(1-methyl-1H-pyrazol-4-yl)pyrimidin-2-yl)amino)-N-(2-hydroxy-2-methylpropyl)-3-methoxybenzamide (Compound 26);
3-(4-(5-chloro-2-((2-methoxy-4-(morpholine-4-carbonyl)phenyl)amino)pyrimidin-4-yl)-1H-pyrazol-1-yl)-3-methylbutanenitrile (Compound 27);
(4-((5-chloro-4-(1-(2-methoxyethyl)-1H-pyrazol-4-yl)pyrimidin-2-yl)amino)-3-methoxyphenyl)(morpholino)methanone (Compound 28);
(3-methoxy-4-((4-(1-(tetrahydro-2H-pyran-4-yl)-1H-pyrazol-4-yl)-5-(trifluoromethyl)pyrimidin-2-yl)amino)phenyl)(morpholino)methanone (Compound 29);
4-((5-chloro-4-(1-methyl-1H-pyrazol-4-yl)pyrimidin-2-yl)amino)-N-(4,4-difluorocyclohexyl)-3-methoxybenzamide (Compound 30);
(3-methoxy-4-((4-(1-methyl-1H-pyrazol-4-yl)-5-(trifluoromethyl)pyrimidin-2-yl)amino)phenyl)(4-(4-methylpiperazin-1-yl)piperidin-1-yl)methanone (Compound 31) ;
(4-((5-chloro-4-(1-methyl-1H-pyrazol-4-yl)pyrimidin-2-yl)amino)-3-methoxyphenyl)(2-oxa-7-azaspiro[3.5]nonan-7-yl)methanone (Compound 32);
(4-((5-chloro-4-(1-methyl-1H-pyrazol-4-yl)pyrimidin-2-yl)amino)-3-methoxyphenyl) (1-oxa-6-azaspiro[3.3]heptan-6-yl)methanone (Compound 33);
(4-((5-chloro-4-(1-methyl-1H-pyrazol-4-yl)pyrimidin-2-yl)amino)-3-methoxyphenyl)(4-isopropylpiperazin-1-yl)methanone (Compound 34);
(4-((5-chloro-4-(1-(tetrahydro-2H-pyran-4-yl)-1H-pyrazol-4-yl)pyrimidin-2-yl)amino)-3-methoxyphenyl)(2-oxa-7-azaspiro[3.5]nonan-7-yl)methanone (Compound 35);
(3-methoxy-4-((4-(1-methyl-1H-pyrazol-4-yl)-5-(trifluoromethyl)pyrimidin-2-yl)amino)phenyl)(morpholino)methanone (Compound 36);
(4-((5-chloro-4-(1-isopropyl-1H-pyrazol-4-yl)pyrimidin-2-yl)amino)-3-methoxyphenyl)(morpholino)methanone (Compound 37);
4-((5-chloro-4-(1-methyl-1H-pyrazol-4-yl)pyrimidin-2-yl)amino)-3-methoxy-N-(1-methylpiperidin-4-yl)benzamide (Compound 38);
(4-((5-chloro-4-(1-methyl-1H-pyrazol-4-yl)pyrimidin-2-yl)amino)-3-methoxyphenyl)(4-(2-hydroxypropan-2-yl)piperidin-1-yl)methanone (Compound 39) ;
(4-((5-chloro-4-(1-methyl-1H-pyrazol-4-yl)pyrimidin-2-yl)amino)-3-methoxyphenyl)(2-oxa-6-azaspiro[3.3]heptan-6-yl)methanone (Compound 40);
(4-((5-chloro-4-(1-methyl-1H-pyrazol-4-yl)pyrimidin-2-yl)amino)-3-methoxyphenyl)(4,4-difluoropiperidin-1-yl)methanone (Compound 41);
(4-((5-chloro-4-(1-methyl-1H-pyrazol-4-yl)pyrimidin-2-yl)amino)-3-methoxyphenyl)(2,2-dimethylmorpholino)methanone (Compound 42);
(4-((5-chloro-4-(1-methyl-1H-pyrazol-4-yl)pyrimidin-2-yl)amino)-3-methoxyphenyl)(3-morpholinoazetidin-1-yl)methanone (Compound 43);
1-(4-(4-((5-chloro-4-(1-methyl-1H-pyrazol-4-yl)pyrimidin-2-yl)amino)-3-methoxybenzoyl)piperazin-1-yl)ethan-1-one(Compound 44);
(4-isopropylpiperazin-1-yl) (3-methoxy-4- ((4- (1-methyl-1H-pyrazol-4-yl)-5-(trifluoromethyl)pyrimidin-2-yl)amino)phenyl)methanone (Compound 45);
(2-fluoro-5-methoxy-4-((4-(1-methyl-1H-pyrazol-4-yl)-5-(trifluoromethyl)pyrimidin-2-yl)amino)phenyl)(morpholino)methanone (Compound 46);
(3-methoxy-4-((4-(1-methyl-1H-pyrazol-4-yl)-5-(trifluoromethyl)pyrimidin-2-yl)amino)phenyl)(4-phenylpiperazin-1-yl)methanone (Compound 47);
(2-fluoro-5-methoxy-4-((4-(1-methyl-1H-pyrazol-4-yl)-5-(trifluoromethyl)pyrimidin-2-yl)amino)phenyl) (4-isopropylpiperazin-1-yl)methanone (Compound 48);
(4-((5-chloro-4-(1-isopropyl-1H-pyrazol-4-yl)pyrimidin-2-yl)amino)-2-fluoro-5-methoxyphenyl)(morpholino)methanone (Compound 49);
(4-((5-chloro-4-(1-methyl-1H-pyrazol-4-yl)pyrimidin-2-yl)amino)-3-methoxyphenyl)(4-(oxetan-3-yl)piperazin-1-yl)methanone (Compound 50);
((1R,5S)-8-oxa-3-azabicyclo[3.2.1]octan-3-yl) (4-((5-chloro-4-(1-methyl-1H-pyrazol-4-yl)pyrimidin-2-yl)amino)-3-methoxyphenyl)methanone (Compound 51);
(4-((5-chloro-4-(1-ethyl-1H-pyrazol-4-yl)pyrimidin-2-yl)amino)-3-methoxyphenyl) (pyridin-4-yl)methanone (Compound 52);
(4-((5-chloro-4-(1-propyl-1H-pyrazol-4-yl)pyrimidin-2-yl)amino)-3-methoxyphenyl) (pyridin-4-yl)methanone (Compound 53);
(4-((5-chloro-4-(1-isopropyl-1H-pyrazol-4-yl)pyrimidin-2-yl)amino)-3-methoxyphenyl) (pyridin-4-yl)methanone (Compound 54);
(4-((5-chloro-4-(1-methyl-1H-pyrazol-4-yl)pyrimidin-2-yl)amino)-3-methoxyphenyl) (pyridin-4-yl)methanone (Compound 55);
(4-((5-chloro-4-(1-ethyl-1H-pyrazol-4-yl)pyrimidin-2-yl)amino)-3-methoxyphenyl)(morpholino)methanone (Compound 56);
(4-((5-chloro-4-(1-propyl-1H-pyrazol-4-yl)pyrimidin-2-yl)amino)-3-methoxyphenyl)(morpholino)methanone (Compound 57);
(4-((5-chloro-4-(1-cyclopropyl-1H-pyrazol-4-yl)pyrimidin-2-yl)amino)-3-methoxyphenyl)(morpholino)methanone (Compound 58);
(4-((4-(1-(tert-butyl)-1H-pyrazol-4-yl)-5-chloropyrimidin-2-yl)amino)-3-methoxyphenyl)(morpholino)methanone (Compound 59);
4-((5-chloro-4-(1-isopropyl-1H-pyrazol-4-yl)pyrimidin-2-yl)amino)-3-methoxy-N-(1-methylpiperidin-4-yl)benzamide (Compound 60);
(4-((5-chloro-4-(1-methyl-1H-pyrazol-4-yl)pyrimidin-2-yl)amino)-3-methoxyphenyl)(4-methylpiperazin-1-yl)methanone (Compound 61);
(4-((5-chloro-4-(1-isopropyl-1H-pyrazol-4-yl)pyrimidin-2-yl)amino)-3-methoxyphenyl)(4-methylpiperazin-1-yl)methanone (Compound 62);
(4-((4-(1-isopropyl-1H-pyrazol-4-yl)-5-(trifluoromethyl)pyrimidin-2-yl)amino)-3-methoxyphenyl)(morpholino)methanone (Compound 63);
(4-((5-chloro-4-(1-isopropyl-1H-pyrazol-4-yl)pyrimidin-2-yl)amino)-3-methoxyphenyl)(4-isopropylpiperazin-1-yl)methanone (Compound 64);
(2-fluoro-4-((4-(1-isopropyl-1H-pyrazol-4-yl)-5-(trifluoromethyl)pyrimidin-2-yl)amino)-5-methoxyphenyl)(morpholino)methanone (Compound 65);
(4-((5-chloro-4-(1-isopropyl-1H-pyrazol-4-yl)pyrimidin-2-yl)amino)-3-methoxyphenyl)(tetrahydro-2H-pyran-4-yl)methanone (Compound 66);
(4-((5-chloro-4-(1-methyl-1H-pyrazol-4-yl)pyrimidin-2-yl)amino)-3-methoxyphenyl)(4-methyltetrahydro-2H-pyran-4-yl)methanone (Compound 67);
(4-((5-chloro-4-(1-isopropyl-1H-pyrazol-4-yl)pyrimidin-2-yl)amino)-3-methoxyphenyl)(4-methyltetrahydro-2H-pyran-4-yl)methanone (Compound 68);
(4-((5-chloro-4-(1-methyl-1H-pyrazol-4-yl)pyrimidin-2-yl)amino)-2-fluoro-5-methoxyphenyl)(morpholino)methanone (Compound 69);
(4-((5-chloro-4-(1-isopropyl-1H-pyrazol-4-yl)pyrimidin-2-yl)amino)-3-methoxyphenyl)(4-(2-hydroxypropan-2-yl)piperidin-1-yl)methanone (Compound 70) ;
(4-((5-chloro-4-(1-isopropyl-1H-pyrazol-4-yl)pyrimidin-2-yl)amino)-3-methoxyphenyl)(2-oxa-7-azaspiro[3.5]nonan-7-yl)methanone (Compound 71);
(4-((5-chloro-4-(1-isopropyl-1H-pyrazol-4-yl)pyrimidin-2-yl)amino)-3-methoxyphenyl) (4-(oxetan-3-yl)piperazin-1-yl)methanone (Compound 72);
(4-((5-chloro-4-(1-isopropyl-1H-pyrazol-4-yl)pyrimidin-2-yl)amino)-3-methoxyphenyl) (3-morpholinoazetidin-1-yl)methanone (Compound 73);
(4-((5-chloro-4-(1-isopropyl-1H-pyrazol-4-yl)pyrimidin-2-yl)amino)-3-methoxyphenyl)(morpholino)methanethione (Compound 74);
(4-((5-chloro-4-(1-isopropyl-1H-pyrazol-4-yl)pyrimidin-2-yl)amino)-3-methoxyphenyl) (1-isopropylpiperidin-4-yl)methanone (Compound 75);
(4-((5-chloro-4-(1-isopropyl-1H-pyrazol-4-yl)pyrimidin-2-yl)amino)-3-methoxyphenyl) (4-methoxypiperidin-1-yl)methanone (Compound 76);
(4-((5-chloro-4-(1-isopropyl-1H-pyrazol-4-yl)pyrimidin-2-yl)amino)-3-methoxyphenyl) (3-oxa-9-azaspiro[5.5]undecan-9-yl)methanone (Compound 77);
1-(4-(4-((5-chloro-4-(1-isopropyl-1H-pyrazol-4-yl)pyrimidin-2-yl)amino)-3-methoxybenzoyl)piperazin-1-yl)ethan-1-one(Compound 78);
(4-((5-chloro-4-(1-isopropyl-1H-pyrazol-4-yl)pyrimidin-2-yl)amino)-3-methoxyphenyl) (4-(methylsulfonyl)piperidin-1-yl)methanone (Compound 79);
4-((5-chloro-4-(1-isopropyl-1H-pyrazol-4-yl)pyrimidin-2-yl)amino)-3-methoxy-N-(tetrahydro-2H-pyran-4-yl)benzamide(Compound 80);
N-(1-(2-(o-tolyl)quinolin-3-yl)ethyl)-6,7-dihydro-5H-pyrrolo[2,3-d]pyrimidin-4-amine (Compound 81);
4-((5-chloro-4-(1-isopropyl-1H-pyrazol-4-yl)pyrimidin-2-yl)amino)-N-cyclopropyl-3-methoxybenzamide(Compound 82);
(4-((5-chloro-4-(1-isopropyl-1H-pyrazol-4-yl)pyrimidin-2-yl)amino)-3-methoxyphenyl) (3-hydroxyazetidin-1-yl)methanone (Compound 83);
(4-((5-chloro-4-(1-isopropyl-1H-pyrazol-4-yl)pyrimidin-2-yl)amino)-3-methoxyphenyl)(1,1-dioxidothiomorpholino)methanone (Compound 84);
(4-((4-(1-(tert-butyl)-1H-pyrazol-4-yl)-5-chloropyrimidin-2-yl) amino)-3-methoxyphenyl) (4-methylpiperazin-1-yl)methanone (Compound 85);
(S)-(4-((5-chloro-4-(1-isopropyl-1H-pyrazol-4-yl)pyrimidin-2-yl)amino)-3-methoxyphenyl)(2-(hydroxymethyl)pyrrolidin-1-yl)methanone (Compound 86);
(4-((5-chloro-4-(1-isopropyl-1H-pyrazol-4-yl)pyrimidin-2-yl)amino)-3-methoxyphenyl)(hexahydropyrrolo[1,2-a]pyrazin-2(1H)-yl)methanone (Compound 87);
(4-((4-(1-(tert-butyl)-1H-pyrazol-4-yl)-5-chloropyrimidin-2-yl) amino)-3-methoxyphenyl) (4-isopropylpiperazin-1-yl)methanone (Compound 88);
((1R,5S)-8-oxa-3-azabicyclo[3.2.1]octan-3-yl)(4-((5-chloro-4-(1-isopropyl-1H-pyrazol-4-yl)pyrimidin-2-yl)amino)-3-methoxyphenyl)methanone (Compound 89);
(4-((5-chloro-4-(1-isopropyl-1H-pyrazol-4-yl)pyrimidin-2-yl)amino)-3-methoxyphenyl)(2-oxa-6-azaspiro[3.3]heptan-6-yl)methanone (Compound 90);
4-((5-chloro-4-(1-isopropyl-1H-pyrazol-4-yl)pyrimidin-2-yl)amino)-N-((1r,4r)-4-hydroxycyclohexyl)-3-methoxybenzamide(Compound 91);
(6-((5-chloro-4-(1-isopropyl-1H-pyrazol-4-yl)pyrimidin-2-yl)amino)pyridin-3-yl)(morpholino)methanone (Compound 92);
4-((5-chloro-4-(1-isopropyl-1H-pyrazol-4-yl)pyrimidin-2-yl)amino)-N-(4-(dimethylamino)cyclohexyl)-3-methoxybenzamide (Compound 93) ;
(4-((5-chloro-4-(1-isopropyl-1H-pyrazol-4-yl)pyrimidin-2-yl)amino)-3-methoxyphenyl)(4-(dimethylamino)piperidin-1-yl)methanone (Compound 94);
((1R,5S)-3-oxa-8-azabicyclo[3.2.1]octan-8-yl) (4-((5-chloro-4-(1-isopropyl-1H-pyrazol-4-yl)pyrimidin-2-yl)amino)-3-methoxyphenyl)methanone (Compound 95);
4-((4-(1-(tert-butyl)-1H-pyrazol-4-yl)-5-chloropyrimidin-2-yl)amino)-3-methoxy-N-(1-methylpiperidin-4-yl)benzamide(Compound 96);
(R)-(4-((5-chloro-4-(1-isopropyl-1H-pyrazol-4-yl)pyrimidin-2-yl)amino)-3-methoxyphenyl) (3-hydroxypyrrolidin-1-yl)methanone (Compound 97);
(4-((5-chloro-4-(1-isopropyl-1H-pyrazol-4-yl)pyrimidin-2-yl)amino)-3-methoxyphenyl) (5-methylhexahydropyrrolo[3,4-c]pyrrol-2(1H)-yl)methanone (Compound 98);
4-((5-chloro-4-(1-isopropyl-1H-pyrazol-4-yl)pyrimidin-2-yl)amino)-N-(1-isopropylpiperidin-4-yl)-3-methoxybenzamide (Compound 99);
2-(4-((5-chloro-4-(1-isopropyl-1H-pyrazol-4-yl)pyrimidin-2-yl)amino)-3-methoxybenzoyl)hexahydropyrrolo[1,2-a]pyrazin-6(2H)-one (Compound 100);
4-((5-chloro-4-(1-isopropyl-1H-pyrazol-4-yl)pyrimidin-2-yl)amino)-3-methoxy-N,N-dimethylbenzamide(Compound 101);
(4-((5-chloro-4-(1-isopropyl-1H-pyrazol-4-yl)pyrimidin-2-yl)amino)-2-fluoro-5-methoxyphenyl) (4-methylpiperazin-1-yl) methanethione (Compound 102);
(4-((5-chloro-4-(1-isopropyl-1H-pyrazol-4-yl)pyrimidin-2-yl)amino)-3-methoxyphenyl) (4-methylpiperazin-1-yl) methanethione (Compound 103);
(4-((5-chloro-4-(1-isopropyl-1H-pyrazol-4-yl)pyrimidin-2-yl)amino)-2-fluoro-5-methoxyphenyl) (4-methylpiperazin-1-yl)methanone (Compound 104);
(4-((5-chloro-4-(1-isopropyl-1H-pyrazol-4-yl)pyrimidin-2-yl)amino)-3-(difluoromethoxy)phenyl)(morpholino)methanone (Compound 105) ;
(4-((5-chloro-4-(1-isopropyl-1H-pyrazol-4-yl)pyrimidin-2-yl)amino)-2-fluoro-5-methoxyphenyl)(morpholino) methanethione (Compound 106);
(5-chloro-4-((5-chloro-4-(1-isopropyl-1H-pyrazol-4-yl)pyrimidin-2-yl)amino)-2-fluorophenyl)(4-methylpiperazin-1-yl)methanone (Compound 107);
(4-((5-chloro-4-(1-isopropyl-1H-pyrazol-4-yl)pyrimidin-2-yl)amino)-3-(difluoromethoxy)phenyl) (4-methylpiperazin-1-yl)methanone (Compound 108);
(4-((5-chloro-4-(1-isopropyl-1H-pyrazol-4-yl)pyrimidin-2-yl)amino)-3-methylphenyl)(4-methylpiperazin-1-yl)methanone (Compound 109);
(4-((5-chloro-4-(1-isopropyl-1H-pyrazol-4-yl)pyrimidin-2-yl) amino)-2-fluoro-5-methoxyphenyl) (4-(dimethylamino)piperidin-1-yl)methanone (Compound 110);
(4-((5-chloro-4-(1-isopropyl-1H-pyrazol-4-yl)pyrimidin-2-yl)amino)-2-fluoro-5-methoxyphenyl) (4-(dimethylamino)piperidin-1-yl)methanone (Compound 111);
4-((5-chloro-4-(1-isopropyl-1H-pyrazol-4-yl)pyrimidin-2-yl)amino)-3-methoxy-N-phenylbenzamide(Compound 112);
4-((5-chloro-4-(1-isopropyl-1H-pyrazol-4-yl)pyrimidin-2-yl)amino)-N-(2-fluorophenyl)-3-methoxybenzamide(Compound 113);
4-((5-chloro-4-(1-isopropyl-1H-pyrazol-4-yl)pyrimidin-2-yl)amino)-N-(3-fluorophenyl)-3-methoxybenzamide(Compound 114);
4-((5-chloro-4-(1-isopropyl-1H-pyrazol-4-yl)pyrimidin-2-yl)amino)-N-(4-fluorophenyl)-3-methoxybenzamide(Compound 115);
4-((5-chloro-4-(1-isopropyl-1H-pyrazol-4-yl)pyrimidin-2-yl)amino)-N-(3-cyanophenyl)-3-methoxybenzamide(Compound 116);
4-((5-chloro-4-(1-isopropyl-1H-pyrazol-4-yl)pyrimidin-2-yl)amino)-N-(4-cyanophenyl)-3-methoxybenzamide(Compound 117);
4-((5-chloro-4-(1-isopropyl-1H-pyrazol-4-yl)pyrimidin-2-yl)amino)-N-(2-fluorobenzyl)-3-methoxybenzamide(Compound 118);
4-((5-chloro-4-(1-isopropyl-1H-pyrazol-4-yl)pyrimidin-2-yl)amino)-N-(2-fluorobenzyl)-3-methoxybenzamide(Compound 119);
4-((5-chloro-4-(1-isopropyl-1H-pyrazol-4-yl)pyrimidin-2-yl)amino)-N-(4-fluorobenzyl)-3-methoxybenzamide(Compound 120);
4-((5-chloro-4-(1-isopropyl-1H-pyrazol-4-yl)pyrimidin-2-yl)amino)-3-methoxy-N-(o-tolyl)benzamide(Compound 121);
4-((5-chloro-4-(1-isopropyl-1H-pyrazol-4-yl)pyrimidin-2-yl)amino)-3-methoxy-N-(m-tolyl)benzamide(Compound 122);
4-((5-chloro-4-(1-isopropyl-1H-pyrazol-4-yl)pyrimidin-2-yl)amino)-3-methoxy-N-(p-tolyl)benzamide(Compound 123);
4-((5-chloro-4-(1-isopropyl-1H-pyrazol-4-yl)pyrimidin-2-yl)amino)-3-methoxy-N-(2-methylbenzyl)benzamide (Compound 124);
4-((5-chloro-4-(1-isopropyl-1H-pyrazol-4-yl)pyrimidin-2-yl)amino)-3-methoxy-N-(3-methylbenzyl)benzamide (Compound 125);
4-((5-chloro-4-(1-isopropyl-1H-pyrazol-4-yl)pyrimidin-2-yl)amino)-3-methoxy-N-(4-methylbenzyl)benzamide (Compound 126);
4-((5-chloro-4-(1-isopropyl-1H-pyrazol-4-yl)pyrimidin-2-yl)amino)-N-(4-(dimethylamino)phenyl)-3-methoxybenzamide (Compound 127);
4-((5-chloro-4-(1-isopropyl-1H-pyrazol-4-yl)pyrimidin-2-yl)amino)-N-(4-cyanobenzyl)-3-methoxybenzamide (Compound 128);
4-((5-chloro-4-(1-isopropyl-1H-pyrazol-4-yl)pyrimidin-2-yl)amino)-N-(2,3-dihydro-1H-inden-2-yl)-3-methoxybenzamide (Compound 129);
4-((5-chloro-4-(1-isopropyl-1H-pyrazol-4-yl)pyrimidin-2-yl)amino)-3-methoxy-N-((tetrahydrofuran-3-yl)methyl)benzamide (Compound 130);
4-((5-chloro-4-(1-isopropyl-1H-pyrazol-4-yl)pyrimidin-2-yl)amino)-N-(3-cyano-4-fluorophenyl)-3-methoxybenzamide(Compound 131);
N-(5-chloro-2-fluorophenyl)-4-((5-chloro-4-(1-isopropyl-1H-pyrazol-4-yl)pyrimidin-2-yl)amino)-3-methoxybenzamide (Compound 132);
4-((5-chloro-4-(1-isopropyl-1H-pyrazol-4-yl)pyrimidin-2-yl)amino)-N-(1-(4-fluorophenyl)ethyl)-3-methoxybenzamide (Compound 133);
4-((5-chloro-4-(1-isopropyl-1H-pyrazol-4-yl)pyrimidin-2-yl)amino)-N-(2,4-difluorobenzyl)-3-methoxybenzamide (Compound 134);
4-((5-chloro-4-(1-isopropyl-1H-pyrazol-4-yl)pyrimidin-2-yl)amino)-N-(3,5-difluorobenzyl)-3-methoxybenzamide (Compound 135);
4-((5-chloro-4-(1-isopropyl-1H-pyrazol-4-yl)pyrimidin-2-yl)amino)-3-methoxy-N-(2-methylpyridin-4-yl)benzamide (Compound 136);
4-((5-chloro-4-(1-isopropyl-1H-pyrazol-4-yl)pyrimidin-2-yl)amino)-N-isopropyl-3-methoxybenzamide(Compound 137);
4-((5-chloro-4-(1-isopropyl-1H-pyrazol-4-yl)pyrimidin-2-yl)amino)-N-(2-hydroxyethyl)-3-methoxybenzamide (Compound 138);
4-((5-chloro-4-(1-isopropyl-1H-pyrazol-4-yl)pyrimidin-2-yl)amino)-N,N-diethyl-3-methoxybenzamide (Compound 139);
4-((5-chloro-4-(1-isopropyl-1H-pyrazol-4-yl)pyrimidin-2-yl)amino)-3-methoxy-N-(2-methoxyethyl)benzamide (Compound 140);
4-((5-chloro-4-(1-isopropyl-1H-pyrazol-4-yl)pyrimidin-2-yl)amino)-N-(2-hydroxyethyl)-3-methoxy-N-methylbenzamide (Compound 141);
4-((5-chloro-4-(1-isopropyl-1H-pyrazol-4-yl)pyrimidin-2-yl)amino)-N-isopropyl-3-methoxy-N-methylbenzamide (Compound 142);
4-((5-chloro-4-(1-isopropyl-1H-pyrazol-4-yl)pyrimidin-2-yl)amino)-3-methoxy-N-methyl-N-(1-methylpiperidin-4-yl)benzamide (Compound 143);
4-((5-chloro-4-(1-isopropyl-1H-pyrazol-4-yl)pyrimidin-2-yl)amino)-3-methoxy-N-(1-methyl-1H-pyrazol-4-yl)benzamide (Compound 144);
4-((5-chloro-4-(1-isopropyl-1H-pyrazol-4-yl)pyrimidin-2-yl)amino)-N-(1-isopropyl-1H-pyrazol-4-yl)-3-methoxybenzamide (Compound 145);
4-((5-chloro-4-(1-isopropyl-1H-pyrazol-4-yl)pyrimidin-2-yl)amino)-N-(4-chlorobenzyl)-3-methoxybenzamide (Compound 146);
4-((5-chloro-4-(1-isopropyl-1H-pyrazol-4-yl)pyrimidin-2-yl)amino)-N-(3-chlorobenzyl)-3-methoxybenzamide (Compound 147);
4-((5-chloro-4-(1-isopropyl-1H-pyrazol-4-yl)pyrimidin-2-yl)amino)-N-(2-chlorobenzyl)-3-methoxybenzamide (Compound 148);
4-((5-chloro-4-(1-isopropyl-1H-pyrazol-4-yl)pyrimidin-2-yl)amino)-N-(2-chlorophenyl)-3-methoxybenzamide (Compound 149);
4-((5-chloro-4-(1-isopropyl-1H-pyrazol-4-yl)pyrimidin-2-yl)amino)-N-(3-chlorophenyl)-3-methoxybenzamide (Compound 150);
4-((5-chloro-4-(1-isopropyl-1H-pyrazol-4-yl)pyrimidin-2-yl)amino)-N-(4-chlorophenyl)-3-methoxybenzamide (Compound 151);
4-((5-chloro-4-(1-isopropyl-1H-pyrazol-4-yl)pyrimidin-2-yl)amino)-N-(3-hydroxybenzyl)-3-methoxybenzamide (Compound 152);
4-((5-chloro-4-(1-isopropyl-1H-pyrazol-4-yl)pyrimidin-2-yl)amino)-N-(4-hydroxybenzyl)-3-methoxybenzamide (Compound 153);
4-((5-chloro-4-(1-isopropyl-1H-pyrazol-4-yl)pyrimidin-2-yl)amino)-3-methoxy-N-(2-methoxybenzyl)benzamide (Compound 154);
4-((5-chloro-4-(1-isopropyl-1H-pyrazol-4-yl)pyrimidin-2-yl)amino)-3-methoxy-N-(pyridin-2-ylmethyl)benzamide (Compound 155);
4-((5-chloro-4-(1-isopropyl-1H-pyrazol-4-yl)pyrimidin-2-yl)amino)-3-methoxy-N-(pyridin-3-ylmethyl)benzamide (Compound 156);
4-((5-chloro-4-(1-isopropyl-1H-pyrazol-4-yl)pyrimidin-2-yl)amino)-3-methoxy-N-(pyridin-4-ylmethyl)benzamide (Compound 157);
4-((5-chloro-4-(1-isopropyl-1H-pyrazol-4-yl)pyrimidin-2-yl)amino)-N-(2-hydroxyphenyl)-3-methoxybenzamide (Compound 158);
4-((5-chloro-4-(1-isopropyl-1H-pyrazol-4-yl)pyrimidin-2-yl)amino)-N-(3-hydroxyphenyl)-3-methoxybenzamide (Compound 159);
4-((5-chloro-4-(1-isopropyl-1H-pyrazol-4-yl)pyrimidin-2-yl)amino)-N-(4-hydroxyphenyl)-3-methoxybenzamide (Compound 160);
4-((5-chloro-4-(1-isopropyl-1H-pyrazol-4-yl)pyrimidin-2-yl)amino)-3-methoxy-N-(2-methoxyphenyl)benzamide (Compound 161);
4-((5-chloro-4-(1-isopropyl-1H-pyrazol-4-yl)pyrimidin-2-yl)amino)-3-methoxy-N-(3-methoxyphenyl)benzamide (Compound 162);
4-((5-chloro-4-(1-isopropyl-1H-pyrazol-4-yl)pyrimidin-2-yl)amino)-3-methoxy-N-(4-methoxyphenyl)benzamide (Compound 163);
4-((5-chloro-4-(1-isopropyl-1H-pyrazol-4-yl)pyrimidin-2-yl)amino)-N-(2,3-dihydro-1H-inden-1-yl)-3-methoxybenzamide (Compound 164);
4-((5-chloro-4-(1-isopropyl-1H-pyrazol-4-yl)pyrimidin-2-yl)amino)-3-methoxy-N-(2-methoxybenzyl)-N-methylbenzamide (Compound 165);
4-((5-chloro-4-(1-isopropyl-1H-pyrazol-4-yl)pyrimidin-2-yl)amino)-3-methoxy-N-(2,2,6,6-tetramethylpiperidin-4-yl)benzamide (Compound 166);
4-((5-chloro-4-(1-isopropyl-1H-pyrazol-4-yl)pyrimidin-2-yl)amino)-N-(2,6-dimethylpiperidin-1-yl)-3-methoxybenzamide (Compound 167);
4-((5-chloro-4-(1-isopropyl-1H-pyrazol-4-yl)pyrimidin-2-yl)amino)-N-((6-chloropyridin-3-yl)methyl)-3-methoxybenzamide (Compound 168);
4-((5-chloro-4-(1-isopropyl-1H-pyrazol-4-yl)pyrimidin-2-yl)amino)-3-methoxy-N-methyl-N-(2-(pyridin-2-yl)ethyl)benzamide (Compound 169);
4-((5-chloro-4-(1-isopropyl-1H-pyrazol-4-yl)pyrimidin-2-yl)amino)-N-(3-(difluoromethoxy)phenyl)-3-methoxybenzamide (Compound 170);
4-((5-chloro-4-(1-isopropyl-1H-pyrazol-4-yl)pyrimidin-2-yl)amino)-N-(3-ethylphenyl)-3-methoxybenzamide (Compound 171);
4-((5-chloro-4-(1-isopropyl-1H-pyrazol-4-yl)pyrimidin-2-yl)amino)-N-(2-ethylphenyl)-3-methoxybenzamide (Compound 172);
4-((5-chloro-4-(1-isopropyl-1H-pyrazol-4-yl)pyrimidin-2-yl)amino)-N-(4-ethylphenyl)-3-methoxybenzamide (Compound 173);
N-benzyl-4-((5-chloro-4-(1-isopropyl-1H-pyrazol-4-yl)pyrimidin-2-yl)amino)-3-methoxy-N-methylbenzamide (Compound 174);
4-((5-chloro-4-(1-isopropyl-1H-pyrazol-4-yl)pyrimidin-2-yl)amino)-3-methoxy-N-methyl-N-(3-methylbenzyl)benzamide (Compound 175);
ethyl 3-(4-((5-chloro-4-(1-isopropyl-1H-pyrazol-4-yl)pyrimidin-2-yl)amino)-3-methoxybenzamido)propanoate (Compound 176);
N-benzyl-4-((5-chloro-4-(1-isopropyl-1H-pyrazol-4-yl)pyrimidin-2-yl)amino)-N-isopropyl-3-methoxybenzamide (Compound 177);
4-((5-chloro-4-(1-isopropyl-1H-pyrazol-4-yl)pyrimidin-2-yl)amino)-N-(3-isopropoxyphenyl)-3-methoxybenzamide(Compound 178);
4-((5-chloro-4-(1-isopropyl-1H-pyrazol-4-yl)pyrimidin-2-yl)amino)-3-methoxy-N-(1-phenylethyl)benzamide(Compound 179);
4-((5-chloro-4-(1-isopropyl-1H-pyrazol-4-yl)pyrimidin-2-yl)amino)-3-methoxy-N-(3-(trifluoromethyl)phenyl)benzamide (Compound 180);
4-((5-chloro-4-(1-isopropyl-1H-pyrazol-4-yl)pyrimidin-2-yl)amino)-3-methoxy-N-(4-(trifluoromethyl)phenyl)benzamide (Compound 181);
4-((5-chloro-4-(1-isopropyl-1H-pyrazol-4-yl)pyrimidin-2-yl)amino)-3-methoxy-N-(1-phenylpropyl)benzamide (Compound 182);
N-(4-aminobenzyl)-4-((5-chloro-4-(1-isopropyl-1H-pyrazol-4-yl)pyrimidin-2-yl)amino)-3-methoxybenzamide(Compound 183);
4-((5-chloro-4-(1-isopropyl-1H-pyrazol-4-yl)pyrimidin-2-yl)amino)-N-(2-fluoro-5-methylphenyl)-3-methoxybenzamide (Compound 184);
4-((5-chloro-4-(1-isopropyl-1H-pyrazol-4-yl)pyrimidin-2-yl)amino)-N-(2-fluoro-4-methylphenyl)-3-methoxybenzamide (Compound 185);
4-((5-chloro-4-(1-isopropyl-1H-pyrazol-4-yl)pyrimidin-2-yl)amino)-N-(4-fluoro-2-methylphenyl)-3-methoxybenzamide (Compound 186);
4-((5-chloro-4-(1-isopropyl-1H-pyrazol-4-yl)pyrimidin-2-yl)amino)-N-(4-fluoro-3-methylphenyl)-3-methoxybenzamide (Compound 187);
4-((5-chloro-4-(1-isopropyl-1H-pyrazol-4-yl)pyrimidin-2-yl)amino)-N-(3-fluoro-2-methylphenyl)-3-methoxybenzamide (Compound 188);
4-((5-chloro-4-(1-isopropyl-1H-pyrazol-4-yl)pyrimidin-2-yl)amino)-3-methoxy-N-(4-sulfamoylbenzyl)benzamide (Compound 189);
4-((5-chloro-4-(1-isopropyl-1H-pyrazol-4-yl)pyrimidin-2-yl)amino)-3-methoxy-N-(3-(methylsulfonamido)phenyl)benzamide (Compound 190);
4-((5-chloro-4-(1-isopropyl-1H-pyrazol-4-yl)pyrimidin-2-yl)amino)-3-methoxy-N-(3-sulfamoylphenyl)benzamide (Compound 191);
4-((5-chloro-4-(1-isopropyl-1H-pyrazol-4-yl)pyrimidin-2-yl)amino)-3-methoxy-N-(2-(methylsulfonyl)ethyl)benzamide (Compound 192);
methyl (4-((5-chloro-4-(1-isopropyl-1H-pyrazol-4-yl)pyrimidin-2-yl)amino)-3-methoxybenzoyl)-L-valinate (Compound 193);
methyl (4-((5-chloro-4-(1-isopropyl-1H-pyrazol-4-yl)pyrimidin-2-yl)amino)-3-methoxybenzoyl)-L-isoleucinate (Compound 194);
ethyl (4-((5-chloro-4-(1-isopropyl-1H-pyrazol-4-yl)pyrimidin-2-yl)amino)-3-methoxybenzoyl)glycinate (Compound 195);
N-(4-aminophenethyl)-4-((5-chloro-4-(1-isopropyl-1H-pyrazol-4-yl)pyrimidin-2-yl)amino)-3-methoxybenzamide(Compound 196);
N-(3-(1H-imidazol-1-yl)propyl)-4-((5-chloro-4-(1-isopropyl-1H-pyrazol-4-yl)pyrimidin-2-yl)amino)-3-methoxybenzamide (Compound 197);
4-((5-chloro-4-(1-isopropyl-1H-pyrazol-4-yl)pyrimidin-2-yl)amino)-N-(2,2-dimethoxyethyl)-3-methoxybenzamide (Compound 198);
methyl (4-((5-chloro-4-(1-isopropyl-1H-pyrazol-4-yl)pyrimidin-2-yl)amino)-3-methoxybenzoyl)-L-leucinate(Compound 199);
methyl (4-((5-chloro-4-(1-isopropyl-1H-pyrazol-4-yl)pyrimidin-2-yl)amino)-3-methoxybenzoyl)-L-prolinate (Compound 200);
4-((5-chloro-4-(1-isopropyl-1H-pyrazol-4-yl)pyrimidin-2-yl)amino)-3-methoxy-N-(4-methoxybenzyl)-N-methylbenzamide (Compound 201);
4-((5-chloro-4-(1-isopropyl-1H-pyrazol-4-yl)pyrimidin-2-yl)amino)-N-ethyl-3-methoxy-N-methylbenzamide (Compound 202);
4-((5-chloro-4-(1-isopropyl-1H-pyrazol-4-yl)pyrimidin-2-yl)amino)-3-methoxy-N-(4-methoxybenzyl)benzamide (Compound 203);
4-((5-chloro-4-(1-isopropyl-1H-pyrazol-4-yl)pyrimidin-2-yl)amino)-3-methoxy-N-(4-methoxy-3,5-dimethylphenyl)benzamide (Compound 204);
N-(3-aminobenzyl)-4-((5-chloro-4-(1-isopropyl-1H-pyrazol-4-yl)pyrimidin-2-yl)amino)-3-methoxybenzamide (Compound 205);
4-((5-chloro-4-(1-isopropyl-1H-pyrazol-4-yl)pyrimidin-2-yl)amino)-N-(2,3-dihydro-1H-inden-5-yl)-3-methoxybenzamide (Compound 206);
4-((5-chloro-4-(1-isopropyl-1H-pyrazol-4-yl)pyrimidin-2-yl)amino)-N-(2,3-dihydro-1H-inden-4-yl)-3-methoxybenzamide (Compound 207);
4-((5-chloro-4-(1-isopropyl-1H-pyrazol-4-yl)pyrimidin-2-yl)amino)-N-(2,3-dihydro-1H-inden-2-yl)-3-methoxy-N-methylbenzamide (Compound 208);
4-((5-chloro-4-(1-isopropyl-1H-pyrazol-4-yl)pyrimidin-2-yl)amino)-N-(2,3-dihydro-1H-inden-1-yl)-3-methoxy-N-methylbenzamide (Compound 209);
(4-((5-chloro-4-(1-isopropyl-1H-pyrazol-4-yl)pyrimidin-2-yl)amino)-3-methoxyphenyl)(4-(ethyl(methyl)amino)piperidin-1-yl)methanone (Compound 210) ;
N-(1-acetylindolin-5-yl)-4-((5-chloro-4-(1-isopropyl-1H-pyrazol-4-yl)pyrimidin-2-yl)amino)-3-methoxy-N-methylbenzamide (Compound 211);
N-(1-acetylindolin-5-yl)-4-((5-chloro-4-(1-isopropyl-1H-pyrazol-4-yl)pyrimidin-2-yl)amino)-3-methoxybenzamide (Compound 212);
ethyl 3-(4-((5-chloro-4-(1-isopropyl-1H-pyrazol-4-yl)pyrimidin-2-yl)amino)-3-methoxybenzamido)-4,4,4-trifluorobutanoate (Compound 213);
4-((5-chloro-4-(1-isopropyl-1H-pyrazol-4-yl)pyrimidin-2-yl)amino)-N-(2,3-dihydro-1H-inden-5-yl)-3-methoxy-N-methylbenzamide (Compound 214);
4-((5-chloro-4-(1-isopropyl-1H-pyrazol-4-yl)pyrimidin-2-yl)amino)-N-(2,3-dihydro-1H-inden-4-yl)-3-methoxy-N-methylbenzamide (Compound 215);
(4-((5-chloro-4-(1-isopropyl-1H-pyrazol-4-yl)pyrimidin-2-yl)amino)-3-methoxyphenyl)(4-(pyrrolidin-1-yl)piperidin-1-yl)methanone (Compound 216); and
[1,4'-bipiperidin]-1'-yl(4-((5-chloro-4-(1-isopropyl-1H-pyrazol-4-yl)pyrimidin-2-yl)amino)-3-methoxyphenyl)methanone (Compound 217).

3. A pharmaceutical composition for prevention or treatment of a neurodegenerative disease, the composition comprising the compound represented by Chemical Formula 1 of claim 1, an optical isomer thereof, or a pharmaceutically acceptable salt thereof as an active ingredient.

4. The pharmaceutical composition according to claim 3, wherein the neurodegenerative disease is selected from the group consisting of Parkinson's disease, Alzheimer's disease, Huntington's disease, Pick's disease, amyotrophic lateral sclerosis, prion disease, motor neuron disease, spinocerebellar ataxia, spinal muscular atrophy, Creutzfeldt-Jakob disease, and alcohol related dementia.

5. A pharmaceutical composition for prevention or treatment of a cancer, the composition comprising the compound represented by Chemical Formula 1 of claim 1, an optical isomer thereof, or a pharmaceutically acceptable salt thereof as an active ingredient.

6. The pharmaceutical composition according to claim 5, wherein the cancer is selected from the group consisting of kidney cancer, thyroid cancer, breast cancer, liver cancer, and brain cancer.
